# EUROPEAN PATENT APPLICATION

(11) **EP 1 557 178 A1**
(43) Date of publication of application: **27.07.2005**
(21) Application number: 03758999.1
(22) Date of filing: 28.10.2003
(51) Int. Cl.: A61K 45/00, A61K 31/7088, A61K 38/17, A61K 48/00, A61P 3/04, A61P 3/06, A61P 3/10, G01N 33/566, G01N 33/50, G01N 33/15, C12N 15/12

(54) **USE OF SGLT HOMOLOG**

(30) Priority: 29.10.2002 JP 2002314041; 02.06.2003 JP 2003156306
(71) Applicant: Takeda Pharmaceutical Company Limited, Osaka-shi, Osaka 541-0045 (JP)
(72) Inventor: IWAMOTO, Keiji, Takeda Pharmaceutical Company Ltd, Osaka 532-8686 (JP); KATAYAMA, Nozomi,Takeda Pharmaceutical Company Ltd, Osaka 532-8686 (JP); KAWAMURA, Mihoko,Takeda Pharmaceutical Company Ltd, Ibaraki 300-4293 (JP)
(74) Representative: Rickard, Timothy Mark Adrian
(86) International application number: PCT/JP2003/013782
(87) International publication number: WO 2004/039405

(57) **Abstract**

The present invention provides an agent that inhibits or promotes glucose uptake in the small intestine, etc., comprising a compound that inhibits or promotes the activity of Na⁺/glucose transporter (SGLT) homologs.

SEQUENCE LISTING

<110> Takeda Pharmaceutical Compnay Limited

<120> Use of SGLT homolog

<130> G05-0003

<150> JP 2002-314041
<151> 2002-10-29

<150> JP 2003-156306
<151> 2003-6-2

<160> 57

<210> 1
<211> 674
<212> PRT
<213> Homo Sapiens

<400> 1

<210> 2
<211> 2022
<212> DNA
<213> Homo sapiens

<400> 2
   atggggcctg gagcttcagg ggacggggtc aggactgaga cagctccaca catagcactg      60
   gactccagag ttggtctgca cgcctacgac atcagcgtgg tggtcatcta ctttgtcttc     120
   gtcattgctg tggggatctg gtcgtccatc cgtgcaagtc gagggaccat tggcggctat     180
   ttcctggccg ggaggtccat gagctggtgg ccaattggag catctctgat gtccagcaat     240
   gtgggcagtg gcttgttcat cggcctggct gggacagggg ctgccggagg ccttgccgta     300
   ggtggcttcg agtggaacgc aacctggctg ctcctggccc ttggctgggt cttcgtccct     360
   gtgtacatcg cagcaggtgt ggtcacaatg ccgcagtatc tgaagaagcg atttgggggc     420
   cagaggatcc aggtgtacat gtctgtcctg tctctcatcc tctacatctt caccaagatc     480
   tcgactgaca tcttctctgg agccctcttc atccagatgg cattgggctg gaacctgtac     540
   ctctccacag ggatcctgct ggtggtgact gccgtctaca ccattgcagg tggcctcatg     600
   gccgtgatct acacagatgc tctgcagacg gtgatcatgg tagggggagc cctggtcctc     660
   atgtttctgg gctttcagga cgtgggctgg tacccaggcc tggagcagcg gtacaggcag     720
   gccatcccta atgtcacagt ccccaacacc acctgtcacc tcccacggcc cgatgctttc     780
   cacatgcttc gggaccctgt gagcggggac atcccttggc caggtctcat tttcgggctc     840
   acagtgctgg ccacctggtg ttggtgcaca gaccaggtca ttgtgcagcg gtctctctcg     900
   gccaagagtc tgtctcatgc caagggaggc tccgtgctgg ggggctacct gaagatcctc     960
   cccatgttct tcatcgtcat gcctggcatg atcagccggg ccctgttccc agacgaggtg    1020
   ggctgcgtgg accctgatgt ctgccaaaga atctgtgggg cccgagtggg atgttccaac    1080
   attgcctacc ctaagttggt catggccctc atgcctgttg gtctgcgggg gctgatgatt    1140
   gccgtgatca tggccgctct catgagctca ctcacctcca tcttcaacag cagcagcacc    1200
   ctgttcacca ttgatgtgtg gcagcgcttc cgcaggaagt caacagagca ggagctgatg    1260
   gtggtgggca gagtgtttgt ggtgttcctg gttgtcatca gcatcctctg gatccccatc 1320
   atccaaagct ccaacagtgg gcagctcttc gactacatcc aggctgtcac cagttacctg 1380
   gccccaccca tcaccgctct cttcctgctg gccatcttct gcaagagggt cacagagccc 1440
   ggagctttct ggggcctcgt gtttggcctg ggagtggggc ttctgcgtat gatcctggag 1500
   ttctcatacc cagcgccagc ctgtggggag gtggaccgga ggccagcagt gctgaaggac 1560
   ttccactacc tgtactttgc aatcctcctc tgcgggctca ctgccatcgt cattgtcatt 1620
   gtcagcctct gtacaactcc catccctgag gaacagctca cacgcctcac atggtggact 1680
   cggaactgcc ccctctctga gctggagaag gaggcccacg agagcacacc ggagatatcc 1740
   gagaggccag ccggggagtg ccctgcagga ggtggagcgg cagagaactc gagcctgggc 1800
   caggagcagc ctgaagcccc aagcaggtcc tggggaaagt tgctctggag ctggttctgt 1860
   gggctctctg gaacaccgga gcaggccctg agcccagcag agaaggctgc gctagaacag 1920
   aagctgacaa gcattgagga ggagccactc tggagacatg tctgcaacat caatgctgtc 1980
   cttttgctgg ccatcaacat cttcctctgg ggctattttg cg 2022

<210> 3
<211> 678
<212> PRT
<213> Mus musculus

<400> 3
Met Glu Pro Gly Val Ser Arg Asn Gly Val Arg Thr Glu Thr Thr Thr
1               5                   10                  15
Asn Pro Ser Leu Gly Leu His Thr Tyr Asp Ile Val Val Val Val Ile
            20                  25                  30
Tyr Phe Val Phe Val Leu Ala Val Gly Ile Trp Ser Ser Ile Arg Ala
        35                  40                  45
Ser Arg Gly Thr Val Gly Gly Tyr Phe Leu Ala Gly Arg Ser Met Thr
    50                  55                  60
Trp Trp Pro Ile Gly Ala Ser Leu Met Ser Ser Asn Val Gly Ser Gly
65                  70                  75                  80
Leu Phe Ile Gly Leu Ala Gly Thr Gly Ala Ala Gly Gly Leu Ala Val
                85                  90                  95
Gly Gly Phe Glu Trp Asn Ala Thr Phe Leu Leu Leu Ala Leu Gly Trp
            100                 105                 110
Ile Phe Val Pro Val Tyr Ile Ala Ala Gly Val Val Thr Met Pro Gln
        115                 120                 125
Tyr Leu Lys Lys Arg Phe Gly Gly Gln Arg Ile Gln Val Tyr Met Ser
    130                 135                 140
Val Leu Ser Leu Ile Leu Tyr Ile Phe Thr Lys Ile Ser Thr Asp Ile
145                 150                 155                 160
Phe Ser Gly Ala Leu Phe Ile Gln Met Ala Leu Gly Trp Asn Leu Tyr
                165                 170                 175
Leu Ser Thr Val Ile Leu Leu Val Val Thr Ala Val Tyr Thr Ile Ala
            180                 185                 190
Gly Gly Leu Thr Ala Val Ile Tyr Thr Asp Ala Leu Gln Thr Val Ile
        195                 200                 205
Met Val Gly Gly Ala Leu Val Leu Met Phe Leu Gly Phe Gln Glu Val
    210                 215                 220
Gly Trp Tyr Pro Gly Leu Gln Gln Leu Tyr Arg Gln Ala Ile Pro Asn
225                 230                 235                 240
Thr Thr Val Pro Asn Thr Thr Cys His Leu Pro Arg Pro Asp Ala Phe
                245                 250                 255
His Met Leu Arg Asp Pro Val Asn Gly Asp Ile Pro Trp Pro Gly Leu
            260                 265                 270
Ile Phe Gly Leu Thr Val Leu Ala Thr Trp Cys Trp Cys Thr Asp Gln
        275                 280                 285
Val Ile Val Gln Arg Ser Leu Ala Ala Lys Asn Leu Ser His Ala Lys
    290                 295                 300
Gly Gly Ser Val Leu Gly Gly Tyr Leu Lys Ile Leu Pro Met Phe Phe
305                 310                 315                 320
Ile Val Met Pro Gly Met Ile Ser Arg Ala Leu Tyr Pro Asp Glu Val
                325                 330                 335
Ala Cys Val Asp Pro Asp Ile Cys Gln Arg Val Cys Gly Ala Arg Val
            340                 345                 350
Gly Cys Ser Asn Ile Ala Tyr Pro Lys Leu Val Met Ala Leu Met Pro
        355                 360                 365
Val Gly Leu Arg Gly Leu Met Ile Ala Val Ile Met Ala Ala Leu Met
    370                 375                 380

Ser Ser Leu Thr Ser Ile Phe Asn Ser Ser Ser Thr Leu Phe Ala Ile
385                 390                 395                 400
Asp Val Trp Gln Arg Phe Arg Arg Gln Ala Ser Glu Gln Glu Leu Met
                405                 410                 415
Val Val Gly Arg Leu Phe Val Val Phe Leu Val Val Ile Ser Ile Leu
            420                 425                 430
Trp Ile Pro Ile Ile Gln Ser Ser Asn Ser Gly Gln Leu Phe Asp Tyr
        435                 440                 445
Ile Gln Ser Ile Thr Ser Tyr Leu Ala Pro Pro Ile Thr Ala Leu Phe
    450                 455                 460
Leu Leu Ala Ile Phe Cys Lys Arg Val Asn Glu Pro Gly Ala Phe Trp
465                 470                 475                 480
Gly Leu Met Phe Gly Leu Val Val Gly Ile Leu Arg Met Ile Leu Glu
                485                 490                 495
Phe Ser Tyr Ser Ala Pro Ala Cys Gly Glu Met Asp Arg Arg Pro Ala
            500                 505                 510
Val Leu Lys Asp Phe His Tyr Leu Tyr Phe Ala Leu Leu Leu Cys Gly
        515                 520                 525
Leu Thr Ala Ile Ile Ile Val Val Ile Ser Phe Phe Thr Glu Pro Ile
    530                 535                 540
Pro Asp Asp Lys Leu Ala Arg Leu Thr Trp Trp Thr Arg Asn Cys Ala
545                 550                 555                 560
Val Ser Asp Leu Gln Lys Lys Thr Ser Val Ser Val Asn Asn Thr Glu
                565                 570                 575
Asp Asp Asn Ser Pro Gly Leu Ala Gly Arg Pro Val Val Glu Gly Pro
            580                 585                 590
Ala Gly Asp Glu Glu Glu Ala Asn Thr Thr Gln Gly Pro Glu Gln Pro
        595                 600                 605
Gly Ala Leu His Arg Ser Trp Gly Lys Trp Leu Trp Asn Trp Phe Cys
    610                 615                 620
Gly Leu Ser Gly Ala Pro Gln Gln Ala Leu Ser Pro Ala Glu Lys Ala
625                 630                 635                 640
Val Leu Glu Gln Lys Leu Thr Ser Ile Glu Glu Glu Pro Leu Trp Arg
                645                 650                 655
Arg Val Cys Asn Ile Asn Ala Ile Ile Leu Leu Ala Ile Asn Ile Phe
            660                 665                 670
Leu Trp Gly Tyr Phe Ala
        675         678

<210> 4
<211> 2034
<212> DNA
<213> Mus musculus

<400> 4
atggaaccag gagtgtcaag gaatggagtc agaactgaga caacaacgaa cccaagcctg      60
gggctacata cctatgacat cgtggtggtg gtcatctatt ttgtctttgt tcttgctgtg     120
ggaatttggt catccatccg tgcaagtcga gggaccgttg gtggctattt cctggctggg     180
agatccatga cctggtggcc aattggagca tctctaatgt ccagcaatgt gggcagtggc     240
ttatttatcg gcctggctgg aacaggggct gctggaggac ttgctgttgg tggctttgag     300
tggaacgcaa ccttcctgct tctagccctg ggctggatct ttgtccctgt gtacatagca     360
gctggtgtgg tcaccatgcc acagtacctg aagaaacgat ttgggggaca gaggatccag     420
gtgtacatgt cagttctttc tctcatcctc tacatcttca ccaagatatc gactgatatc     480
ttctctggag ccctcttcat ccagatggcc ttgggctgga atctctatct ctccacagtc     540
atcttgctgg tggtgacagc tgtctacacc attgcagggg gcctcacagc tgtgatctac     600
acagatgctc tacagactgt gatcatggtt gggggagctc tggtcctcat gtttctgggc     660
tttcaggagg ttggctggta cccaggcctg cagcagctct atagacaggc catccccaat     720
accacagttc ccaataccac ctgtcacctc ccacggcctg atgccttcca catgcttcga     780
gatcctgtga atggagacat cccctggcca ggtctcattt ttggcctcac agtcttggcc     840
acctggtgtt ggtgcacaga ccaggtgatt gtgcagaggt ctctcgcagc caagaatctt     900
tcacatgcca agggaggctc cgtgctaggg ggctacctaa agatcctccc aatgttcttc     960
attgtcatgc ctggcatgat cagcagggcc ctgtacccag atgaagttgc ctgtgtggac    1020
cctgacatct gtcaaagagt gtgtggggcc agagttggat gctccaatat tgcctacccc    1080
aagctggtta tggctctcat gcctgtgggg ctgcgaggcc tgatgattgc tgtgatcatg    1140
gctgccctca tgagctcact cacctctatc ttcaacagca gtagcaccct gtttgccata    1200
gatgtgtggc agcgcttccg caggcaggca tcggagcaag agctgatggt ggtaggcagg    1260
ttgttcgtag tcttcctggt agtcatcagc atcctctgga tccccatcat ccagagctcc    1320
aatagtgggc agctctttga ctacatccaa tctatcacca gctacttagc cccacccatc    1380

acagccctct tcctgctggc tatcttctgc aagagggtca acgagcctgg tgccttctgg 1440
ggcctcatgt ttggcctggt cgtcggaata ctgcgtatga ttctggagtt ctcatactcg 1500
gccccagcct gtggggagat ggacaggcgg ccagctgttc tgaaggactt ccactacctg 1560
tactttgccc ttctcctctg tggactgacc gcgatcatca ttgtcgtaat cagcttcttc 1620
acggagccca tccccgatga caagcttgct cgcctgacct ggtggacaag gaactgtgcc 1680
gtatctgacc tgcagaagaa aacctctgtg agtgtgaaca acacagagga tgacaactct 1740
ccaggactgg cagggaggcc agtggtagag ggccctgcag gagatgagga agaagcaaac 1800
accactcagg ggcctgaaca accaggagcc ctacacaggt cctggggaaa atggctgtgg 1860
aactggttct gcggactctc aggagcccca cagcaagccc tgagcccagc tgagaaggct 1920
gtgttggagc agaagctgac cagcatcgag gaggagccgc tctggagacg tgtctgcaac 1980
atcaacgcca tcatcctgct agccatcaac atctttctct ggggctattt tgcg 2034

<210> 5
<211> 681
<212> PRT
<213> Rattus norvegicus

<400> 5
Met Glu Pro Gly Ala Ser Arg Asp Gly Leu Arg Ala Glu Thr Thr His
1               5                   10                  15
Gln Ala Leu Gly Ser Gly Val Ser Leu His Thr Tyr Asp Ile Val Val
            20                  25                  30
Val Val Ile Tyr Phe Val Phe Val Leu Ala Val Gly Ile Trp Ser Ser
        35                  40                  45
Ile Arg Ala Ser Arg Gly Thr Ile Gly Gly Tyr Phe Leu Ala Gly Arg
    50                  55                  60
Ser Met Thr Trp Trp Pro Ile Gly Ala Ser Leu Met Ser Ser Asn Val
65                  70                  75                  80
Gly Ser Gly Leu Phe Ile Gly Leu Ala Gly Thr Gly Ala Ala Gly Gly
                85                  90                  95
Leu Ala Val Gly Gly Phe Glu Trp Asn Ala Thr Phe Leu Leu Leu Ala
            100                 105                 110
Leu Gly Trp Ile Phe Val Pro Val Tyr Ile Ala Ala Gly Val Val Thr
        115                 120                 125
Met Pro Gln Tyr Leu Lys Lys Arg Phe Gly Gly Gln Arg Ile Gln Val
    130                 135                 140
Tyr Met Ser Val Leu Ser Leu Ile Leu Tyr Ile Phe Thr Lys Ile Ser
145                 150                 155                 160
Thr Asp Ile Phe Ser Gly Ala Leu Phe Ile Gln Met Ala Leu Gly Trp
                165                 170                 175
Asn Leu Tyr Leu Ser Thr Val Ile Leu Leu Val Val Thr Ala Val Tyr
            180                 185                 190
Thr Ile Ala Gly Gly Leu Thr Ala Val Ile Tyr Thr Asp Ala Leu Gln
        195                 200                 205
Thr Val Ile Met Val Gly Gly Ala Leu Val Leu Met Phe Leu Gly Phe
    210                 215                 220
Arg Glu Val Gly Trp Tyr Pro Gly Leu Gln Gln Leu Tyr Arg Gln Ser
225                 230                 235                 240
Ile Pro Asn Val Thr Val Pro Asn Thr Thr Cys His Leu Pro Arg Ser
                245                 250                 255
Asp Ala Phe His Met Leu Arg Asp Pro Val Asn Gly Asp Ile Pro Trp
            260                 265                 270
Pro Gly Leu Ile Phe Gly Leu Thr Val Leu Ala Thr Trp Cys Trp Cys
        275                 280                 285
Thr Asp Gln Val Ile Val Gln Arg Ser Leu Ser Ala Lys Ser Leu Ser
    290                 295                 300
His Ala Lys Gly Gly Ser Val Leu Gly Gly Tyr Leu Lys Ile Leu Pro
305                 310                 315                 320
Met Phe Phe Ile Val Met Pro Gly Met Ile Ser Arg Ala Leu Tyr Pro
                325                 330                 335
Asp Glu Val Ala Cys Val Asp Pro Asp Ile Cys Gln Arg Val Cys Gly
            340                 345                 350
Ala Arg Val Gly Cys Ser Asn Ile Ala Tyr Pro Lys Leu Val Met Ala
        355                 360                 365
Leu Met Pro Val Gly Leu Arg Gly Leu Met Ile Ala Val Ile Met Ala
    370                 375                 380
Ala Leu Met Ser Ser Leu Thr Ser Ile Phe Asn Ser Ser Ser Thr Leu
385                 390                 395                 400

Phe Ala Ile Asp Val Trp Gln Arg Val Arg Arg Gln Ala Ser Glu Gln
                405                 410                 415
Glu Leu Met Val Val Gly Arg Leu Phe Val Val Phe Leu Val Leu Ile
            420                 425                 430
Ser Ile Leu Trp Ile Pro Ile Ile Gln Ser Ser Asn Ser Gly Gln Leu
        435                 440                 445
Phe Asp Tyr Ile Gln Ser Ile Thr Ser Tyr Leu Ala Pro Pro Ile Thr
    450                 455                 460
Ala Leu Phe Leu Leu Ala Ile Phe Cys Lys Arg Val Thr Glu Pro Gly
465                 470                 475                 480
Ala Phe Trp Gly Leu Met Phe Gly Leu Val Val Gly Ile Leu Arg Met
                485                 490                 495
Ile Leu Glu Phe Ser Tyr Ser Ala Pro Ala Cys Gly Glu Lys Asp Arg
            500                 505                 510
Arg Pro Ala Val Leu Lys Asp Phe His Tyr Leu Tyr Phe Ala Leu Leu
        515                 520                 525
Leu Cys Gly Leu Thr Ala Ile Ile Ile Val Ile Ile Ser Phe Phe Thr
    530                 535                 540
Glu Pro Ile Pro Asp Glu Lys Leu Ala Arg Leu Thr Trp Trp Thr Arg
545                 550                 555                 560
Ser Cys Pro Ile Ser Glu Leu Gln Lys Lys Val Ser Val Ser Val Asn
                565                 570                 575
Asn Thr Glu Ser Asp Asn Ser Pro Ala Leu Ala Gly Arg Pro Val Met
            580                 585                 590
Glu Gly Thr Ala Gly Asp Glu Glu Glu Ala Asn Thr Thr Ser Glu Pro
        595                 600                 605
Glu Gln Pro Glu Val Leu His Arg Ser Trp Gly Lys Trp Leu Trp Asn
    610                 615                 620
Trp Phe Cys Gly Leu Ser Gly Thr Pro Gln Gln Ala Leu Ser Pro Ala
625                 630                 635                 640
Glu Lys Ala Glu Leu Glu Gln Lys Leu Thr Ser Ile Glu Glu Glu Pro
                645                 650                 655
Leu Trp Arg Cys Val Cys Asn Ile Asn Ala Ile Ile Leu Leu Ala Ile
            660                 665                 670
Asn Ile Phe Leu Trp Gly Tyr Phe Ala
        675                 680

<210> 6
<211> 2043
<212> DNA
<213> Rattus norvegicus

<400> 6
   atggaacctg gagcttcaag ggatggactc agagctgaga caacacacca agccctgggc      60
   tctggagtca gcctgcacac ctatgacatc gtggtggtgg tcatctactt tgtctttgtc     120
   cttgctgtgg gaatttggtc gtccatccgc gcaagccgag ggaccattgg tggctatttc     180
   ctggctggaa gatccatgac ctggtggcca attggagcat ctctaatgtc cagcaatgtg     240
   ggcagtggct tattcatcgg cctggctgga acaggggctg ctggaggcct tgctgtgggt     300
   ggcttcgagt ggaatgcaac ttttctgctt ctggccctgg gctggatctt tgtccctgtg     360
   tacatcgcag ctggtgtggt caccatgcca cagtacctga agaaacgatt tggggggcag     420
   aggatccagg tgtacatgtc agtcctgtct ctcatactct acatcttcac caagatatcg     480
   actgatatct tctctggagc cctcttcatc cagatggcct tgggctggaa tctctatctc     540
   tccacagtca tcctgctggt ggtgacagct gtctacacca ttgcaggggg cctcacagct     600
   gtgatctaca cagatgctct acagaccgtg atcatggttg ggggagccct ggtcctcatg     660
   tttctgggct ttcgggaggt cggctggtac ccaggcttgc agcagctcta tagacagtcc     720
   atccccaatg tcacagttcc caacactacc tgtcacctcc cacggtctga tgccttccac     780
   atgcttcgag atcctgtgaa cggggacatc ccctggccag gtcttatttt tggcctcaca     840
   gtcttggcca cctggtgttg gtgcacggac caggtgattg tgcagaggtc tctctcggcc     900
   aagagtcttt cacatgccaa gggaggatca gtgttagggg gctacctaaa gatcctccca     960
   atgttcttca ttgtcatgcc cggcatgatc agcagggccc tgtacccaga tgaagtcgcc    1020
   tgtgtggacc ctgacatctg tcagagagtg tgtggggcca gagttggatg ctccaatatt    1080
   gcctacccca aacttgttat ggctctcatg cctgtgggtc tgcgaggcct gatgattgcc    1140
   gtgatcatgg ctgccctcat gagctcactc acctccatct tcaacagcag tagcaccctg    1200
   tttgccatag atgtgtggca gcgagtccgc aggcaggcat cggagcaaga gctgatggtg    1260
   gtaggcaggt tgtttgtagt cttcctggta ctcatcagca tcctctggat ccccatcatc    1320
   cagagctcca atagtgggca gctctttgac tacatccaat ccatcaccag ctacctagcc    1380
   ccgcccatca cagccctctt cctgctggcc atcttctgca agagggtcac tgagcctggt    1440
   gccttctggg gcctcatgtt tggcctggta gtgggaatac tgcgtatgat tctggagttc    1500
   tcatactcag ccccagcctg tggggagaag gacaggcggc cagctgttct taaggacttc 1560
   cactacctgt actttgccct cctcctctgt ggacttaccg ccatcatcat tgtcataatc 1620
   agcttcttca cggagcccat ccccgacgaa aagcttgctc gcctgacctg gtggacaagg 1680
   agctgtccca tatctgaact acagaagaaa gtctctgtga gtgtgaacaa cacagagagt 1740
   gacaactctc cagcactggc agggaggcca gtgatggagg gcactgcagg agatgaggaa 1800
   gaagcaaaca ccacctcaga gcctgaacaa ccagaagtcc tacacaggtc ctgggggaaa 1860
   tggctgtgga actggttctg cggactctct ggaacaccac agcaagcact gagcccagct 1920
   gagaaggctg agctggagca gaagctgacc agcatcgagg aagagccact ctggagatgt 1980
   gtctgcaaca tcaatgccat catcctgctg gccatcaaca tctttctctg gggctatttt 2040
   gcg 2043

<210> 7
<211> 22
<212> DNA
<213> Artificial Sequence

<220>
<223> Primer

<400> 7
cccgatgctt tccacatgct tc 22

<210> 8
<211> 22
<212> DNA
<213> Artificial Sequence

<220>
<223> Primer

<400> 8
acaatgacct ggtctgtgca cc 22

<210> 9
<211> 28
<212> DNA
<213> Artificial Sequence

<220>
<223> Probe

<400> 9
acatcccttg gccaggtctc attttcgg 28

<210> 10
<211> 23
<212> DNA
<213> Artificial Sequence

<220>
<223> Primer
<400> 10
gggggccaga ggatccaggt gta 23

<210> 11
<211> 22
<212> DNA
<213> Artificial Sequence

<220>
<223> Primer
<400> 11
gcaatcatca gcccccgcag ac 22

<210> 12
<211> 20
<212> DNA
<213> Artificial Sequence

<220>
<223> Primer

<400> 12
agcaccctct tcaccatgga                                                    20

<210> 13
<211> 21
<212> DNA
<213> Artificial Sequence

<220>
<223> Primer

<400> 13
aaacaacctt ccggcaatca t                                                  21

<210> 14
<211> 25
<212> DNA
<213> Artificial Sequence

<220>
<223> Probe

<400> 14
ccaaggtccg caagagagca tctga                                              25

<210> 15
<211> 22
<212> DNA
<213> Artificial Sequence

<220>
<223> Primer

<400> 15
tgtgtcgtcc cttcagaatg tg                                                 22

<210> 16
<211> 27
<212> DNA
<213> Artificial Sequence

<220>
<223> Primer
<400> 16
agaactagtt caggcaaaat atgcatg                                            27

<210> 17
<211> 15
<212> PRT
<213> Homo sapiens

<400> 17
His Asn Leu Arg Asp Pro Val Ser Gly Asp Ile Pro Trp Gly Cys
                5                   10                  15
<210> 18
<211> 21
<212> DNA
<213> Artificial Sequence

<220>
<223> Primer

<400> 18
tgcacagacc aggtgattgt g                                                  21

<210> 19
<211> 17
<212> DNA
<213> Artificial Sequence

<220>
<223> Primer

<400> 19
gcacggagcc tcccttg                                                    17

<210> 20
<211> 25
<212> DNA
<213> Artificial Sequence

<220>
<223> Probe

<400> 20
ctcgcagcca acaatctttc acatg                                           25

<210> 21
<211> 22
<212> DNA
<213> Artificial Sequence

<220>
<223> Primer

<400> 21
atctctaatg tccagcaatg tg                                              22

<210> 22
<211> 20
<212> DNA
<213> Artificial Sequence

<220>
<223> Primer

<400> 22
accagcttgg ggtaggcaat                                                 20

<210> 23
<211> 20
<212> DNA
<213> Artificial Sequence

<220>
<223> Primer

<400> 23
ctcacagtct tggccacctg                                                 20

<210> 24
<211> 20
<212> DNA
<213> Artificial Sequence

<220>
<223> Primer

<400> 24
agaaccggct ctctctggag                                                 20

<210> 25

<211> 22
<212> DNA
<213> Artificial Sequence

<220>
<223> Probe

<400> 25
tgcacggacc aggtgattgt gc                                                22

<210> 26
<211> 22
<212> DNA
<213> Artificial Sequence

<220>
<223> Primer

<400> 26
tctggagtca gcctgcacac ct                                                22

<210> 27
<211> 22
<212> DNA
<213> Artificial Sequence

<220>
<223> Primer

<400> 27
cagccttctc agctgggctc ag                                                22

<210> 28
<211> 23
<212> DNA
<213> Artificial Sequence

<220>
<223> Primer

<400> 28
gggggccaga ggatccaggt gta                                               23

<210> 29
<211> 26
<212> DNA
<213> Artificial Sequence

<220>
<223> Primer

<400> 29
aaaatagccc cagaggaaga tgttga                                            26

<210> 30
<211> 20
<212> DNA
<213> Artificial Sequence

<220>
<223> Primer

<400> 30
atcctgactg ggtttgcttt                                                   20

<210> 31
<211> 20
<212> DNA

<213> Artificial Sequence

<220>
<223> Primer

<400> 31
atgctgatgc caatcagcac                                                    20

<210> 32
<211> 20
<212> DNA
<213> Artificial Sequence

<220>
<223> Primer

<400> 32
agagctacga gctgcctgac                                                    20

<210> 33
<211> 20
<212> DNA
<213> Artificial Sequence

<220>
<223> Primer

<400> 33
acatctgctg gaaggtggac                                                    20

<210> 34
<211> 24
<212> DNA
<213> Artificial Sequence

<220>
<223> Primer

<400> 34
tgccacagta cttgaagaaa cgat                                               24

<210> 35
<211> 20
<212> DNA
<213> Artificial Sequence

<220>
<223> Primer

<400> 35
tgaagaacat tgggaggatt                                                    20

<210> 36
<211> 23
<212> DNA
<213> Artificial Sequence

<220>
<223> Primer

<400> 36
caatgaagta ggagggtatg agg                                                23

<210> 37
<211> 23
<212> DNA
<213> Artificial Sequence

<220>
<223> Primer

<400> 37
tggcgctgtt gaagatggag gtc                                              23

<210> 38
<211> 22
<212> DNA
<213> Artificial Sequence

<220>
<223> Primer

<400> 38
atctctaatg tccagcaatg tg                                               22

<210> 39
<211> 22
<212> DNA
<213> Artificial Sequence

<220>
<223> Primer

<400> 39
gcaatcatca gcccccgcag ac                                               22

<210> 40
<211> 20
<212> DNA
<213> Artificial Sequence

<220>
<223> Primer

<400> 40
atcctgactg ggtttgcttt                                                  20

<210> 41
<211> 20
<212> DNA
<213> Artificial Sequence

<220>
<223> Primer

<400> 41
atgctgatgc caatcagcac                                                  20

<210> 42
<211> 21
<212> DNA
<213> Artificial Sequence

<220>
<223> Primer

<400> 42
ggcaatggaa ccaggagtgt c                                                21

<210> 43
<211> 25
<212> DNA
<213> Artificial Sequence

<220>
<223> Primer

<400> 43
tcacgcaaaa tagccccaga gaaag                                          25

<210> 44
<211> 22
<212> DNA
<213> Artificial Sequence

<220>
<223> Primer

<400> 44
atggacagta gcaccttgag cc                                             22

<210> 45
<211> 22
<212> DNA
<213> Artificial Sequence

<220>
<223> Primer

<400> 45
tcaggcaaaa taggcatggc ag                                             22

<210> 46
<211> 22
<212> DNA
<213> Artificial Sequence

<220>
<223> Primer

<400> 46
caatggaacc tggagcttca ag                                             22

<210> 47
<211> 25
<212> DNA
<213> Artificial Sequence

<220>
<223> Primer

<400> 47
tcacgcaaaa tagccccaga gaaag                                          25

<210> 48
<211> 22
<212> DNA
<213> Artificial Sequence

<220>
<223> Primer

<400> 48
atggacagta gcaccttgag cc                                             22

<210> 49
<211> 22
<212> DNA
<213> Artificial Sequence

<220>
<223> Primer

<400> 49

tcaggcaaaa taggcgtggc ag                                                22

<210> 50
<211> 681
<212> PRT
<213> Mesocricetus auratus

<400> 50
Met Glu Pro Gly Asp Ser Gly Asp Ala Val Ser Ala Glu Ala Ala Pro
                5                   10                  15
His Leu Ala Leu Asp Ser Gly Val Ser Leu His Ala Tyr Asp Ile Leu
            20                  25                  30
Val Val Val Ile Tyr Phe Val Phe Val Leu Ala Val Gly Ile Trp Ser
        35                  40                  45
Ser Val Arg Ala Ser Arg Gly Thr Ile Gly Gly Tyr Phe Leu Ala Gly
    50                  55                  60
Arg Ser Met Thr Trp Trp Pro Ile Gly Ala Ser Leu Met Ser Ser Asn
65                  70                  75                  80
Val Gly Ser Gly Leu Phe Ile Gly Leu Ala Gly Thr Gly Ala Ala Gly
                85                  90                  95
Gly Leu Ala Val Gly Gly Phe Glu Trp Asn Ala Thr Trp Leu Leu Leu
            100                 105                 110
Ala Leu Gly Trp Ile Phe Val Pro Val Tyr Ile Ala Ala Gly Val Val
        115                 120                 125
Thr Met Pro Gln Tyr Leu Lys Lys Arg Phe Gly Gly Gln Arg Ile Gln
    130                 135                 140
Val Tyr Met Ser Val Leu Ser Leu Ile Leu Tyr Ile Phe Thr Lys Ile
145                 150                 155                 160
Ser Thr Asp Ile Phe Ser Gly Ala Ile Phe Ile Gln Met Ala Leu Gly
                165                 170                 175
Trp Asn Leu Tyr Leu Ser Thr Val Ile Leu Leu Val Val Thr Ala Val
            180                 185                 190
Tyr Thr Ile Ala Gly Gly Leu Thr Ala Val Ile Tyr Thr Asp Ala Leu
        195                 200                 205
Gln Thr Val Ile Met Val Gly Gly Ala Leu Val Leu Met Phe Leu Gly
    210                 215                 220
Phe Gln Glu Val Gly Trp Tyr Pro Gly Leu Gln Gln Leu Tyr Lys Gln
225                 230                 235                 240
Ala Ile Pro Asn Val Thr Val Pro Asn Thr Thr Cys His Leu Pro Arg
                245                 250                 255
Pro Asp Ala Phe His Met Leu Arg Asp Pro Val Asn Gly Asp Ile Pro
            260                 265                 270
Trp Pro Gly Leu Ile Phe Gly Leu Thr Val Leu Ala Thr Trp Cys Trp
        275                 280                 285
Cys Thr Asp Gln Val Ile Val Gln Arg Ser Leu Ser Ala Lys Ser Leu
    290                 295                 300
Ser His Ala Lys Gly Gly Ser Val Leu Gly Gly Tyr Leu Lys Ile Leu
305                 310                 315                 320
Pro Met Phe Phe Ile Val Met Pro Gly Met Ile Ser Arg Ala Leu Tyr
                325                 330                 335
Pro Asp Glu Val Ala Cys Val Asn Pro Asp Ile Cys Gln Arg Val Cys
            340                 345                 350
Gly Ala Arg Val Gly Cys Ser Asn Ile Ala Tyr Pro Lys Leu Ile Met
        355                 360                 365
Ala Leu Met Pro Val Gly Leu Arg Gly Leu Met Ile Ala Val Ile Met
    370                 375                 380
Ala Ala Leu Met Ser Ser Leu Thr Ser Ile Phe Asn Ser Ser Ser Thr
385                 390                 395                 400
Leu Phe Val Ile Asp Val Trp Gln Arg Phe Arg Lys Gln Ala Thr Glu
                405                 410                 415
Gln Glu Leu Met Val Val Gly Arg Leu Phe Ile Val Phe Leu Val Val
            420                 425                 430
Ile Ser Ile Leu Trp Ile Pro Ile Ile Gln Ser Ser Asn Ser Gly Gln
        435                 440                 445
Leu Phe Asp Tyr Ile Gln Ser Ile Thr Ser Tyr Leu Ala Pro Pro Ile
    450                 455                 460
Thr Ala Leu Phe Leu Leu Ala Ile Phe Ser Lys Arg Val Thr Glu Pro
465                 470                 475                 480

Gly Ala Phe Trp Gly Leu Thr Leu Gly Leu Ala Val Gly Ile Val Arg
                485                 490                 495
Met Ile Leu Glu Phe Ser Tyr Pro Ala Pro Ala Cys Gly Glu Met Asp
            500                 505                 510
Arg Arg Pro Ala Val Leu Arg Asp Val His Tyr Leu Tyr Phe Ala Leu
        515                 520                 525
Leu Leu Cys Gly Leu Ser Ala Ile Ile Thr Val Ile Ile Ser Phe Cys
    530                 535                 540
Thr Glu Pro Ile Pro Asp Glu Lys Leu Ala Arg Leu Thr Trp Trp Thr
545                 550                 555                 560
Arg Asn Cys Pro Leu Pro Glu Val Glu Lys Arg Ala Ser Val Ser Gly
                565                 570                 575
Asp Met Glu Gly Glu Asn Thr Pro Gly Leu Ala Gly Thr Pro Ala Val
            580                 585                 590
Glu Gly Pro Ser Gly Asp Gly Glu Glu Ala Arg Pro Thr Gln Gly Pro
        595                 600                 605
Glu Lys Pro Arg Ala Gln His Arg Ser Trp Gly Lys Trp Leu Trp Ser
    610                 615                 620
Trp Phe Cys Gly Leu Ser Gly Ala Pro Gln Gln Ala Leu Ser Ala Ala
625                 630                 635                 640
Glu Lys Ala Ala Leu Glu Lys Lys Leu Thr Ser Ile Glu Glu Glu Pro
                645                 650                 655
Leu Trp Arg His Val Cys Asn Ile Asn Ala Ile Ile Leu Leu Ala Ile
            660                 665                 670
Asn Ile Phe Leu Trp Gly Tyr Phe Ala
        675                 680

<210> 51
<211> 2046
<212> DNA
<213> Mesocricetus auratus

<400> 51
atggagcctg gagattcagg ggatgcagtc agcgctgagg cagcaccaca cttggcactg      60
gactctggag tcagcctgca tgcctatgac atcctggtgg tggtcatcta ctttgtcttc     120
gtccttgctg tggggatctg gtcatctgtc cgtgcaagca gagggaccat tggtggctat     180
ttcctggctg ggagatccat gacttggtgg ccaatcggag catcgctgat gtccagcaat     240
gtgggcagtg gcttgttcat cggcctggct gggacagggg ctgctggagg ccttgctgtg     300
ggtggcttcg agtggaatgc aacctggctg ctcttggctc tgggctggat ctttgtccct     360
gtgtacatcg ctgctggtgt ggtcaccatg ccacagtact tgaagaaacg atttggagga     420
cagaggatcc aggtgtatat gtcagtcctg tctctcatcc tctacatctt caccaagata     480
tcgactgaca tcttctctgg agcaatcttt atccagatgg ccttaggttg gaacctctat     540
ctctccacag tgatcttgct ggtggtgaca gctgtctaca ccattgcagg agggctcact     600
gctgtgatct acacagatgc tctacagacc gtcatcatgg tagggggagc actggtcctc     660
atgttcttgg gttttcaaga ggtgggctgg tacccaggcc tgcagcagct atataagcaa     720
gccattccca acgtcacagt tcccaacacc acttgtcacc tcccacggcc tgatgccttc     780
cacatgcttc gtgatcctgt gaatggggac atcccatggc caggtttaat ttttggactc     840
acagtcctgg caacctggtg ttggtgcaca gaccaggtga ttgtgcagag gtctctctcc     900
gccaagagtc tttcccatgc caaggggggc tcagtgctgg gaggctacct aaaaatcctc     960
ccaatgttct tcattgtcat gcctggcatg atcagccggg ctctgtaccc agatgaagtt    1020
gcctgtgtaa accctgacat ctgtcaaaga gtgtgtgggg ccagagtggg atgctccaac    1080
attgcctacc caaagctgat catggctctc atgcccgtgg gtctaagggg tctgatgatc    1140
gctgtgatca tggctgccct gatgagctca ctcacctcca tcttcaacag cagtagcacc    1200
ctatttgtca tagatgtgtg gcagcgcttc cgcaagcagg caacggaaca agagttgatg    1260
gtggtaggca ggttgttcat agtcttccta gtagtcatca gcatcctctg gatccccatc    1320
atccagagct ccaacagtgg gcagctcttt gactacatcc aatctatcac cagctaccta    1380
gccccaccca tcacagccct cttcctgctg gccatcttca gcaaaagggt cactgagcct    1440
ggtgccttct ggggcctcac tttgggccta gcagtgggaa tagtgcgcat gatccttgaa    1500
ttctcatatc ctgccccagc ctgtggggag atggacagga ggcctgctgt cctgagagac    1560
gtccactacc tgtattttgc cctcctcctc tgtggacttt ctgccatcat cactgtcata    1620
atcagtttct gcacagagcc catccctgat gaaaagcttg ctcgcttgac ctggtggacg    1680
aggaactgcc ccttacctga agtggagaag agagcctctg tgagtgggga catggagggg    1740
gaaaacactc cagggctggc agggacacca gctgtggagg gcccctcagg agatggagaa    1800
gaagcaagac ccacccaggg gcctgaaaaa ccaagagccc agcataggtc ttgggggaaa    1860
tggctgtgga gctggttctg cggactctca ggggccccac agcaagccct gagcgcagct    1920
gagaaggctg cattggagaa gaagctgacc agcatcgagg aggagcccct ctggagacat    1980
gtctgcaaca tcaatgccat catcctgctg gccatcaaca tctttctctg gggctatttt    2040
gcgtga                                                                2046

<210> 52
<211> 657
<212> PRT
<213> Mesocricetus auratus

<400> 52
Met Asp Ser Ser Thr Leu Ser Pro Ala Val Thr Ala Thr Asp Ser Pro
                5                   10                  15
Ile Pro Ser Tyr Glu Arg Ile Arg Asn Ala Ala Asp Ile Ser Val Ile
            20                  25                  30
Val Ile Tyr Phe Val Val Val Met Ala Val Gly Leu Trp Ala Met Phe
        35                  40                  45
Ser Thr Asn Arg Gly Thr Val Gly Gly Phe Phe Leu Ala Gly Arg Ser
    50                  55                  60
Met Val Trp Trp Pro Ile Gly Ala Ser Leu Phe Ala Ser Asn Ile Gly
65                  70                  75                  80
Ser Gly His Phe Val Gly Leu Ala Gly Thr Gly Ala Ala Ser Gly Ile
                85                  90                  95
Ala Met Gly Gly Phe Glu Trp Asn Ala Leu Ile Phe Val Val Val Leu
            100                 105                 110
Gly Trp Ile Phe Val Pro Ile Tyr Ile Arg Ala Gly Val Val Thr Met
        115                 120                 125
Pro Glu Tyr Leu Arg Lys Arg Phe Gly Gly Lys Arg Ile Gln Ile Tyr
    130                 135                 140
Leu Ser Ile Leu Ser Leu Leu Leu Tyr Ile Phe Thr Lys Ile Ser Ala
145                 150                 155                 160
Asp Ile Phe Ser Gly Ala Ile Phe Ile Asn Leu Ala Leu Gly Leu Asp
                165                 170                 175
Ile Tyr Leu Ala Ile Phe Ile Leu Leu Ala Ile Thr Ala Leu Tyr Thr
            180                 185                 190
Ile Thr Gly Gly Leu Ala Ala Val Ile Tyr Thr Asp Ala Leu Gln Thr
        195                 200                 205
Ala Ile Met Leu Val Gly Ser Ile Ile Leu Thr Ala Phe Ala Phe Asn
    210                 215                 220
Glu Val Gly Gly Tyr Glu Ala Phe Val Glu Lys Tyr Met Lys Ala Ile
225                 230                 235                 240
Pro Ser Met Ile Ser Asp Gly Asn Leu Thr Ile Lys Glu Glu Cys Tyr
                245                 250                 255
Thr Pro Lys Glu Asp Ser Phe His Ile Phe Arg Asp Pro Ile Lys Gly
            260                 265                 270
Asp Ile Pro Trp Pro Gly Leu Ile Phe Gly Leu Ser Ile Leu Ala Leu
        275                 280                 285
Trp Tyr Trp Cys Thr Asp Gln Val Ile Val Gln Arg Cys Leu Ser Ala
    290                 295                 300
Lys Asn Met Ser His Val Lys Ala Gly Cys Thr Leu Cys Gly Tyr Leu
305                 310                 315                 320
Met Val Met Thr Gly Met Val Ser Arg Ile Leu Tyr Thr Asp Lys Ile
                325                 330                 335
Ala Cys Val Val Pro Ser Glu Cys Lys Lys Tyr Cys Gly Thr Ser Val
            340                 345                 350
Gly Cys Thr Asn Ile Ala Tyr Pro Thr Leu Val Val Glu Leu Met Pro
        355                 360                 365
Asp Gly Leu Arg Gly Leu Met Leu Ser Val Met Met Ala Ser Leu Met
    370                 375                 380
Ser Ser Leu Thr Ser Ile Phe Asn Ser Ala Ser Thr Leu Phe Thr Met
385                 390                 395                 400
Asp Ile Tyr Thr Lys Ile Arg Lys Arg Ala Ser Glu Arg Glu Leu Met
                405                 410                 415
Ile Ala Gly Arg Leu Phe Met Leu Leu Leu Ile Ala Ile Ser Ile Ala
            420                 425                 430
Trp Val Pro Ile Val Gln Ser Ala Gln Ser Gly Gln Leu Phe Asp Tyr
        435                 440                 445
Ile Gln Ser Ile Thr Ser Tyr Leu Gly Pro Pro Ile Gly Ala Val Phe
    450                 455                 460
Leu Leu Ala Ile Phe Cys Lys Arg Val Asn Glu Gln Gly Ala Phe Trp
465                 470                 475                 480
Gly Leu Ile Leu Gly Phe Phe Ile Gly Val Ala Arg Met Ile Thr Glu

485                 490                 495
Phe Ala Tyr Gly Thr Gly Ser Cys Met Glu Pro Ser Asn Cys Pro Thr
            500                 505                 510
Ile Ile Cys Gly Val His Tyr Leu Tyr Phe Ala Ile Ile Leu Phe Val
        515                 520                 525
Ile Cys Val Ile Thr Ile Leu Thr Val Ser Phe Leu Thr Lys Pro Ile
    530                 535                 540
Pro Asp Val His Leu Tyr Arg Leu Cys Trp Ser Leu Arg Asn Ser Lys
545                 550                 555                 560
Glu Glu Arg Ile Asp Leu Asp Ala Gly Asp Glu Glu Thr Trp Glu Asp
                565                 570                 575
Ser Lys Asp Thr Ile Glu Ile Asp Thr Glu Ala Pro Gln Lys Glu Lys
            580                 585                 590
Gly Cys Phe Arg Arg Ala Tyr Asp Met Phe Cys Gly Leu Asp Gln Asp
        595                 600                 605
Lys Gly Pro Lys Met Thr Lys Glu Glu Glu Glu Ala Met Lys Leu Lys
    610                 615                 620
Met Thr Asp Thr Ser Glu Gln Pro Leu Trp Arg Thr Val Val Asn Ile
625                 630                 635                 640
Asn Gly Ile Ile Leu Leu Ala Val Ala Val Phe Cys His Gly Tyr Phe
                645                 650                 655
Ala

<210> 53
<211> 1974
<212> DNA
<213> Mesocricetus auratus

<400> 53
atggacagta gcaccttgag ccccgcggtc acggccacag attcacccat cccgtcttat      60
gaacgcattc gcaatgctgc tgacatctca gtcattgtca tctacttcgt ggtggtgatg     120
gctgtcgggc tgtgggcgat gttttccact aatcgtggga ctgttggagg cttcttcctc     180
gcaggccgaa gtatggtgtg gtggccaatt ggagcctctc tctttgccag taacattgga     240
agcggtcact ttgtgggact ggcagggacg ggagcagcct caggcattgc catgggtggc     300
tttgaatgga atgccttgat tttcgtggtg gtgctgggct ggatattcgt ccctatttac     360
atcagggctg gggtggtgac gatgccggag tatctacgga agcggtttgg aggcaagcga     420
atccagatct acctttccat tctgtccttg ttgctttaca tttttaccaa gatctcagca     480
gacatcttct ctggagctat atttatcaat ctagccttgg gtctggatat atacttggcc     540
atctttatcc tactagcaat cactgccctt tatacaatca cagggggcct ggcagcggtg     600
atctacacag atgccttgca gaccgcaatc atgctggtgg ggtctattat cctgaccgca     660
tttgctttca atgaagtagg agggtatgag gcatttgttg agaagtacat gaaagccatt     720
ccaagtatga tttctgatgg aaatctgacc atcaaggaag aatgttacac tcccaaggag     780
gactcgttcc atatattccg agatcctatt aagggagaca ttccatggcc tgggctcatc     840
tttggcctgt ccatcctcgc cctgtggtac tggtgcacag accaggtcat cgtgcagcgc     900
tgcctctcag ctaagaacat gtctcacgtg aaggccggct gtaccctgtg cggctatctg     960
atggtgatga cgggaatggt cagccggatt ctgtacacag acaaaatcgc ctgtgtcgtc    1020
ccctcggaat gtaagaaata ctgtggtacc tcagttggct gcaccaacat tgcctatcca    1080
accttggtgg tggagctcat gcctgatgga cttcgaggcc tgatgttgtc agtcatgatg    1140
gcctcactca tgagctcttt gacctccatc ttcaacagcg ccagcaccct ctttaccatg    1200
gacatctaca ccaagatccg gaagagagca tctgagaggg agctcatgat tgcaggaagg    1260
ttgttcatgc tcctgctgat tgccatcagc atcgcctggg tgcccatcgt gcagtcggct    1320
caaagtggac agctctttga ttacatccag tctatcacca gctacttggg gccacccatt    1380
ggggctgtct tcctgctggc tattttctgc aagagagtca atgaacaagg agccttctgg    1440
ggactgatcc taggcttctt tattggggtc gcccgtatga tcaccgagtt tgcctatgga    1500
actgggagct gcatggagcc cagcaactgc cccacgatca tctgtggggt ccactatttg    1560
tactttgcca tcatcctctt tgttatctgt gtaatcacca tcttgaccgt ctccttcctc    1620
accaagccca ttccagatgt gcacctgtac cgtctgtgtt ggagtctacg caacagcaaa    1680
gaagaacgga tcgacctgga tgccggagat gaggaaacct gggaagactc taaggacaca    1740
attgagatag acacagaggc tccccaaaag gagaaaggat gtttcaggag ggcatatgac    1800
atgttctgtg gcctcgacca ggacaaagga ccaaagatga ctaaggaaga ggaggaagcc    1860
atgaagctga agatgacaga cacatctgag cagcctttgt ggaggacggt ggtaaacatc    1920
aatggcatca tcctgttggc tgtggctgtc ttttgccacg gatattttgc ttga          1974

<210> 54
<211> 22
<212> DNA
<213> Artificial Sequence

<220>
<223> Primer

<400> 54
gcaatggagc ctggagattc ag                                                22

<210> 55
<211> 18
<212> DNA
<213> Artificial Sequence

<220>
<223> Primer

<400> 55
agcctgcctc tggtcttg                                                     18

<210> 56
<211> 31
<212> DNA
<213> Artificial Sequence

<220>
<223> Primer

<400> 56
atggacagta gcaccttgag ccccgcggtc a                                      31

<210> 57
<211> 29
<212> DNA
<213> Artificial Sequence

<220>
<223> Primer

<400> 57
gattcaagca aaatatccgt ggcaaaaga                                         29

## Description

### TECHNICAL FIELD

The present invention relates to a glucose uptake regulator (inhibitor or promoter) in the small intestine, comprising a compound or its salts that regulate (inhibit or promote) the activity of a Na⁺/glucose transporter (SGLT) homolog or the expression of a gene for the homolog; a method of screening a compound or its salts that regulate the glucose uptake activity of said homolog in the small intestine; a compound or its salts obtainable by the screening method; a pharmaceutical comprising the compound or salts thereof; etc.

### BACKGROUND ART

For intracellular or extracellular translocation of glucose, membrane proteins called glucose transporters must be present on cell membranes.

Glucose transporters are roughly classified into passive transporters or facilitative-diffusion glucose transporters (GLUT) and active transporters or Na⁺/glucose transporters (SGLT), which are coupled to Na⁺ ion transportation to transport glucose against its concentration gradient. GLUT has eight isoforms that share a common structure to traverse the cell membrane of about 50,000 molecular weight 12 times.

SGLT shares a common structure to traverse the cell membrane of 75,000 molecular weight 14 times.

The functions and expression sites of SGLTs 1 and 2 are outlined in Nippon Rinsho (Japanese Clinical), 55: 1997, extra number; Diabetes, I: 59-64.

It is known that human SGLT1 is expressed specifically in the small intestine and the kidney, and has a high affinity to glucose and a low transport activity, while human SGLT2 is expressed specifically in kidney, and has a low affinity to glucose and a high transport activity. SGLTs undertake the role of absorbing glucose in the small intestine and reabsorbing glucose in the kidney, which has been once excreted into the urine.

It is shown in a diabetes model rat that in consequence of inhibiting glucose reabsorption in the kidney by inhibiting SGLT, glucose is excreted in the urine to decrease the blood glucose level (Diabetes, 48: 1794-1800, 1999).

The SGLT homologs are proteins disclosed in WO 02/53738 and expressed in kidney. By activation of the SGLT homologs, the homologs act to inhibit gluconeogenesis and then correct fasting hyperglycemia. It is thus considered that the SGLT homologs will be available as antidiabetic agents.

On the other hand, postprandial hyperglycemia in diabetes occurs decreased insulin secretion concurrently with an increase of blood sugar level after a meal, in combination with insulin resistance in the liver and muscles. An α-glucosidase inhibitor is known to correct postprandial hyperglycemia. The α-glucosidase inhibitor suppresses the digestion of polysaccharides to monosaccharides thereby to delay glucose absorption rate from the intestinal tract. However, the α-glucosidase inhibitor cannot reduce glucose absorption during meals and hence is less effective in reducing the HbAlc value as an indicator for long-range blood sugar level. Moreover, the α-glucosidase inhibitor causes occasional side effects of watery diarrhea, abdomen enlarged feeling due to sucrose or maltose remained undigested in the small intestine.

### DISCLOSURE OF INVENTION

When SGLT1 believed to take part chiefly in absorbing sugar in the small intestine is inhibited to reduce glucose absorption during meals, it can be expected that SGLT1 would improve postprandial hyperglycemia more potently than the α-glucosidase inhibitor. In addition, due to modest fluid retention of monosaccharide glucose as compared to disaccharides, it is expected that the side effects of gastrointestinal symptoms can be alleviated.

However, phlorizin and its derivatives, which are inhibitors of SGLT1, are shown to block SGLT in rat thereby to suppress reabsorption of glucose in kidney so that glucose is secreted into urine to lower blood sugar levels (Diabetes 48: 1794-1800, 1999) but their glucose absorption suppressing effects through the intestinal tract are reportedly poor (Journal of Medicinal Chemistry 42: 5311-5324, 1999).

This is believed to be because other SGLT strongly resistant to phlorizin may be present in the small intestine (Am. J. Physiol. 256: G618-G623, 1989, Am. J. Physiol. 270: G833-G843, 1996) but its entity remains unclear to date. It is the problem to clarify phlorizin-resistant SGLT and provide a method of screening a compound having the effect of suppressing glucose absorption from the intestinal tract by applying the SGLT inhibitory action and a compound obtainable by the screening method.

Accordingly, it is the current situation that development of drugs capable of specifically regulating (inhibiting or promoting) glucose uptake in the small intestine has been awaited.

In order to solve the foregoing problem, the present inventors made search for Gene Logic database and thus found that the human SGLT homolog which is a Na⁺/glucose transporter protein is expressed in the small intestine approximately twice human SGLT1. Based on the finding, further investigations were made and as a result, found out that the SGLT homolog is an important transporter for absorption of glucose in the small intestine. Extensive investigations have been further made, on the assumption that inhibition of the SGLT homolog would lead to an effective antidiabetic drug for suppressing the increase of blood sugar level after meals and its promotion will result in an effective antihypoglycemic agent for promoting the absorption of glucose or a digestive drug. As a result, the present invention has come to be accomplished.

That is, the present invention relates to the following features, etc.
(1) A glucose uptake inhibitor in the small intestine comprising a compound or a salt thereof that inhibits the activity of a Na⁺/glucose transporter (SGLT) homolog.
(2) A glucose uptake inhibitor in the small intestine comprising a compound or a salt thereof that inhibits the expression of a gene for Na⁺/glucose transporter (SGLT) homolog.
(3) The inhibitor according to (1) or (2), which is a postprandial hyperglycemia-improving agent.
(4) The inhibitor according to (1) through (3), which is an agent for the prevention/treatment of diabetes or hyperlipemia.
(5) A glucose uptake promoter in the small intestine comprising a compound or a salt thereof that promotes the activity of a Na⁺/glucose transporter (SGLT) homolog.
(6) A glucose uptake promoter in the small intestine comprising a compound or a salt thereof that promotes the expression of a gene for Na⁺/glucose transporter (SGLT) homolog.
(7) The promoter according to (5) or (6), which is a glucose absorption promoter.
(8) The agent according to (1) through (7), wherein the Na⁺/glucose transporter (SGLT) homolog is a protein comprising the same or substantially the same amino acid sequence as the amino acid sequence represented by SEQ ID NO: 1, its partial peptide, or a salt thereof.
(9) The agent according to (1) through (7), wherein the Na⁺/glucose transporter (SGLT) homolog is a protein comprising the same or substantially the same amino acid sequence as the amino acid sequence represented by SEQ ID NO: 3, its partial peptide, or a salt thereof.
(10) The agent according to (1) through (7), wherein the Na⁺/glucose transporter (SGLT) homolog is a protein comprising the same or substantially the same amino acid sequence as the amino acid sequence represented by SEQ ID NO: 5 or SEQ ID NO: 50, its partial peptide, or a salt thereof.
(11) A glucose uptake inhibitor in the small intestine comprising an antisense polynucleotide comprising the entire or part of a base sequence complementary or substantially complementary to a base sequence of a polynucleotide encoding a Na⁺/glucose transporter (SGLT) homolog.
(12) The inhibitor according to (11), which is a postprandial hyperglycemia-improving agent.
(13) The inhibitor according to (11) or (12), which is an agent for the prevention/treatment of diabetes or hyperlipemia.
(14) The inhibitor according to (11) through (13), wherein the polynucleotide encoding the Na⁺/glucose transporter (SGLT) homolog is a polynucleotide comprising the same or substantially the same base sequence as the base sequence represented by SEQ ID NO: 2, SEQ ID NO: 4, SEQ ID NO: 6 or SEQ ID NO: 51.
(15) A glucose uptake inhibitor in the small intestine comprising an antibody to a Na⁺/glucose transporter (SGLT) homolog.
(16) The inhibitor according to (15), which is a postprandial hyperglycemia-improving agent.
(17) The inhibitor according to (15) or (16), which is an agent for the prevention/treatment of diabetes or hyperlipemia.
(18) The inhibitor according to (15) through (17), wherein the Na⁺/glucose transporter (SGLT) homolog is a protein comprising the same or substantially the same amino acid sequence as the amino acid sequence represented by SEQ ID NO: 1, SEQ ID NO: 3, SEQ ID NO: 5 or SEQ ID NO: 50, its partial peptide, or a salt thereof.
(19) A diagnostic agent for postprandial hyperglycemia comprising an antibody to a Na⁺/glucose transporter (SGLT) homolog.
(20) A diagnostic agent for postprandial hyperglycemia comprising a polynucleotide encoding a Na⁺/glucose transporter (SGLT) homolog.
(21) A method of screening a compound or its salt that regulates the glucose uptake activity of a Na⁺/glucose transporter (SGLT) homolog in the small intestine, which comprises using the homolog.
(22) The screening method according to (21), wherein the Na⁺/glucose transporter (SGLT) homolog is a protein comprising the same or substantially the same amino acid sequence as the amino acid sequence represented by SEQ ID NO: 1, SEQ ID NO: 3, SEQ ID NO: 5 or SEQ ID NO: 50, its partial peptide, or a salt thereof.
(23) A kit for screening a compound or its salt that regulates the glucose uptake activity of a Na⁺/glucose transporter (SGLT) homolog in the small intestine, comprising the homolog.
(24) A compound or its salt, which is obtainable using the screening method according to (21) or (22) or the screening kit according to (23).
(25) A pharmaceutical comprising the compound or its salt according to (24).
(26) The pharmaceutical according to (25), which is a postprandial hyperglycemia-improving agent.
(27) The pharmaceutical according to (25) or (26), which is an agent for the prevention/treatment of diabetes or hyperlipemia.
(28) A method of screening a compound or its salt that regulates the glucose uptake activity of a Na⁺/glucose transporter (SGLT) homolog in the small intestine, which comprises using a polynucleotide encoding the homolog.
(29) The screening method according to (28), wherein the polynucleotide encoding the Na⁺/glucose transporter (SGLT) homolog is a polynucleotide comprising the same or substantially the same base sequence as the base sequence represented by SEQ ID NO: 2, SEQ ID NO: 4, SEQ ID NO: 6 or SEQ ID NO: 51.
(30) A kit for screening comprising a compound or its salt that regulates the glucose uptake activity of a Na⁺/glucose transporter (SGLT) homolog in the small intestine, which comprises using a polynucleotide encoding the homolog.
(31) A compound or its salt, which is obtainable using the screening method according to (28) or (29) or the screening kit according to (30).
(32) A pharmaceutical comprising the compound or its salt according to (31).
(33) The pharmaceutical according to (32), which is a postprandial hyperglycemia-improving agent.
(34) The pharmaceutical according to (32) or (33), which is an agent for the prevention/treatment of diabetes or hyperlipemia.
(35) A method of inhibiting glucose uptake in the small intestine, which comprises inhibiting the activity of a Na⁺/glucose transporter (SGLT) homolog.
(36) A method of inhibiting glucose uptake in the small intestine, which comprises inhibiting the expression of a gene for Na⁺/glucose transporter (SGLT) homolog.
(37) The method according to (35) or (36), which is a method of improving postprandial hyperglycemia.
(38) The method according to (35) through (37), which is a method for the prevention/treatment of diabetes or hyperlipemia.
(39) A method of promoting glucose uptake in the small intestine, which comprises promoting the activity of a Na⁺/glucose transporter (SGLT) homolog.
(40) A method of promoting glucose uptake in the small intestine, which comprises promoting the expression of a gene for Na⁺/glucose transporter (SGLT) homolog.
(41) The method according to (39) or (40), which is a method of promoting glucose absorption.
(42) A method of inhibiting glucose uptake in the small intestine, which comprises administering to a mammal an effective dose of a compound or its salt that inhibits the activity of a Na⁺/glucose transporter (SGLT) homolog.
(43) A method of inhibiting glucose uptake in the small intestine, which comprises administering to a mammal an effective dose of a compound or its salt that inhibits the expression of a gene for Na⁺/glucose transporter (SGLT) homolog.
(44) The method according to (42) or (43), which is a method of improving postprandial hyperglycemia.
(45) The method according to (42) through (44), which is a method for the prevention/treatment of diabetes or hyperlipemia.
(46) A method of promoting glucose uptake in the small intestine, which comprises administering to a mammal an effective dose of a compound or its salt that promotes the activity of a Na⁺/glucose transporter (SGLT) homolog.
(47) A method of promoting glucose uptake in the small intestine, which comprises administering to a mammal an effective dose of a compound or its salt that promotes the expression of a gene for Na⁺/glucose transporter (SGLT) homolog.
(48) The method according to (46) or (47), which is a method of promoting glucose absorption.
(49) Use of a compound or its salt that inhibits the activity of a Na⁺/glucose transporter (SGLT) homolog to manufacture a glucose uptake inhibitor in the small intestine.
(50) Use of a compound or its salt that inhibits the expression of a gene for Na⁺/glucose transporter (SGLT) homolog to manufacture a glucose uptake inhibitor in the small intestine.
(51) The use according to (49) or (50), wherein the glucose uptake inhibitor in the small intestine is a postprandial hyperglycemia-improving agent.
(52) The use according to (49) through (51), wherein the glucose uptake inhibitor in the small intestine is an agent for the prevention/treatment of diabetes or hyperlipemia.
(53) Use of a compound or its salt that promotes the activity of a Na⁺/glucose transporter (SGLT) homolog to manufacture a glucose uptake promoter in the small intestine.
(54) Use of a compound or its salt that promotes the activity of a Na⁺/glucose transporter (SGLT) homolog to manufacture a glucose uptake promoter in the small intestine.
(55) The use according to (53) or (54), wherein the glucose uptake promoter in the small intestine is a glucose absorption promoter.

### BRIEF DESCRIPTION OF THE DRAWINGS

FIG. 1 is a graph showing distribution of the expressed human SGLT homolog in the gastrointestinal tract, which was obtained in EXAMPLE 2.
FIG. 2 is a graph showing expression analysis results of SGLT1 and the SGLT homolog in normal human small intestine epithelial cells in primary culture, which was obtained in EXAMPLE 3.
FIG. 3 shows photographs displaying the results of immunostained human small intestine slices, using human small intestine anti-human SGLT homolog antibody, which results were obtained in EXAMPLE 4.
FIG. 4 is a graph showing the results of change in expression of the SGLT homolog in diabetic mice, which were obtained in EXAMPLE 5.
FIG. 5 is a graph showing the results of change in expression of the SGLT homolog in diabetic rats, which were obtained in EXAMPLE 5.
FIG. 6 shows photographs displaying the results of comparison in the expression levels of SGLT1 and the SGLT homolog between the small intestines from human, mouse, rat, hamster and monkey, which results were obtained in EXAMPLE 6.
FIG. 7 is a graph showing the assay results of glucose uptake in the small intestines from human, mouse, rat, hamster and monkey by the organ culture system, which results were obtained in EXAMPLE 7.

### BEST MODE FOR CARRYING OUT THE INVENTION

The Na⁺/glucose transporter (SGLT) homologs used in the present invention (hereinafter sometimes referred to as the protein of the present invention or the protein used in the present invention) preferably include, for example, the SGLT homologs disclosed in WO02/53738, the SGLT homologs disclosed in WO01/75067, the SGLT homolog (TRICH) disclosed in WO01/92304, the SGLT homolog (TRICH) disclosed in WO02/4520, the SGLT homologs disclosed in WO02/10216, etc. Among them, the SGLT homologs disclosed in WO02/53738 are particularly preferred, and more preferably, a protein comprising the same or substantially the same amino acid sequence as the amino acid sequence represented by SEQ ID NO: 1, SEQ ID NO: 3, SEQ ID NO: 5 or SEQ ID NO: 50, is used.

The Na⁺/glucose transporter (SGLT) homologs may be any protein derived from any cells of human and warm-blooded animals (e.g., guinea pig, hamster, rat, mouse, fowl, rabbit, swine, sheep, bovine, monkey, etc.) such as hepatocyte, splenocyte, nerve cells, glial cells, β cells of pancreas, bone marrow cells, mesangial cells, Langerhans' cells, epidermic cells, epithelial cells, goblet cells, endothelial cells, smooth muscle cells, fibroblasts, fibrocytes, myocytes, fat cells, immune cells (e.g., macrophage, T cells, B cells, natural killer cells, mast cells, neutrophils, basophils, eosinophils, monocytes), megakaryocytes, synovial cells, chondrocytes, bone cells, osteoblasts, osteoclasts, mammary gland cells, hepatocytes or interstitial cells; or the corresponding precursor cells, stem cells, cancer cells, etc.; or any tissues where such cells are present, such as brain or any of brain regions (e.g., olfactory bulb, amygdaloid nucleus, basal ganglia, hippocampus, thalamus, hypothalamus, cerebral cortex, medulla oblongata, cerebellum), spinal cord, hypophysis, stomach, pancreas, kidney, liver, gonad, thyroid, gall-bladder, bone marrow, adrenal gland, skin, muscle, lung, gastrointestinal tract (e.g., large intestine and small intestine), blood vessel, heart, thymus, spleen, submandibular gland, peripheral blood, prostate, testis, ovary, placenta, uterus, bone, joint, skeletal muscle, etc.; the homologs may also be synthetic proteins.

In the specification, the term "substantially the same amino acid sequence" is used to mean an amino acid sequence having at least about 70% homology, preferably at least about 80% homology, more preferably at least about 90% homology and most preferably at least about 95% homology, to the amino acid sequence to be compared. Homology in the amino acid sequence can be measured under the following conditions (an expectation value = 10; gaps are allowed; matrix = BLOSUM62; filtering = OFF) using the homology scoring algorithm NCBI BLAST (National Center for Biotechnology Information Basic Local Alignment Search Tool).

Preferred examples of the protein comprising substantially the same amino acid sequence as the amino acid sequence represented by, e.g., SEQ ID NO: 1 include proteins having substantially the same amino acid sequence as the amino acid sequence represented by, e.g., SEQ ID NO: 1 and having an activity substantially equivalent to that of the protein having the amino acid sequence represented by SEQ ID NO: 1, etc.

As the substantially equivalent activities, there are, for example, an active glucose transport activity, and the like. The substantially equivalent is used to mean that the nature of these properties is equivalent in terms of property (e.g., physiologically or pharmacologically). Thus, the active glucose transport activity is preferably equivalent (e.g., about 0.01 to 100 times, preferably about 0.1 to 10 times, more preferably 0.5 to 2 times), but differences in degree such as a level of these activities, quantitative factors such as a molecular weight of the protein may be present and allowable.

These activities including the active glucose transport activity, etc. can be determined according to known methods, for example, the method described in "Cloning and functional expression of an SGLT-1-like protein from the Xenopus laevis intestine" (Am. J. Physiol., 276: G1251-G1259, 1999) or a modification thereof.

Examples of the protein used in the present invention include so-called muteins such as proteins comprising (i) an amino acid sequence represented by SEQ ID NO: 1, of which 1, 2 or more (e.g., about 1 to about 100, preferably about 1 to about 30, more preferably about 1 to about 10, and most preferably several (1 to 5)) amino acids are deleted; (ii) an amino acid sequence represented by SEQ ID NO: 1, to which 1, 2 or more (e.g., about 1 to about 100, preferably about 1 to about 30, more preferably about 1 to about 10, and most preferably several (1 to 5)) amino acids are added; (iii) an amino acid sequence represented by SEQ ID NO: 1, in which 1, 2 or more (e.g., about 1 to about 100, preferably about 1 to about 30, more preferably about 1 to about 10, and most preferably several (1 to 5)) amino acids are inserted; (iv) an amino acid sequence represented by SEQ ID NO: 1, in which 1, 2 or more (e.g., about 1 to about 100, preferably about 1 to about 30, more preferably about 1 to about 10, and most preferably several (1 to 5)) amino acids are substituted by other amino acids; or (v) a combination of the above amino acid sequences, and the like.

Where the amino acid sequence is inserted, deleted or substituted as described above, the position of its insertion, deletion or substitution is not particularly limited.

Throughout the specification, the proteins are represented in accordance with the conventional way of describing proteins, that is, the N-terminus (amino terminus) at the left hand and the C-terminus (carboxyl terminus) at the right hand. In the protein used in the present invention including the protein having the amino acid sequence represented by SEQ ID NO: 1, the C-terminus may be in any form of a carboxyl group (-COOH), a carboxylate (-COO-), an amide (-CONH₂) and an ester (-COOR).

Herein, examples of the ester group shown by R include a C₁₋₆ alkyl group such as methyl, ethyl, n-propyl, isopropyl, n-butyl, etc.; a C₃₋₈ cycloalkyl group such as cyclopentyl, cyclohexyl, etc.; a C₆₋₁₂ aryl group such as phenyl, α-naphthyl, etc.; a C₇₋₁₄ aralkyl such as a phenyl-C₁₋₂ alkyl group, e.g., benzyl, phenethyl, etc.; an α-naphthyl-C₁₋₂ alkyl group such as α-naphthylmethyl, etc.; pivaloyloxymethyl and the like.

Where the protein used in the present invention contains a carboxyl group (or a carboxylate) at a position other than the C-terminus, the carboxyl group may be amidated or esterified and such an amide or ester is also included within the protein used in the present invention. Examples of the ester group in this case may be the C-terminal esters described above, etc.

Furthermore, examples of the protein used in the present invention include variants wherein the amino group at the N-terminal amino acid residues (e.g., methionine residue) is protected with a protecting group (e.g., a C₁₋₆ acyl group such as a C₁₋₆ alkanoyl group, e.g., formyl group, acetyl group, etc.); those wherein the N-terminal region is cleaved in vivo and the glutamyl group thus formed is pyroglutaminated; those wherein a substituent (e.g., -OH, -SH, amino group, imidazole group, indole group, guanidino group, etc.) on the side chain of an amino acid in the molecule is protected with a suitable protecting group (e.g., a C₁₋₆ acyl group such as a C₁₋₆ alkanoyl group, e.g., formyl group, acetyl group, etc.), or conjugated proteins such as glycoproteins having sugar chains; etc.

Specific examples of the protein used in the present invention are a protein comprising the amino acid sequence represented by SEQ ID NO: 1, and the like.

The partial peptide of the protein used in the present invention may be any peptide as long as it is a partial peptide of the protein used in the present invention described above and preferably has the property equivalent to that of the protein used in the present invention described above.

The peptides which are preferably used include peptides having sequences of at least 20, preferably at least 50, more preferably at least 70, much more preferably at least 100, and most preferably at least 200, amino acids, in the constituent amino acid sequence of the protein used in the present invention, and the like.

The partial peptide used in the present invention may contain deletion of at least 1 or 2 (preferably about 1 to about 10 and more preferably several (1 to 5)) amino acids in the amino acid sequence; addition of at least 1 or 2 (preferably about 1 to about 20, more preferably about 1 to about 10 and most preferably several (1 to 5)) amino acids in the amino acid sequence; insertion of at least 1 or 2 (preferably about 1 to about 20, more preferably about 1 to about 10 and most preferably several (1 to 5)) amino acids in the amino acid sequence; or substitution of at least 1 or 2 (preferably about 1 to about 10, more preferably several and most preferably about 1 to about 5) amino acids in the amino acid sequence by other amino acids.

Preferably, the partial peptides of the present invention are peptides comprising, e.g., the 176-201 amino acid sequence, the 471-491 amino acid sequence, etc. in the amino acid sequence represented by SEQ ID NO: 1. In the amino acid sequence represented by SEQ ID NO: 3, preferred examples are peptides comprising the 172-197 amino acid sequence and the 467-487 amino acid sequence. In the amino acid sequence represented by SEQ ID NO: 5, preferred examples are peptides comprising the 175-200 amino acid sequence and the 470-490 amino acid sequence. In the amino acid sequence represented by SEQ ID NO: 50, preferred examples are peptides comprising the 176-201 amino acid sequence and the 471-491 amino acid sequence. In the partial peptide used in the present invention, the C-terminus may be in any form of a carboxyl group (-COOH), a carboxylate (-COO-), an amide (-CONH₂) or an ester (-COOR).

Furthermore, the partial peptide used in the present invention includes variants having a carboxyl group (or a carboxylate) at a position other than the C-terminus, those having an amino group protected with a protecting group at the N-terminal amino acid residues (e.g., methionine residue); those being cleaved at the N-terminal region in vivo and with the glutamyl group thus formed being pyroglutaminated; those having a substituent on the side chain of an amino acid in the molecule wherein the substituent is protected with a suitable protecting group, or conjugated peptides such as so-called glycopeptides having sugar chains; etc., as in the protein used in the present invention described above.

The partial peptide used in the present invention may also be used as an antigen for producing antibodies.

For the purpose of preparing the antibody of the present invention later described, examples include peptides comprising the 261-275 amino acid sequence, the 399-417 amino acid sequence, the 500-649 amino acid sequence, etc. in the amino acid sequence represented by SEQ ID NO: 1. In the amino acid sequence represented by SEQ ID NO: 3, examples include peptides comprising the 257-271 amino acid sequence, the 395-413 amino acid sequence, the 496-645 amino acid sequence, etc. In the amino acid sequence represented by SEQ ID NO: 5, examples include peptides comprising the 260-274 amino acid sequence, the 398-416 amino acid sequence, the 499-648 amino acid sequence, etc. In the amino acid sequence represented by SEQ ID NO: 50, examples include peptides comprising the 261-275 amino acid sequence, the 399-417 amino acid sequence, the 500-649 amino acid sequence, etc.

As salts of the protein or partial peptide used in the present invention, salts with physiologically acceptable acids (e.g., inorganic acids or organic acids) or bases (e.g., alkali metal salts) may be employed, preferably in the form of physiologically acceptable acid addition salts. Examples of such salts include salts with inorganic acids (e.g., hydrochloric acid, phosphoric acid, hydrobromic acid, sulfuric acid), salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid) and the like.

The protein or partial peptide used in the present invention or salts thereof may be manufactured by publicly known methods used to purify a protein from human or warm-blooded animal cells or tissues described above. Alternatively, they may also be manufactured by culturing transformants containing DNAs encoding these proteins. Furthermore, they may also be manufactured by a modification of the methods for peptide synthesis, which will be later described.

Where these proteins are manufactured from human or mammalian tissues or cells, human or mammalian tissues or cells are homogenized, extracted with an acid or the like, and the extract is purified/isolated by a combination of chromatography techniques such as reverse phase chromatography, ion exchange chromatography, and the like.

To synthesize the protein or partial peptide used in the present invention or its salts, or amides thereof, commercially available resins that are used for protein synthesis may be used. Examples of such resins include chloromethyl resin, hydroxymethyl resin, benzhydrylamine resin, aminomethyl resin, 4-benzyloxybenzyl alcohol resin, 4-methylbenzhydrylamine resin, PAM resin, 4-hydroxymethylmethylphenyl acetamidomethyl resin, polyacrylamide resin, 4-(2',4'-dimethoxyphenyl-hydroxymethyl)phenoxy resin, 4-(2',4'-dimethoxyphenyl-Fmoc-aminoethyl) phenoxy resin, etc. Using these resins, amino acids, in which α-amino groups and functional groups on the side chains are appropriately protected, are condensed on the resin in accordance with the sequence of the objective protein according to various condensation methods publicly known in the art. At the end of the reaction, the protein or partial peptide is excised from the resin and at the same time, the protecting groups are removed. Then, intramolecular disulfide bond-forming reaction is performed in a highly diluted solution to obtain the objective protein or partial peptide, or amides thereof.

For condensation of the protected amino acids described above, a variety of activation reagents for protein synthesis may be used, and carbodiimides are particularly employed. Examples of such carbodiimides include DCC, N,N'-diisopropylcarbodiimide, N-ethyl-N'-(3-dimethylaminopropyl)carbodiimide, etc. For activation by these reagents, the protected amino acids in combination with a racemization inhibitor (e.g., HOBt, HOOBt) are added directly to the resin, or the protected amino acids are previously activated in the form of symmetric acid anhydrides, HOBt esters or HOOBt esters, followed by adding the thus activated protected amino acids to the resin.

Solvents suitable for use to activate the protected amino acids or condense with the resin may be appropriately chosen from solvents that are known to be usable for protein condensation reactions. Examples of such solvents are acid amides such as N,N-dimethylformamide, N,N-dimethylacetamide, N-methylpyrrolidone, etc.; halogenated hydrocarbons such as methylene chloride, chloroform, etc.; alcohols such as trifluoroethanol, etc.; sulfoxides such as dimethylsulfoxide, etc.; ethers such as pyridine, dioxane, tetrahydrofuran, etc.; nitriles such as acetonitrile, propionitrile, etc.; esters such as methyl acetate, ethyl acetate, etc.; and appropriate mixtures of these solvents. The reaction temperature is appropriately chosen from the range known to be applicable to protein binding reactions and is usually selected in the range of approximately -20°C to 50°C. The activated amino acid derivatives are used generally in an excess of 1.5 to 4 times. The condensation is examined using the ninhydrin reaction; when the condensation is insufficient, the condensation can be completed by repeating the condensation reaction without removal of the protecting groups. When the condensation is yet insufficient even after repeating the reaction, unreacted amino acids are acetylated with acetic anhydride or acetylimidazole to avoid any possible effect on the subsequent reaction.

Examples of the protecting groups used to protect the starting amino groups include Z, Boc, t-pentyloxycarbonyl, isobornyloxycarbonyl, 4-methoxybenzyloxycarbonyl, Cl-Z, Br-Z, adamantyloxycarbonyl, trifluoroacetyl, phthaloyl, formyl, 2-nitrophenylsulphenyl, diphenylphosphinothioyl, Fmoc, etc.

A carboxyl group can be protected by, e.g., alkyl esterification (linear, branched or cyclic alkyl esterification of, e.g., methyl, ethyl, propyl, butyl, t-butyl, cyclopentyl, cyclohexyl, cycloheptyl, cyclooctyl, 2-adamantyl, etc.), aralkyl esterification (e.g., benzyl ester, 4-nitrobenzyl ester, 4-methoxybenzyl ester, 4-chlorobenzyl ester, benzhydryl ester, etc.), phenacyl esterification, benzyloxycarbonyl hydrazidation, t-butoxycarbonyl hydrazidation, trityl hydrazidation, or the like.

The hydroxyl group of serine can be protected through, for example, its esterification or etherification. Examples of groups appropriately used for the esterification include a lower (C₁₋₆) alkanoyl group, such as acetyl group, an aroyl group such as benzoyl group, and a group derived from carbonic acid such as benzyloxycarbonyl group, ethoxycarbonyl group, etc. Examples of a group appropriately used for the etherification include benzyl group, tetrahydropyranyl group, t-butyl group, etc.

Examples of groups for protecting the phenolic hydroxyl group of tyrosine include Bzl, Cl₂-Bzl 2-nitrobenzyl, Br-Z, t-butyl, etc.

Examples of groups used to protect the imidazole moiety of histidine include Tos, 4-methoxy-2,3,6-trimethylbenzenesulfonyl, DNP, benzyloxymethyl, Bum, Boc, Trt, Fmoc, etc.

Examples of the activated carboxyl groups in the starting material include the corresponding acid anhydrides, azides, activated esters [esters with alcohols (e.g., pentachlorophenol, 2,4,5-trichlorophenol, 2,4-dinitrophenol, cyanomethyl alcohol, p-nitrophenol, HONB, N-hydroxysuccimide, N-hydroxyphthalimide, HOBt)]. As the amino acids in which the amino groups are activated in the starting material, the corresponding phosphoric amides are employed.

To eliminate (split off) the protecting groups, there are used catalytic reduction under hydrogen gas flow in the presence of a catalyst such as Pd-black or Pd-carbon; an acid treatment with anhydrous hydrogen fluoride, methanesulfonic acid, trifluoromethanesulfonic acid, trifluoroacetic acid, or a mixture solution of these acids; a treatment with a base such as diisopropylethylamine, triethylamine, piperidine or piperazine; reduction with sodium in liquid ammonia, etc. The elimination of the protecting group by the acid treatment described above is carried out generally at a temperature of approximately -20°C to 40°C. In the acid treatment, it is efficient to add a cation scavenger such as anisole, phenol, thioanisole, m-cresol, p-cresol, dimethylsulfide, 1,4-butanedithiol, 1,2-ethanedithiol, etc. Furthermore, 2,4-dinitrophenyl group known as the protecting group for the imidazole of histidine is removed by a treatment with thiophenol. Formyl group used as the protecting group of the indole of tryptophan is eliminated by the aforesaid acid treatment in the presence of 1,2-ethanedithiol, 1,4-butanedithiol, etc. as well as by a treatment with an alkali such as a dilute sodium hydroxide solution, dilute ammonia, etc.

Protection of functional groups that should not be involved in the reaction of the starting materials, protecting groups, elimination of the protecting groups and activation of functional groups involved in the reaction may be appropriately selected from publicly known groups and publicly known means.

In another method for obtaining the amides of the desired protein or partial peptide, for example, the α-carboxyl group of the carboxy terminal amino acid is first protected by amidation; the peptide (protein) chain is then extended from the amino group side to a desired length. Thereafter, a protein or partial peptide, in which only the protecting group of the N-terminal α-amino group of the peptide chain has been eliminated, and a protein or partial peptide, in which only the protecting group of the C-terminal carboxyl group has been eliminated, are manufactured. The two proteins or peptides are condensed in a mixture of the solvents described above. The details of the condensation reaction are the same as described above. After the protected protein or peptide obtained by the condensation is purified, all the protecting groups are eliminated by the method described above to give the desired crude protein or peptide. This crude protein or peptide is purified by various known purification means. Lyophilization of the major fraction gives the amide of the desired protein or peptide.

To prepare the esterified protein or peptide, for example, the α-carboxyl group of the carboxy terminal amino acid is condensed with a desired alcohol to prepare the amino acid ester, which is followed by procedures similar to the preparation of the amidated protein or peptide above to give the desired esterified protein or peptide.

The partial peptide used in the present invention or salts thereof can be manufactured by publicly known methods for peptide synthesis, or by cleaving the protein used in the present invention with an appropriate peptidase. For the methods for peptide synthesis, for example, either solid phase synthesis or liquid phase synthesis may be used. That is, the partial peptide or amino acids that can construct the partial peptide used in the present invention are condensed with the remaining part. Where the product contains protecting groups, these protecting groups are removed to give the desired peptide. Publicly known methods for condensation and elimination of the protecting groups are described in (i) to (v) below.
(i) M. Bodanszky & M.A. Ondetti: Peptide Synthesis, Interscience Publishers, New York (1966)
(ii) Schroeder & Luebke: The Peptide, Academic Press, New York (1965)
(iii) Nobuo Izumiya, et al.: Peptide Gosei-no-Kiso to Jikken (Basics and experiments of peptide synthesis), published by Maruzen Co. (1975)
(iv) Haruaki Yajima & Shunpei Sakakibara: Seikagaku Jikken Koza (Biochemical Experiment) 1, Tanpakushitsu no Kagaku (Chemistry of Proteins) IV, 205 (1977)
(v) Haruaki Yajima ed.: Zoku Iyakuhin no Kaihatsu (A sequel to Development of Pharmaceuticals), Vol. 14, Peptide Synthesis, published by Hirokawa Shoten

After completion of the reaction, the product may be purified and isolated by a combination of conventional purification methods such as solvent extraction, distillation, column chromatography, liquid chromatography and recrystallization to give the partial peptide used in the present invention. When the partial peptide obtained by the above methods is in a free form, the partial peptide can be converted into an appropriate salt by a publicly known method or its modification; when the partial peptide is obtained in a salt form, it can be converted into a free form or other different salt form by a publicly known method or its modification.

The polynucleotide encoding the protein used in the present invention may be any polynucleotide so long as it contains the base sequence encoding the protein used in the present invention described above. Preferably, the polynucleotide is a DNA. The DNA may also be any one of genomic DNA, genomic DNA library, cDNA derived from the cells or tissues described above, cDNA library derived from the cells or tissues described above and synthetic DNA.

The vector used for the library may be any of bacteriophage, plasmid, cosmid, phagemid and the like. In addition, the DNA can be amplified by reverse transcriptase polymerase chain reaction (hereinafter abbreviated as RT-PCR) with total RNA or mRNA fraction prepared from the above-described cells or tissues.

The DNA encoding the protein used in the present invention may be any one of, for example, a DNA comprising the base sequence represented by SEQ ID NO: 2, or a DNA comprising a base sequence hybridizable to the base sequence represented by SEQ ID NO: 2 under high stringent conditions and encoding a protein which has the properties of substantially the same nature as those of the protein having the amino acid sequence represented by SEQ ID NO: 1 described above.
The DNA encoding the protein used in the present invention may be any one of, for example, a DNA comprising the base sequence represented by SEQ ID NO: 4, or a DNA comprising a base sequence hybridizable to the base sequence represented by SEQ ID NO: 4 under high stringent conditions and encoding a protein which has the properties of substantially the same nature as those of the protein having the amino acid sequence represented by SEQ ID NO: 3 described above. The DNA encoding the protein used in the present invention may be any one of, for example, a DNA comprising the base sequence represented by SEQ ID NO: 6, or a DNA comprising a base sequence hybridizable to the base sequence represented by SEQ ID NO: 6 under high stringent conditions and encoding a protein which has the properties of substantially the same nature as those of the protein having the amino acid sequence represented by SEQ ID NO: 5 described above. The DNA encoding the protein used in the present invention may be any one of, for example, a DNA comprising the base sequence represented by SEQ ID NO: 51, or a DNA comprising a base sequence hybridizable to the base sequence represented by SEQ ID NO: 51 under high stringent conditions and encoding a protein which has the properties of substantially the same nature as those of the protein having the amino acid sequence represented by SEQ ID NO: 50 described above.

Specific examples of the DNA that is hybridizable to the base sequence represented by SEQ ID NO: 2 under high stringent conditions include DNAs comprising at least about 70% homology, preferably at least about 80% homology, more preferably at least about 90% homology and most preferably at least about 95% homology, to the base sequence represented by SEQ ID NO: 2; and the like. Homology in the base sequence can be measured under the following conditions (an expectation value = 10; gaps are allowed; filtering = ON; match score = 1; mismatch score = -3) using the homology scoring algorithm NCBI BLAST (National Center for Biotechnology Information Basic Local Alignment Search Tool).

The hybridization can be carried out by publicly known methods or by modifications thereof, for example, by the method described in Molecular Cloning, 2nd (J. Sambrook et al., Cold Spring Harbor Lab. Press, 1989). A commercially available library can also be used according to the instructions of the attached manufacturer's protocol. The hybridization can be carried out preferably under high stringent conditions.

The high stringent conditions used herein are, for example, those in a sodium concentration at about 19 to 40 mM, preferably about 19 to 20 mM at a temperature of about 50 to 70°C, preferably about 60 to 65°C. In particular, hybridization conditions in a sodium concentration at about 19 mM at a temperature of about 65°C are most preferred.

More specifically, as the DNA encoding the protein comprising the amino acid sequence represented by SEQ ID NO: 1, there are employed a DNA comprising the base sequence represented by SEQ ID NO: 2, and the like.

The polynucleotide (e.g., DNA) encoding the partial peptide used in the present invention may be any polynucleotide so long as it contains the base sequence encoding the partial peptide used in the present invention described above. The polynucleotide may also be any of genomic DNA, genomic DNA library, cDNA derived from the cells and tissues described above, cDNA library derived from the cells and tissues described above and synthetic DNA.

As the DNA encoding the partial peptide used in the present invention, there are employed, for example, a DNA comprising a part of the DNA having the base sequence represented by SEQ ID NO: 2, or a DNA comprising a base sequence hybridizable to the base sequence represented by SEQ ID NO: 2 under high stringent conditions and comprising a part of DNA encoding a protein having the activities of substantially the same nature as those of the protein of the present invention, and the like.

The DNA hybridizable to the base sequence represented by SEQ ID NO: 2 indicates the same meaning as described above.

Methods for the hybridization and the high stringent conditions that can be used are the same as those described above.

For cloning of DNAs that completely encode the protein or partial peptide used in the present invention (hereinafter sometimes merely referred to as the protein of the present invention in the description of cloning of DNAs encoding the protein and partial peptide and their expression), the DNA can be either amplified by PCR using synthetic DNA primers containing a part of the base sequence of the protein of the present invention, or the DNA inserted into an appropriate vector can be selected by hybridization with a labeled DNA fragment or synthetic DNA that encodes a part or entire region of the protein of the present invention. The hybridization can be carried out, for example, according to the method described in Molecular Cloning, 2nd (J. Sambrook et al., Cold Spring Harbor Lab. Press, 1989). Where the hybridization is carried out using commercially available library, the procedures may be conducted in accordance with the protocol described in the attached instructions.

Substitution of the base sequence of DNA can be effected by publicly known methods such as the ODA-LA PCR method, the Gapped duplex method, the Kunkel method, etc., or its modification, using PCR, a publicly known kit available as Mutan™-super Express Km (manufactured by Takara Shuzo Co., Ltd.) or Mutan™-K (manufactured by Takara Shuzo Co., Ltd.), etc.

The cloned DNA encoding the protein can be used as it is, depending upon purpose or, if desired, after digestion with a restriction enzyme or after addition of a linker thereto. The DNA may contain ATG as a translation initiation codon at the 5' end thereof and TAA, TGA or TAG as a translation termination codon at the 3' end thereof. These translation initiation and termination codons may also be added by using an appropriate synthetic DNA adapter.

The expression vector for the protein of the present invention can be manufactured, for example, by (a) excising the desired DNA fragment from the DNA encoding the protein of the present invention, and then (b) ligating the DNA fragment with an appropriate expression vector downstream a promoter in the vector.

Examples of the vector include plasmids derived form E. coli (e.g., pBR322, pBR325, pUC12, pUC13), plasmids derived from Bacillus subtilis (e.g., pUB 110, pTP5, pC194), plasmids derived from yeast (e.g., pSH19, pSH15), bacteriophages such as λ phage, etc., animal viruses such as retrovirus, vaccinia virus, baculovirus, etc. as well as pA1-11, pXT1, pRc/CMV, pRc/RSV, pcDNA I/Neo, etc.

The promoter used in the present invention may be any promoter if it matches well with a host to be used for gene expression. In the case of using animal cells as the host, examples of the promoter include SRα promoter, SV40 promoter, LTR promoter, CMV promoter, HSV-TK promoter, etc.

Among them, it is preferred to use CMV (cytomegalovirus) promoter, SRα promoter, etc. Where the host is bacteria of the genus Escherichia, preferred examples of the promoter include trp promoter, lac promoter, recA promoter, λP_{L} promoter, Ipp promoter, T7 promoter, etc. In the case of using bacteria of the genus Bacillus as the host, preferred example of the promoter are SPO1 promoter, SPO2 promoter, penP promoter, etc. When yeast is used as the host, preferred examples of the promoter are PHO5 promoter, PGK promoter, GAP promoter, ADH promoter, etc. When insect cells are used as the host, preferred examples of the promoter include polyhedrin prompter, P10 promoter, etc.

In addition to the foregoing examples, the expression vector may further optionally contain an enhancer, a splicing signal, a poly A addition signal, a selection marker, SV40 replication origin (hereinafter sometimes abbreviated as SV40ori), etc. Examples of the selection marker include dihydrofolate reductase (hereinafter sometimes abbreviated as dhfr) gene [methotrexate (MTX) resistance], ampicillin resistant gene (hereinafter sometimes abbreviated as Amp^{r}), neomycin resistant gene (hereinafter sometimes abbreviated as Neo^{r}, G418 resistance), etc. In particular, when dhfr gene is used as the selection marker using dhfr gene-deficient Chinese hamster cells, selection can also be made on a thymidine free medium.

If necessary, a signal sequence that matches with a host is added to the N-terminus of the protein of the present invention. Examples of the signal sequence that can be used are PhoA signal sequence, OmpA signal sequence, etc. when bacteria of the genus Escherichia is used as the host; α-amylase signal sequence, subtilisin signal sequence, etc. when bacteria of the genus Bacillus is used as the host; MFα signal sequence, SUC2 signal sequence, etc. when yeast is used as the host; and insulin signal sequence, α-interferon signal sequence, antibody molecule signal sequence, etc. when animal cells are used as the host, respectively.

Using the vector containing the DNA encoding the protein of the present invention thus constructed, transformants can be manufactured.

Examples of the host, which may be employed, are bacteria belonging to the genus Escherichia, bacteria belonging to the genus Bacillus, yeast, insect cells, insects, animal cells, etc.

Specific examples of the bacteria belonging to the genus Escherichia include Escherichia coli K12 DH1 [Proc. Natl. Acad. Sci. U.S.A., 60, 160 (1968)], JM103 [Nucleic Acids Research, 9, 309 (1981)), JA221 [Journal of Molecular Biology, 120, 517 (1978)], HB101 [Journal of Molecular Biology, 41, 459 (1969)], C600 [Genetics, 39, 440 (1954)], etc.

Examples of the bacteria belonging to the genus Bacillus include Bacillus subtilis MI114 [Gene, 24, 255 (1983)], 207-21 [Journal of Biochemistry, 95, 87 (1984)], etc.

Examples of yeast include Saccharomyces cereviseae AH22, AH22R⁻, NA87-11A, DKD-5D, 20B-12, Schizosaccharomyces pombe NCYC1913, NCYC2036, Pichia pastoris KM71, etc.

Examples of insect cells include, for the virus AcNPV, Spodoptera frugiperda cell (Sf cell), MG1 cell derived from mid-intestine of Trichoplusia ni, High Five™ cell derived from egg of Trichoplusia ni, cells derived from Mamestra brassicae, cells derived from Estigmena acrea, etc.; and for the virus BmNPV, Bombyx mori N cell (BmN cell), etc. is used. Examples of the Sf cell which can be used are Sf9 cell (ATCC CRL1711), Sf21 cell (both cells are described in Vaughn, J. L. et al., In Vivo, 13, 213-217 (1977)), etc.

As the insect, for example, a larva of Bombyx mori can be used [Maeda et al., Nature, 315, 592 (1985)].

Examples of animal cells include monkey cell COS-7, Vero, Chinese hamster cell CHO (hereinafter referred to as CHO cell), dhfr gene-deficient Chinese hamster cell CHO (hereinafter simply referred to as CHO (dhfr⁻) cell), mouse L cell, mouse AtT-20, mouse myeloma cell, mouse ATDC5 cell, rat GH3, human FL cell, etc. In addition, there are also used human cancer cell lines (DLD-1cells, HCT-15 cells, SW-480 cells, LoVo cells, HCT-116 cells, WiDr cells, HT-29 cells, LS-174T cells, SNU-C1 cells, SNU-C4 cells, SNU-C2A cells, CX-1 cells, GI-112 cells, HL-60 cells, Raji cells, G361 cells, S3 cells), etc.

Bacteria belonging to the genus Escherichia can be transformed, for example, by the method described in Proc. Natl. Acad. Sci. U.S.A., 69, 2110 (1972), Gene, 17, 107 (1982), etc.

Bacteria belonging to the genus Bacillus can be transformed, for example, by the method described in Molecular & General Genetics, 168, 111 (1979), etc.

Yeast can be transformed, for example, by the method described in Methods in Enzymology, 194, 182-187 (1991), Proc. Natl. Acad. Sci. U.S.A., 75, 1929 (1978), etc.

Insect cells or insects can be transformed, for example, according to the method described in Bio/Technology, 6, 47-55 (1988), etc.

Animal cells can be transformed, for example, according to the method described in Saibo Kogaku (Cell Engineering), extra issue 8, Shin Saibo Kogaku Jikken Protocol (New Cell Engineering Experimental Protocol), 263-267 (1995) (published by Shujunsha), or Virology, 52, 456 (1973).

Thus, the transformants transformed with the expression vectors containing the DNAs encoding the protein can be obtained.

Where the host is bacteria belonging to the genus Escherichia or the genus Bacillus, the transformant can be appropriately cultured in a liquid medium, which contains materials required for growth of the transformant such as carbon sources, nitrogen sources, inorganic materials, and the like. Examples of the carbon sources include glucose, dextrin, soluble starch, sucrose, etc.; examples of the nitrogen sources include inorganic or organic materials such as ammonium salts, nitrate salts, corn steep liquor, peptone, casein, meat extract, soybean cake, potato extract, etc.; and, examples of the inorganic materials are calcium chloride, sodium dihydrogenphosphate, magnesium chloride, etc. In addition, yeast extracts, vitamins, growth promoting factors etc. may also be added to the medium. Preferably, pH of the medium is adjusted to about 5 to about 8.

A preferred example of the medium for culturing the bacteria belonging to the genus Escherichia is M9 medium supplemented with glucose and Casamino acids [Miller, Journal of Experiments in Molecular Genetics, 431-433, Cold Spring Harbor Laboratory, New York, 1972]. If necessary, a chemical such as 3β-indolylacrylic acid can be added to the medium thereby to activate the promoter efficiently.

Where the bacteria belonging to the genus Escherichia are used as the host, the transformant is usually cultivated at about 15 to 43°C for about 3 to 24 hours. If necessary, the culture may be aerated or agitated.

Where the bacteria belonging to the genus Bacillus are used as the host, the transformant is cultured generally at about 30 to 40°C for about 6 to 24 hours. If necessary, the culture can be aerated or agitated.

Where yeast is used as the host, the transformant is cultivated, for example, in Burkholder's minimal medium [Bostian, K. L. et al., Proc. Natl. Acad. Sci. U.S.A., 77, 4505 (1980)] or in SD medium supplemented with 0.5% Casamino acids [Bitter, G. A. et al., Proc. Natl. Acad. Sci. U.S.A., 81, 5330 (1984)]. Preferably, pH of the medium is adjusted to about 5 to 8. In general, the transformant is cultivated at about 20 to 35°C for about 24 to 72 hours. If necessary, the culture can be aerated or agitated.

Where insect cells or insects are used as the host, the transformant is cultivated in, for example, Grace's Insect Medium (Grace, T. C. C., Nature), 195, 788 (1962)) to which an appropriate additive such as immobilized 10% bovine serum is added. Preferably, pH of the medium is adjusted to about 6.2 to about 6.4. Normally, the transformant is cultivated at about 27°C for about 3 days to about 5 days and, if necessary, the culture can be aerated or agitated.

Where animal cells are employed as the host, the transformant is cultured in, for example, MEM medium containing about 5 to 20% fetal bovine serum [Science, 122, 501 (1952)], DMEM medium [Virology, 8, 396 (1959)], RPMI 1640 medium [The Journal of the American Medical Association, 199, 519 (1967)], 199 medium [Proceeding of the Society for the Biological Medicine, 73, 1 (1950)], etc. Preferably, pH of the medium is adjusted to about 6 to about 8. The transformant is usually cultivated at about 30°C to about 40°C for about 15 to 60 hours and, if necessary, the culture can be aerated or agitated.

As described above, the protein of the present invention can be produced in the transformant, in the cell membrane of the transformant, or outside of the transformant.

The protein of the present invention can be separated and purified from the culture described above by the following procedures.

When the protein of the present invention is extracted from the bacteria or cells, the bacteria or cell is collected after culturing by a publicly known method and suspended in an appropriate buffer. The bacteria or cell is then disrupted by publicly known methods such as ultrasonication, a treatment with lysozyme and/or freeze-thaw cycling, followed by centrifugation, filtration, etc to produce crude extract of the protein. Thus, the crude extract of the protein can be obtained. The buffer used for the procedures may contain a protein modifier such as urea or guanidine hydrochloride, or a surfactant such as Triton X-100™, etc. When the protein of the present invention is secreted in the culture broth, the supernatant can be separated, after completion of the cultivation, from the bacteria or cell to collect the supernatant by a publicly known method.

The protein contained in the supernatant or the extract thus obtained can be purified by appropriately combining the publicly known methods for separation and purification. Such publicly known methods for separation and purification include a method utilizing difference in solubility such as salting out, solvent precipitation, etc.; a method mainly utilizing difference in molecular weight such as dialysis, ultrafiltration, gel filtration, SDS-polyacrylamide gel electrophoresis, etc.; a method utilizing difference in electric charge such as ion exchange chromatography, etc.; a method utilizing difference in specific affinity such as affinity chromatography, etc.; a method utilizing difference in hydrophobicity such as reverse phase high performance liquid chromatography, etc.; a method utilizing difference in isoelectric point such as isoelectrofocusing electrophoresis; and the like.

When the protein thus obtained is in a free form, the protein can be converted into the salt by publicly known methods or modifications thereof. On the other hand, when the protein is obtained in the form of a salt, it can be converted into the free form or in the form of a different salt by publicly known methods or modifications thereof.

The protein produced by the recombinant can be treated, prior to or after the purification, with an appropriate protein-modifying enzyme so that the protein can be subjected to addition of an appropriate modification or removal of a partial polypeptide. Examples of the protein-modifying enzyme include trypsin, chymotrypsin, arginyl endopeptidase, protein kinase, glycosidase and the like.

The presence of the thus produced protein of the present invention can be determined by an enzyme immunoassay or western blotting using a specific antibody.

The antibodies to the protein or partial peptide used in the present invention, or its salts may be any of polyclonal and monoclonal antibodies, as long as they are capable of recognizing the protein or partial peptide used in the present invention, or its salts.

The antibodies to the protein or partial peptide used in the present invention, or its salts (hereinafter they are sometimes collectively referred to as the protein of the present invention in the description of the antibodies) can be produced by a publicly known method of producing an antibody or antiserum, using the protein of the present invention as an antigen.

### [Preparation of monoclonal antibody]

### (a) Preparation of monoclonal antibody-producing cells

The protein of the present invention is administered to warm-blooded animals either solely or together with carriers or diluents to the site where the production of antibody is possible by the administration. In order to potentiate the antibody productivity upon the administration, complete Freund's adjuvants or incomplete Freund's adjuvants may be administered. The administration is usually carried out once every about 2 to about 6 weeks and about 2 to about 10 times in total. Examples of the applicable warm-blooded animals are monkeys, rabbits, dogs, guinea pigs, mice, rats, sheep, goats and fowl, with the use of mice and rats being preferred.

In the preparation of monoclonal antibody-producing cells, a warm-blooded animal, e.g., mouse, immunized with an antigen wherein the antibody titer is noted is selected, then spleen or lymph node is collected after 2 to 5 days from the final immunization and antibody-producing cells contained therein are fused with myeloma cells from homozoic or heterozoic animal to give monoclonal antibody-producing hybridomas. Measurement of the antibody titer in antisera may be carried out, for example, by reacting a labeled protein, which will be described later, with the antiserum followed by assaying the binding activity of the labeling agent bound to the antibody. The fusion may be carried out, for example, by the known method by Koehler and Milstein [Nature, 256, 495, (1975)]. Examples of the fusion accelerator are polyethylene glycol (PEG), Sendai virus, etc., of which PEG is preferably employed.

Examples of the myeloma cells are those collected from warm-blooded animals such as NS-1, P3U1, SP2/0, AP-1, etc. In particular, P3U1 is preferably employed. A preferred ratio of the count of the antibody-producing cells used (spleen cells) to the count of myeloma cells is within a range of approximately 1:1 to 20:1. When PEG (preferably, PEG 1000 to PEG 6000) is added in a concentration of approximately 10 to 80% followed by incubation at 20 to 40°C, preferably at 30 to 37°C for 1 to 10 minutes, an efficient cell fusion can be carried out.

Various methods can be used for screening of monoclonal antibody-producing hybridomas. Examples of such methods include a method which comprises adding the supernatant of a hybridoma to a solid phase (e.g., a microplate) adsorbed with the protein as an antigen directly or together with a carrier, adding an anti-immunoglobulin antibody (where mouse cells are used for the cell fusion, anti-mouse immunoglobulin antibody is used) labeled with a radioactive substance or an enzyme or Protein A and detecting the monoclonal antibody bound to the solid phase, and a method which comprises adding the supernatant of hybridoma to a solid phase adsorbed with an anti-immunoglobulin antibody or Protein A, adding the protein labeled with a radioactive substance or an enzyme and detecting the monoclonal antibody bound to the solid phase, or the like.

The monoclonal antibody can be screened according to publicly known methods or their modifications. In general, the screening can be performed in a medium for animal cells supplemented with HAT (hypoxanthine, aminopterin and thymidine). Any screening and growth medium can be employed as far as the hybridoma can grow there. For example, RPMI 1640 medium containing 1 to 20%, preferably 10 to 20% fetal bovine serum, GIT medium (Wako Pure Chemical Industries, Ltd.) containing 1 to 10% fetal bovine serum, a serum free medium for cultivation of a hybridoma (SFM-101, Nissui Seiyaku Co., Ltd.) and the like, can be used for the screening and growth medium. The culture is carried out generally at 20 to 40°C, preferably at 37°C, for about 5 days to about 3 weeks, preferably 1 to 2 weeks, normally in 5% CO₂. The antibody titer of the culture supernatant of a hybridoma can be determined as in the assay for the antibody titer in antisera described above.

### (b) Purification of monoclonal antibody

Separation and purification of a monoclonal antibody can be carried out by publicly known methods, such as separation and purification of immunoglobulins [for example, salting-out, alcohol precipitation, isoelectric point precipitation, electrophoresis, adsorption and desorption with ion exchangers (e.g., DEAE), ultracentrifugation, gel filtration, or a specific purification method which comprises collecting only an antibody with an activated adsorbent such as an antigen-binding solid phase, Protein A or Protein G and dissociating the binding to obtain the antibody.]

### [Preparation of polyclonal antibody]

The polyclonal antibody of the present invention can be manufactured by publicly known methods or modifications thereof. For example, a warm-blooded animal is immunized with an immunogen (protein antigen) per se, or a complex of immunogen and a carrier protein is formed and the animal is immunized with the complex in a manner similar to the method described above for the manufacture of monoclonal antibodies. The product containing the antibody to the protein of the present invention is collected from the immunized animal followed by separation and purification of the antibody.

In the complex of immunogen and carrier protein used to immunize a warm-blooded animal, the type of carrier protein and the mixing ratio of carrier to hapten may be any type and in any ratio, as long as the antibody is efficiently produced to the hapten immunized by crosslinking to the carrier. For example, bovine serum albumin, bovine thyroglobulin or hemocyanin is coupled to hapten in a carrier-to-hapten weight ratio of approximately 0.1 to 20, preferably about 1 to 5.

A variety of condensation agents can be used for the coupling of carrier to hapten. Glutaraldehyde, carbodiimide, maleimide activated ester and activated ester reagents containing thiol group or dithiopyridyl group are used for the coupling.

The condensation product is administered to warm-blooded animals either solely or together with carriers or diluents to the site that can produce the antibody by the administration. In order to potentiate the antibody productivity upon the administration, complete Freund's adjuvant or incomplete Freund's adjuvant may be administered. The administration is usually made once every about 2 to 6 weeks and about 3 to 10 times in total.

The polyclonal antibody can be collected from the blood, ascites, etc., preferably from the blood of warm-blooded animal immunized by the method described above.

The polyclonal antibody titer in antiserum can be assayed by the same procedure as that for the determination of serum antibody titer described above. The separation and purification of the polyclonal antibody can be carried out, following the method for the separation and purification of immunoglobulins performed as in the separation and purification of monoclonal antibodies described hereinabove.

The antisense polynucleotide having a complementary or substantially complementary base sequence to the base sequence of a polynucleotide encoding the protein or partial peptide used in the present invention (e.g., DNA (hereinafter these DNAs are sometimes collectively referred to as the DNA of the present invention in the description of antisense polynucleotide)) can be any antisense polynucleotide, so long as it possesses a base sequence complementary or substantially complementary to the base sequence of the polynucleotide (e.g., DNA) of the present invention and capable of suppressing the expression of said DNA, but antisense DNA is preferred.

The base sequence substantially complementary to the DNA of the present invention may include, for example, a base sequence having at least about 70% homology, preferably at least about 80% homology, more preferably at least about 90% homology and most preferably at least about 95% homology, to the entire base sequence or to its partial base sequence (i.e., complementary strand to the DNA of the present invention), and the like. Especially in the entire base sequence of the complementary strand to the DNA of the present invention, preferred are (a) an antisense polynucleotide having at least about 70% homology, preferably at least about 80% homology, more preferably at least about 90% homology and most preferably at least about 95% homology, to the complementary strand of the base sequence which encodes the N-terminal region of the protein of the present invention (e.g., the base sequence around the initiation codon) in the case of antisense polynucleotide directed to translation inhibition and (b) an antisense polynucleotide having at least about 70% homology, preferably at least about 80% homology, more preferably at least about 90% homology and most preferably at least about 95% homology, to the complementary strand of the entire base sequence of the DNA of the present invention having intron, in the case of antisense polynucleotide directed to RNA degradation by RNaseH, respectively.

Specific examples include an antisense polynucleotide containing the entire or part of a base sequence complementary or substantially complementary to a base sequence of DNA containing the base sequence represented by SEQ ID NO: 2, preferably an antisense polynucleotide containing the entire or part of a base sequence complementary to a base sequence of DNA containing the base sequence represented by SEQ ID NO: 2 (more preferably, an antisense polynucleotide containing the entire or part of a base sequence complementary to a base sequence of DNA containing the base sequence represented by SEQ ID NO: 2), etc.

The antisense polynucleotide is generally constituted by bases of about 10 to about 40, preferably about 15 to about 30.

To prevent digestion with a hydrolase such as nuclease, etc., the phosphoric acid residue (phosphate) of each nucleotide that constitutes the antisense DNA may be substituted with chemically modified phosphoric acid residues, e.g., phosphorothioate, methyl phosphonate, phosphorodithionate, etc. Also, the sugar (deoxyribose) in each nucleotide may be replaced by a chemically modified structure such as 2'-O-methylation, etc. The base part (pyrimidine, purine) may also be chemically modified and may be any one which hybridizes to a DNA containing the base sequence represented by SEQ ID NO: 2. These antisense polynucleotides may be synthesized using a publicly known DNA synthesizer, etc.

According to the present invention, the antisense polynucleotide capable of inhibiting the replication or expression of a gene for the protein of the present invention can be designed and synthesized based on the base sequence information of cloned or identified protein-encoding DNA. Such a nucleotide (nucleic acid) is hybridizable to RNA of a gene for the protein of the present invention to inhibit the synthesis or function of said RNA or is capable of modulating and/or controlling the expression of a gene for the protein of the present invention via interaction with RNA associated with the protein of the present invention. Polynucleotides complementary to the selected sequences of RNA associated with the protein of the present invention and polynucleotides specifically hybridizable to RNA associated with the protein of the present invention are useful in modulating and/or controlling the in vivo and in vitro expression of the protein gene of the present invention, and are useful for the treatment or diagnosis of diseases, etc. The term "corresponding" is used to mean homologous to or complementary to a particular sequence of the nucleotide including the gene, base sequence or nucleic acid. The term "corresponding" between nucleotides, base sequences or nucleic acids and peptides (proteins) usually refer to amino acids of a peptide (protein) under the order derived from the sequence of nucleotides (nucleic acids) or their complements. In the protein genes, the 5' end hairpin loop, 5' end 6-base-pair repeats, 5' end untranslated region, polypeptide translation initiation codon, protein coding region, ORF translation termination codon, 3' end untranslated region, 3' end palindrome region, and 3' end hairpin loop, may be selected as preferred target regions, though any other region may be selected as a target in the protein genes.

The relationship between the targeted nucleic acids and the polynucleotides complementary to at least a part of the target region, specifically the relationship between the target nucleic acids and the polynucleotides hybridizable to the target region, can be denoted to be "antisense." Examples of the antisense polynucleotides include polynucleotides containing 2-deoxy-D-ribose, polynucleotides containing D-ribose, any other type of polynucleotides which are N-glycosides of a purine or pyrimidine base, or other polymers containing non-nucleotide backbones (e.g., commercially available protein nucleic acids and synthetic sequence-specific nucleic acid polymers) or other polymers containing nonstandard linkages (provided that the polymers contain nucleotides having such a configuration that allows base pairing or base stacking, as is found in DNA or RNA), etc. The antisense polynucleotides may be double-stranded DNA, single-stranded DNA, double-stranded RNA, single-stranded RNA or a DNA:RNA hybrid, and may further include unmodified polynucleotides (or unmodified oligonucleotides), those with publicly known types of modifications, for example, those with labels known in the art, those with caps, methylated polynucleotides, those with substitution of one or more naturally occurring nucleotides by their analogue, those with intramolecular modifications of nucleotides such as those with uncharged linkages (e.g., methyl phosphonates, phosphotriesters, phosphoramidates, carbamates, etc.) and those with charged linkages or sulfur-containing linkages (e.g., phosphorothioates, phosphorodithioates, etc.), those having side chain groups such as proteins (nucleases, nuclease inhibitors, toxins, antibodies, signal peptides, poly-L-lysine, etc.), saccharides (e.g., monosaccharides, etc.), those with intercalators (e.g., acridine, psoralen, etc.), those containing chelators (e.g., metals, radioactive metals, boron, oxidative metals, etc.), those containing alkylating agents, those with modified linkages (e.g., α anomeric nucleic acids, etc.), and the like. Herein the terms "nucleoside", "nucleotide" and "nucleic acid" are used to refer to moieties that contain not only the purine and pyrimidine bases, but also other heterocyclic bases, which have been modified. Such modifications may include methylated purines and pyrimidines, acylated purines and pyrimidines and other heterocyclic rings. Modified nucleotides and modified nucleotides also include modifications on the sugar moiety, wherein, for example, one or more hydroxyl groups may optionally be substituted with a halogen atom(s), an aliphatic group(s), etc., or may be converted into the corresponding functional groups such as ethers, amines, or the like.

The antisense polynucleotide of the present invention is RNA, DNA or a modified nucleic acid (RNA, DNA). Specific examples of the modified nucleic acid are, but not limited to, sulfur and thiophosphate derivatives of nucleic acids and those resistant to degradation of polynucleoside amides or oligonucleoside amides. The antisense nucleic acids of the present invention can be modified preferably based on the following design, that is, by increasing the intracellular stability of the antisense nucleic acid, enhancing the cell permeability of the antisense nucleic acid, increasing the affinity of the nucleic acid to the targeted sense strand to a higher level, or minimizing the toxicity, if any, of the antisense nucleic acid.

Many of such modifications are known in the art, as disclosed in J. Kawakami, et al., Pharm. Tech. Japan, Vol. 8, pp. 247, 1992; Vol. 8, pp. 395, 1992; S. T. Crooke, et al. ed., Antisense Research and Applications, CRC Press, 1993; etc.

The antisense nucleic acid of the present invention may contain altered or modified sugars, bases or linkages. The antisense nucleic acid may also be provided in a specialized form such as liposomes, microspheres, or may be applied to gene therapy, or may be provided in combination with attached moieties. Such attached moieties include polycations such as polylysine that act as charge neutralizers of the phosphate backbone, or hydrophobic moieties such as lipids (e.g., phospholipids, cholesterols, etc.) that enhance the interaction with cell membranes or increase uptake of the nucleic acid. Preferred examples of the lipids to be attached are cholesterols or derivatives thereof (e.g., cholesteryl chloroformate, cholic acid, etc.). These moieties may be attached to the nucleic acid at the 3' or 5' ends thereof and may also be attached thereto through a base, sugar, or intramolecular nucleoside linkage. Other moieties may be capping groups specifically placed at the 3' or 5' ends of the nucleic acid to prevent degradation by nucleases such as exonuclease, RNase, etc. Such capping groups include, but are not limited to, hydroxyl protecting groups known in the art, including glycols such as polyethylene glycol, tetraethylene glycol and the like.

The inhibitory action of the antisense nucleic acid can be examined using the transformant of the present invention, the gene expression system of the present invention in vivo and in vitro, or the translation system for the protein of the present invention in vivo and in vitro. The nucleic acid can be applied to cells by a variety of publicly known methods.

Hereinafter, the protein of the present invention, its partial peptides, or salts thereof (hereinafter sometimes merely referred to as the protein of the present invention), the polynucleotide (e.g., DNA (hereinafter sometimes merely referred to as the DNA of the present invention)) encoding the protein of the present invention or its partial peptides, the antibodies to the protein of the present invention, its partial peptides, or salts thereof (hereinafter sometimes referred to as the antibodies of the present invention) and the antisense polynucleotides to the DNA of the present invention (hereinafter sometimes merely referred to as the antisense polynucleotides of the present invention) are specifically described for their applications.

### (1) Agents for the prevention/treatment of various diseases associated with the protein of the invention

The protein of the present invention has a glucose active transport activity as the Na⁺/glucose transporter and is responsible for glucose uptake in the small intestine.
In the specification, the term "small intestine" may broadly refer to the small intestine in (namely, including the duodenum) and preferably is used to mean the small intestine in a narrow sense (namely, excluding the duodenum). The uptake of glucose may also be rephrased as the uptake of monosaccharides (e.g., glucose, mannose, fructose, sorbose, galactose, ribose, arabinose, xylose, etc.) and preferably refers to the uptake of glucose.

Therefore, the protein of the present invention is effective for the improvement of postprandial hyperglycemia, etc. by suppressing the activity of the protein of the present invention or the expression level of a gene for the protein in the small intestine and thus can be used as pharmaceuticals for the treatment/prevention of diseases including diabetes mellitus, obesity, hyperlipemia, metabolic syndrome, etc. Further by enhancing the activity of the protein of the present invention or the expression level of a gene for the protein in the small intestine, the protein can be used as pharmaceuticals such as antihypoglycemic agents like glucose absorption promoters, or digestive medicines, etc.

For example, when the uptake of glucose into the small intestine cannot be expected in a patient sufficiently or normally due to a decrease or deficiency of the protein of the present invention in the small intestine, the role of the protein of the present invention can be exhibited sufficiently or normally, (a) by administering the DNA of the present invention directly to the patient thereby to express the protein of the present invention; (b) by inserting the DNA of the present invention into cells to express the protein of the present invention and transplant the cells to the patient; or (c) by administering the protein of the present invention to the patient; etc.

Furthermore, for example, when the protein of the present invention is increasingly expressed in the small intestine and hence the uptake of glucose into the small intestine is enhanced in a patient leading to exhibit postprandial hyperglycemia, the role of the protein of the present invention can be suppressed, (a) by administering the antisense DNA of the present invention to the patient; (b) by transplanting a cell producing the antibody of the present invention to the patient; or (c) by administering the antibody of the present invention to the patient; etc.

For example, when the activity of the protein of the present invention cannot be expected in a patient sufficiently or normally due to a decrease or deficiency of the protein of the present invention in the body, the role of the protein of the present invention can be exhibited sufficiently or normally, (a) by administering the DNA of the present invention directly to the patient thereby to express the protein of the present invention; (b) by inserting the DNA of the present invention into cells to express the protein of the present invention and transplant the cells to the patient; or (c) by administering the protein of the present invention to the patient; etc.

Where the DNA (including the antisense DNA) of the present invention is used as the prophylactic/therapeutic agents described above, the DNA of the present invention is administered alone; alternatively, the DNA is inserted into an appropriate vector such as retrovirus vector, adenovirus vector, adenovirus-associated virus vector, etc. and then administered to human or other warm-blooded animal in a conventional manner. The DNA of the present invention may also be administered as intact DNA, or with pharmacologically acceptable carrier such as adjuvants to assist its uptake by gene gun or through a catheter such as a catheter with a hydrogel.

When the protein of the present invention is used as the prophylactic/therapeutic agents described above, it is preferred to use the protein with a purity of at least 90%, preferably at least 95%, more preferably at least 98% and most preferably at least 99%.

The protein of the present invention can be used orally, for example, in the form of tablets which may be sugar coated if necessary, capsules, elixirs, microcapsules etc., or parenterally in the form of injectable preparations such as a sterile solution or suspension in water or other pharmaceutically acceptable liquid. These preparations can be manufactured by mixing the protein of the present invention with a physiologically acceptable carrier, a flavoring agent, an excipient, a vehicle, an antiseptic agent, a stabilizer, a binder, etc. in a unit dosage form required in a generally accepted manner that is applied to making pharmaceutical preparations. The active ingredient in these preparations is controlled in such a dose that an appropriate dose is obtained within the specified range given.

Additives miscible with tablets, capsules, etc. include a binder such as gelatin, corn starch, tragacanth and gum arabic, an excipient such as crystalline cellulose, a swelling agent such as corn starch, gelatin and alginic acid, a lubricant such as magnesium stearate, a sweetening agent such as sucrose, lactose and saccharin, a flavoring agent such as peppermint, akamono oil and cherry, and the like. When the unit dosage is in the form of capsules, liquid carriers such as oils and fats may further be used together with the additives described above. A sterile composition for injection may be formulated by conventional procedures used to make pharmaceutical preparations, e.g., by dissolving or suspending the active ingredients in a vehicle such as water for injection with a naturally occurring vegetable oil such as sesame oil and coconut oil, etc. to prepare the pharmaceutical preparations.

Examples of an aqueous medium for injection include physiological saline and an isotonic solution containing glucose and other auxiliary agents (e.g., D-sorbitol, D-mannitol, sodium chloride, etc.) and may be used in combination with an appropriate dissolution aid such as an alcohol (e.g., ethanol or the like), a polyalcohol (e.g., propylene glycol, polyethylene glycol, etc.), a nonionic surfactant (e.g., polysorbate 80TM, HCO-50, etc.), and the like. Examples of the oily medium include sesame oil and soybean oil, which may also be used in combination with a dissolution aid such as benzyl benzoate, benzyl alcohol, etc. The agent may further be formulated with a buffer (e.g., phosphate buffer, sodium acetate buffer, etc.), a soothing agent (e.g., benzalkonium chloride, procaine hydrochloride, etc.), a stabilizer (e.g., human serum albumin, polyethylene glycol, etc.), a preservative (e.g., benzyl alcohol, phenol, etc.), an antioxidant, etc. The thus prepared liquid for injection is normally filled in an appropriate ampoule.

The vector inserted with the DNA of the present invention is prepared into pharmaceutical preparations as described above and provided normally for use parenterally.

Since the thus obtained pharmaceutical preparation is safe and low toxic, the preparation can be administered to warm-blooded animals (e.g., human, rats, mice, guinea pigs, rabbits, fowl, sheep, swine, bovine, horses, cats, dogs, monkeys, chimpanzees, etc.).

The dose of the protein of the present invention varies depending on target disease, subject to be administered, routes for administration, etc. When the protein, etc. of the present invention is orally administered for the purpose of promoting the absorption of glucose, the protein is administered to adult (as 60 kg body weight) in a dose of normally about 0.1 mg to about 100 mg, preferably about 1.0 to about 50 mg, and more preferably about 1.0 to about 20 mg per day. In parenteral administration, the single dose of the protein, etc. varies depending on subject to be administered, target disease, etc. When the protein, etc. of the present invention is administered to an adult (as 60 kg body weight) in the form of injection for the purpose of promoting the absorption of glucose, it is advantageous to administer the protein, etc. by injecting the protein, etc. into the affected site in a daily dose of about 0.01 to about 30 mg, preferably about 0.1 to about 20 mg, and more preferably about 0.1 to about 10 mg. For other animal species, the corresponding dose as converted per 60 kg body weight can be administered.

The protein containing the same or substantially the same amino acid sequence as the amino acid sequence represented by SEQ ID NO: 1 (hereinafter sometimes referred to as the human SGLT homolog protein) can be utilized as a disease marker for, e.g., diabetes mellitus, since its expression increases tissue-specifically in the human small intestine, pancreas and liver. That is, the protein is useful as a marker for early diagnosis in diabetes mellitus, diagnosis of severity in conditions, or predication in progression of diseases.

The pharmaceuticals comprising the compound or its salts that inhibit the activity of the human SGLT homolog protein in the small intestine can inhibit the uptake of glucose into, e.g., the small intestine to reduce blood sugar and are thus useful as, e.g., postprandial hyperglycemia-improving agents and available also as, e.g., agents for the treatment/prevention of diabetes mellitus, obesity, hyperlipemia, metabolic syndrome, etc.

On the other hand, the pharmaceuticals comprising the compound or its salts that promote the activity of the human SGLT homolog protein in the small intestine can promote the uptake of glucose into, e.g., the small intestine and are thus useful as, e.g., glucose absorption promoters and available also as, e.g., antihypoglycemic agents, digestive medicines, etc.

### (2) Screening of drug candidate compounds for disease

The compound or its salts that regulate (promote or inhibit, preferably inhibit) the activity of the protein of the present invention and the compound or its salts that regulate (promote or inhibit, preferably inhibit) the expression of a gene for the protein of the present invention can be used as, e.g., postprandial hyperglycemia-improving agents, glucose absorption promoters, etc. Preferably, these compounds are postprandial hyperglycemia-improving agents, etc.

Accordingly, the protein of the present invention is useful as a reagent for screening the compound or its salts that regulate (promote or inhibit, preferably inhibit) the activity of the protein of the present invention and the compound or its salts that regulate (promote or inhibit, preferably inhibit) the expression of a gene for the protein of the present invention.

That is, the present invention provides a method of screening the compound or its salts that regulate (promote or inhibit, preferably inhibit) the activity of the protein of the present invention or the compound or its salts that regulate (promote or inhibit, preferably inhibit) the expression of a gene for the protein of the present invention, which comprises using the protein of the present invention. More specifically, in the screening method described above, for example, the expression level of a gene for the protein of the present invention is measured (1) in the presence of a test compound and (2) in the absence of a test compound, followed by comparison between (1) and (2).

In the screening method described above, the method is characterized by measuring, e.g., the glucose uptake action into the small intestine and the gene expression level of the protein of the present invention in the cases (1) and (2), and comparing them.

The present invention further provides:
(1) a method of screening a compound or its salts that promote or inhibit the activity (for example, an active transport activity of glucose, etc.) of the protein of the present invention (hereinafter sometimes simply referred to as the promoter or the inhibitor), which comprises using the protein of the present invention. More specifically, the present invention provides, for example:
(2)
   (i) a method of screening the promoter or the inhibitor, which comprises comparing (i) the glucose uptake activity of a cell capable of producing the protein of the present invention and (ii) the glucose uptake activity of a mixture of a cell capable of producing the protein of the present invention with a test compound.

Specifically, the screening method described above is characterized by determining the glucose uptake activity in the cases of (i) and (ii) through measurement of accumulated glucose analogs such as glucose or 2-deoxy-glucose, which are labeled with ³H, into cells, with the radioactivity, and comparing them as an indicator of the active transport activity of glucose.

In the screening of the present invention, a glucose active transport activity inhibitor (e.g., phlorizin), etc. may be added to the cell capable of producing the protein and used as a positive control.

That is, the present invention provides a method of screening the promoter or the inhibitor, which comprises:
comparing (i) the case in which a glucose active transport activity inhibitor is added to a cell capable of producing the protein of the present invention, at the same time when or before ³H-labeled glucose or glucose analog is taken up into the cell and (ii) the case in which a glucose active transport activity activator or inhibitor and a test compound are added to a cell capable of producing the protein of the present invention, at the same time when or before ³H-labeled glucose or glucose analog is taken up into the cell; and determining a change in the uptake level.

The glucose active transport activity of the protein of the present invention can be determined according to known methods, for example, the method described in "Cloning and functional expression of an SGLT-1-like protein from the Xenopus laevis intestine" (Am. J. Physiol., 276: G1251-G1259, 1999) or a modification thereof.

For example, when a test compound is found to promote the active transport activity of glucose in the case (ii) above by about 20% or more, preferably 30% or more and more preferably about 50% or more, as compared to the case (i), the test compound can be selected as a compound or its salts that promote the activity of the protein of the present invention.

For example, when a test compound is found to inhibit (or suppress) the active transport activity of glucose in the case (ii) above by about 20% or more, preferably 30% or more and more preferably about 50% or more, as compared to the case (i), the test compound can be selected as a compound or its salts that inhibit the activity of the protein of the present invention.

Furthermore, by inserting a secreted alkaline phosphatase gene, a luciferase gene, etc. at the downstream of a SGLT homolog gene promoter for the protein of the present invention, expressing the same in the various cells described above and contacting the test compound with the cells to explore a compound or its salt activating or inhibiting the enzyme activity, the compound or its salts that promote or suppress the expression of the protein (SGLT homolog) of the present invention (namely, promote or inhibit the activity of the protein of the present invention) can be screened.

In a reporter gene assay using the promoter of a gene or the like, in which the gene product is considered to be responsible for regulating the expression of the protein, the present invention further provides a method of screening, which comprises regulating the activity in the assay.

Examples of the test compound include peptides, proteins, non-peptide compounds derived from biomaterials (carbohydrates, fats, etc.), synthetic compounds, fermentation products, cell extracts, plant extracts, animal tissue extracts, etc. These compounds may be novel compounds or publicly known compounds.

To perform the screening method described above, the cells capable of producing the protein of the present invention are cultured in a medium suitable for screening. Any medium is usable so far as it does not adversely affect the expression of a gene for the protein of the present invention.

As the cells capable of producing the protein of the present invention, there are used, for example, a host (transformant) transformed with a vector containing the DNA encoding the protein of the present invention. Preferably, animal cells such as COS7 cells, CHO cells, HEK293 cells, etc. are used as the host. For the screening, the transformant, in which the protein of the present invention has been secreted extracellularly or expressed in the cells, e.g., by culturing through the procedures described above, is preferably employed. The procedures for culturing the cells capable of expressing the protein of the present invention are similar to the culturing procedures for the transformant of the present invention described above. As the cells capable of producing the protein of the present invention, intestinal epithelial cells from human or warm-blooded animal capable of producing the SGLT homolog may also be used, in addition to the transformants described above. Furthermore, the cells in this case may be isolated cells or may be used in the form of tissues, organs, etc. containing these cells.

Examples of the test compound include peptides, proteins, non-peptide compounds, synthetic compounds, fermentation products, cell extracts, plant extracts, animal tissue extracts, etc.

The compound having the activity of promoting the activity of the protein of the present invention potentiates the activity of the protein of the present invention and is thus useful as a safe and low toxic pharmaceutical.

The compound or its salt obtained using the screening method or screening kit of the present invention is the compound selected from, for example, peptides, proteins, non-peptide compounds, synthetic compounds, fermentation products, cell extracts, plant extracts, animal tissue extracts, plasma, etc. The salts of these compounds used are those given above as the salts of the peptide of the present invention.

The gene encoding the protein of the present invention is also increasingly expressed in the small intestine and hence, the compound or its salts that regulate the expression of the gene encoding the protein of the present invention can be used, e.g., as postprandial hyperglycemia-improving agents or glucose absorption promoters. Preferably, the compound or its salts can be used as postprandial hyperglycemia-improving agents.

Therefore, the polynucleotide (e.g., DNA) of the present invention is useful as a reagent for screening the compound or its salts that regulate the expression of the gene encoding the protein of the present invention.

For the screening, there is a method of screening which comprises comparing (iii) the case that a cell capable of producing the protein of the present invention is cultured and (iv) the case that a cell capable of producing the protein used in the present invention is cultured in the presence of a test compound.

In the screening method described above, the expression level of the gene described above (specifically, the level of the protein of the present invention or the level of mRNA encoding the said protein) is determined in the cases of (iii) and (iv), followed by comparison.

Examples of the test compound and the cells capable of producing the protein of the present invention are the same as those described above.

The level of the protein of the present invention can be determined by publicly known methods, e.g., by measuring the aforesaid protein present in the cell extract, etc., using an antibody capable of recognizing the protein of the present invention, in accordance with methods like western blot analysis, ELISA, etc., or their modifications.

The expression level of the gene of the present invention can be determined by publicly known methods, e.g., in accordance with methods such as Northern blotting, Reverse transcription-polymerase chain reaction (RT-PCR), TaqMan polymerase chain reaction, or modifications thereof.

For example, when a test compound inhibits or enhances the expression of the gene in the case (iv) described above by at least about 20%, preferably at least 30% and more preferably at least about 50%, as compared to the case (iii) above, the test compound can be selected to be the compound capable of inhibiting or enhancing the activity of the protein of the present invention.

The screening kit of the present invention comprises the protein used in the present invention, its partial peptide or salts thereof, or the cell capable of producing the protein used in the present invention, or its partial peptide.

The compound or its salts obtained by using the screening method or screening kit of the present invention is a compound selected from the test compounds described above, e.g., peptides, proteins, non-peptide compounds, synthetic compounds, fermentation products, cell extracts, plant extracts, animal tissue extracts, plasma, etc., or its salts, and is also a compound or its salts that regulate the expression of the gene for the protein of the present invention.

The salts of these compounds used are those given above as the salts of the protein of the present invention.

The compound or its salts that regulate the activity of the present invention or the compound or its salts that regulate the expression of the gene encoding the protein of the present invention can be used, for example, as postprandial hyperglycemia-improving agents, glucose absorption promoters, or the like.

Where the compound or its salts obtained by using the screening method or screening kit of the present invention are used as the therapeutic/prophylactic agents described above, these compounds can be converted into pharmaceutical preparations in a conventional manner.

For example, the composition for oral administration includes solid or liquid preparations, specifically, tablets (including dragees and film-coated tablets), pills, granules, powdery preparations, capsules (including soft capsules), syrup, emulsions, suspensions, etc. Such a composition is manufactured by publicly known methods and contains a vehicle, a diluent or excipient conventionally used in the field of pharmaceutical preparations. Examples of the vehicle or excipient for tablets are lactose, starch, sucrose, magnesium stearate, etc.

Examples of the composition for parenteral administration are injectable preparations, suppositories, etc. The injectable preparations may include dosage forms such as intravenous, subcutaneous, intracutaneous and intramuscular injections, drip infusions, intraarticular injection, etc. These injectable preparations may be prepared by methods publicly known. For example, the injectable preparations may be prepared by dissolving, suspending or emulsifying the antibody or its salt described above in a sterile aqueous medium or an oily medium conventionally used for injections. As the aqueous medium for injections, there are, for example, physiological saline, an isotonic solution containing glucose and other auxiliary agents, etc., which may be used in combination with an appropriate solubilizing agent such as an alcohol (e.g., ethanol), a polyalcohol (e.g., propylene glycol, polyethylene glycol), a nonionic surfactant [e.g., polysorbate 80, HCO-50 (polyoxyethylene (50 mols) adduct of hydrogenated castor oil)], etc. As the oily medium, there are employed, e.g., sesame oil, soybean oil, etc., which may be used in combination with a solubilizing agent such as benzyl benzoate, benzyl alcohol, etc. The injection thus prepared is usually filled in an appropriate ampoule. The suppository used for rectal administration may be prepared by blending the aforesaid antibody or its salt with conventional bases for suppositories.

Advantageously, the pharmaceutical compositions for oral or parenteral use described above are prepared into pharmaceutical preparations with a unit dose suited to fit a dose of the active ingredients. Such unit dose preparations include, for example, tablets, pills, capsules, injections (ampoules), suppositories, etc. The amount of the aforesaid compound contained is generally 5 to 500 mg per dosage unit form; it is preferred that the aforesaid antibody is contained in about 5 to about 100 mg especially in the form of injection, and in 10 to 250 mg for the other forms.

Each composition described above may further contain other active components unless formulation causes any adverse interaction with the compound described above.

Since the pharmaceutical preparations thus obtained are safe and low toxic, they can be administered to human or warm-blooded animal (e.g., mouse, rat, rabbit, sheep, swine, bovine, horse, fowl, cat, dog, monkey, chimpanzee, etc.) orally or parenterally.

The dose of the compound or its salts may vary depending upon its action, target disease, subject to be administered, route of administration, etc. For example, when the compound or its salt that regulates the activity of the protein of the present invention is orally administered for the purpose of, e.g., improving postprandial hyperglycemia, the compound or its salt is generally administered to an adult (as 60 kg body weight) in a daily dose of about 0.1 to about 100 mg, preferably about 1.0 to about 50 mg and more preferably about 1.0 to about 20 mg. In parenteral administration, a single dose of the said compound or its salt may vary depending upon subject to be administered, target disease, etc. When the compound or its salt that regulates the activity of the protein of the present invention is administered to an adult (as 60 kg body weight) in the form of an injectable preparation for the purpose of, e.g., improving postprandial hyperglycemia, it is advantageous to administer the compound or its salt by way of intravenous injection in a daily dose of about 0.01 to about 30 mg, preferably about 0.1 to about 20 mg, and more preferably about 0.1 to about 10 mg. For other animal species, the corresponding dose as converted per 60 kg weight can be administered.

### (2a) Quantification for the protein of the present invention, it partial peptide or salts thereof

The antibody to the protein of the present invention (hereinafter sometimes merely referred to as the antibody of the present invention) is capable of specifically recognizing the protein of the present invention, and thus can be used for quantification of the protein of the present invention in a test sample fluid, in particular, for quantification by the sandwich immunoassay; etc.

That is, the present invention provides:
(i) a method of quantifying the protein of the present invention in a test sample fluid, which comprises competitively reacting the antibody of the present invention, a test sample fluid and a labeled form of the protein of the present invention, and measuring the ratio of the labeled form of the protein of the present invention bound to said antibody; and,
(ii) a method of quantifying the protein of the present invention in a test sample fluid, which comprises reacting a test sample fluid simultaneously or continuously with the antibody of the present invention immobilized on a carrier and another labeled antibody of the present invention, and then measuring the activity of the labeling agent on the insoluble carrier.

In the quantification method (ii) described above, it is preferred that one antibody is capable of recognizing the N-terminal region of the protein of the present invention, while another antibody is capable of reacting with the C-terminal region of the protein of the present invention.

The monoclonal antibody to the protein of the present invention (hereinafter sometimes referred to as the monoclonal antibody of the present invention) can be used to quantify the protein of the present invention. In addition, the protein can be detected by means of a tissue staining as well. For these purposes, the antibody molecule per se may be used or F(ab')₂, Fab' or Fab fractions of the antibody molecule may also be used.

The method of quantifying the protein of the present invention using the antibody of the present invention is not particularly limited. Any quantification method can be used, so long as the amount of antibody, antigen or antibody-antigen complex corresponding to the amount of antigen (e.g., the amount of the protein) in a test sample fluid can be detected by chemical or physical means and the amount of the antigen can be calculated from a standard curve prepared from standard solutions containing known amounts of the antigen. For such an assay method, for example, nephrometry, the competitive method, the immunometric method, the sandwich method, etc. are suitably used and in terms of sensitivity and specificity, it is particularly preferred to use the sandwich method described hereinafter.

Examples of the labeling agent used in the assay method using the labeling substance are radioisotopes, enzymes, fluorescent substances, luminescent substances, and the like. As the radioisotopes, there are used, e.g., [¹²⁵I], [¹³¹I], [³H], [¹⁴C], etc. The enzymes described above are preferably enzymes, which are stable and have a high specific activity, and include, e.g., β-galactosidase, β-glucosidase, an alkaline phosphatase, a peroxidase, malate dehydrogenase, etc. As the fluorescent substances, there are used, e.g., fluorescamine, fluorescein isothiocyanate, etc. As the luminescent substances described above there are used, e.g., luminol, a luminol derivative, luciferin, lucigenin, etc. Furthermore, the biotin-avidin system may be used as well for binding of an antibody or antigen to a labeling agent.

For immobilization of the antigen or antibody, physical adsorption may be used. Chemical binding techniques conventionally used for insolubilization or immobilization of proteins, enzymes, etc. may also be used. For carriers, there are used, e.g., insoluble polysaccharides such as agarose, dextran, cellulose, etc.; synthetic resin such as polystyrene, polyacrylamide, silicon, etc., and glass or the like.

In the sandwich method, the immobilized monoclonal antibody of the present invention is reacted with a test fluid (primary reaction), then with a labeled form of another monoclonal antibody of the present invention (secondary reaction), and the activity of the label on the immobilizing carrier is measured, whereby the amount of the protein of the present invention in the test fluid can be quantified. The order of the primary and secondary reactions may be reversed, and the reactions may be performed simultaneously or with an interval. The methods of labeling and immobilization can be performed by the methods described above. In the immunoassay by the sandwich method, the antibody used for immobilized or labeled antibodies is not necessarily one species, but a mixture of two or more species of antibody may be used to increase the measurement sensitivity.

In the methods of assaying the protein of the present invention by the sandwich method of the present invention, antibodies that bind to different sites of the protein of the present invention are preferably used as the monoclonal antibodies of the present invention used for the primary and secondary reactions. That is, in the antibodies used for the primary and secondary reactions are, for example, when the antibody used in the secondary reaction recognizes the C-terminal region of the protein of the present invention, it is preferable to use the antibody recognizing the region other than the C-terminal region for the primary reaction, e.g., the antibody recognizing the N-terminal region.

The monoclonal antibodies of the present invention can be used for the assay systems other than the sandwich method, for example, the competitive method, the immunometric method, nephrometry, etc.

In the competitive method, antigen in a test fluid and the labeled antigen are competitively reacted with antibody, and the unreacted labeled antigen (F) and the labeled antigen bound to the antibody (B) are separated (B/F separation). The amount of the label in B or F is measured, and the amount of the antigen in the test fluid is quantified. This reaction method includes a liquid phase method using a soluble antibody as an antibody, polyethylene glycol for B/F separation and a secondary antibody to the soluble antibody, and an immobilized method either using an immobilized antibody as the primary antibody, or using a soluble antibody as the primary antibody and immobilized antibody as the secondary antibody.

In the immunometric method, antigen in a test fluid and immobilized antigen are competitively reacted with a definite amount of labeled antibody, the immobilized phase is separated from the liquid phase, or antigen in a test fluid and an excess amount of labeled antibody are reacted, immobilized antigen is then added to bind the unreacted labeled antibody to the immobilized phase, and the immobilized phase is separated from the liquid phase. Then, the amount of the label in either phase is measured to quantify the antigen in the test fluid.

In the nephrometry, insoluble precipitate produced after the antigen-antibody reaction in gel or solution is quantified. When the amount of antigen in the test fluid is small and only a small amount of precipitate is obtained, laser nephrometry using scattering of laser is advantageously employed.

For applying each of these immunological methods to the quantification method of the present invention, any particular conditions or procedures are not required. Quantification system for the protein of the present invention or its salts is constructed by adding the usual technical consideration in the art to the conventional conditions and procedures. For the details of these general technical means, reference can be made to the following reviews and texts.

For example, Hiroshi Irie, ed. "Radioimmunoassay" (Kodansha, published in 1974), Hiroshi Irie, ed. "Sequel to the Radioimmunoassay" (Kodansha, published in 1979), Eiji Ishikawa, et al. ed. "Enzyme immunoassay" (Igakushoin, published in 1978), Eiji Ishikawa, et al. ed. "Immunoenzyme assay" (2nd ed.) (Igakushoin, published in 1982), Eiji Ishikawa, et al. ed. "Immunoenzyme assay" (3rd ed.) (Igakushoin, published in 1987), Methods in ENZYMOLOGY, Vol. 70 (Immunochemical Techniques (Part A)), ibid., Vol. 73 (Immunochemical Techniques (Part B)), ibid., Vol. 74 (Immunochemical Techniques (Part C)), ibid., Vol. 84 (Immunochemical Techniques (Part D: Selected Immunoassays)), ibid., Vol. 92 (Immunochemical Techniques (Part E: Monoclonal Antibodies and General Immunoassay Methods)), ibid., Vol. 121 (Immunochemical Techniques (Part I: Hybridoma Technology and Monoclonal Antibodies)) (all published by Academic Press Publishing).

As described above, the protein of the present invention can be quantified with high sensitivity, using the antibody of the present invention.

In addition, the antibody of the present invention can be used to detect the protein of the present invention, which is present in a test sample such as a body fluid, a tissue, etc. The antibody can also be used to prepare an antibody column for purification of the protein of the present invention, detect the protein of the present invention in each fraction upon purification, analyze the behavior of the protein of the present invention in the cells under investigation; etc.

### (3) Gene diagnostic agent

By using the DNA of the present invention, e.g., as a probe, the DNA can detect an abnormality (gene abnormality) of the DNA or mRNA encoding the protein of the present invention or its partial peptide in human or warm-blooded animal (e.g., rat, mouse, guinea pig, rabbit, fowl, sheep, swine, bovine, horse, cat, dog, monkey, chimpanzee, etc.). Therefore, the DNA of the present invention is useful as a gene diagnostic agent for detecting damages to the DNA or mRNA, its mutation, or decreased expression, increased expression, overexpression, etc. of the DNA or mRNA, and so on.

The gene diagnosis described above using the DNA of the present invention can be performed by, for example, the publicly known Northern hybridization assay or the PCR-SSCP assay (Genomics, 5, 874-879 (1989); Proceedings of the National Academy of Sciences of the United States of America, 86, 2766-2770 (1989)), etc.

### (4) Pharmaceutical comprising the antisense polynucleotide

The antisense polynucleotide of the present invention that binds to the DNA of the present invention complementarily to regulate the expression of said DNA is low toxic and can regulate (preferably suppress) the functions of the protein of the present invention or the DNA of the present invention in vivo. Thus, the antisense polynucleotide can be used, for example, as a postprandial hyperglycemia-improving agent, or the like.

Where the antisense polynucleotide described above is used as the aforesaid prophylactic/therapeutic agent, it can be prepared into pharmaceutical preparations by publicly known methods, which are provided for administration.

For example, when the antisense polynucleotide described above is used, the antisense polynucleotide alone is administered directly, or the antisense polynucleotide is inserted into an appropriate vector such as retrovirus vector, adenovirus vector, adenovirus-associated virus vector, etc., followed by treating in a conventional manner. The antisense polynucleotide may then be administered orally or parenterally to human or mammal (e.g., rat, rabbit, sheep, swine, bovine, cat, dog, monkey, etc.) in a conventional manner. The antisense polynucleotide may also be administered as it stands, or may be prepared in pharmaceutical preparations together with a physiologically acceptable carrier to assist its uptake, which are then administered by gene gun or through a catheter such as a catheter with a hydrogel. Alternatively, the antisense polynucleotide may be prepared into an aerosol, which is topically administered into the trachea as an inhaler.

Further for the purposes of improving pharmacokinetics, prolonging a half-life and improving intracellular uptake efficiency, the antisense polynucleotide described above is prepared into pharmaceutical preparations (injectable preparations) alone or together with a carrier such as liposome, etc. and the preparations may be administered intravenously, subcutaneously, or at the affected area, etc.

A dose of the antisense polynucleotide may vary depending on target disease, subject to be administered, route for administration, etc. For example, where the antisense polynucleotide is administered, e.g., for the purpose of improving postprandial hyperglycemia, the antisense polynucleotide is generally administered to an adult (60 kg body weight) in a daily dose of about 0.1 to 100 mg.

Furthermore, the antisense polynucleotide may also be used as an oligonucleotide probe for diagnosis to examine the presence of the DNA of the present invention in tissues or cells and states of its expression.

The antisense polynucleotide described above can, a double-stranded RNA (siRNA to the polynucleotide of the present invention) containing a part of RNA encoding the protein of the present invention, a ribozyme containing a part of RNA encoding the protein of the present invention, a decoy oligonucleotide to the DNA sequence to which the protein of the present invention is bound, etc. can also suppress the expression of the gene of the present invention to suppress the in vivo function of the protein used in the present invention or the DNA used in the present invention, and hence can be used, for example, as a postprandial hyperglycemia-improving agent, etc.

The double-stranded RNA can be designed based on a sequence of the polynucleotide of the present invention and manufactured by modifications of publicly known methods (e.g., Nature, 411, 494, 2001).

The ribozyme can be designed based on a sequence of the polynucleotide of the present invention and manufactured by modifications of publicly known methods (e.g., TRENDS in Molecular Medicine, 7, 221, 2001). For example, the ribozyme can be manufactured by ligating a publicly known ribozyme to a part of the RNA encoding the protein of the present invention. A part of the RNA encoding the protein of the present invention includes a portion proximal to a cleavage site on the RNA of the present invention, which may be cleaved by a publicly known ribozyme (RNA fragment).

The decoy oligonucleotide can be designed and manufactured, based on the DNA sequence to which the protein of the present invention is bound, by publicly known methods (e.g., The Journal of Clinical Investigation, 106, 1071, 2000) or modifications thereof. Specifically, the decoy oligonucleotide may be any one of oligonucleotides having a base sequence hybridizable under high stringent conditions to the base sequence, to which the protein of the present invention can bind. As the base sequence hybridizable to the sequence of the DNA, to which the protein of the present invention binds, there are employed, for example, base sequences having at least about 70% homology, preferably about 80% homology, more preferably at least about 90% homology and most preferably at least about 95% homology, to the sequence of DNA, to which the protein of the present invention binds.

Where the double-stranded RNA, ribozyme or decoy oligonucleotide described above is used as the prophylactic/therapeutic agent described above, the double-stranded RNA, ribozyme or decoy oligonucleotide is prepared into pharmaceutical preparations as in the antisense polynucleotide, and the preparations can be provided for administration.

### (5) Pharmaceutical comprising the antibody of the present invention

The antibody of the present invention, which has the activity of neutralizing the activity of the protein of the present invention can be used as a prophylactic/therapeutic agent, for example, a postprandial hyperglycemia-improving agent, or the like.

The prophylactic/therapeutic agent comprising the antibody of the present invention for the diseases described above is low toxic and can be administered to human or mammals (e.g., rats, rabbits, sheep, swine, bovine, cats, dogs, monkeys, etc.) orally or parenterally (e.g., intraarticularly) in the form of liquid preparation as it is or as a pharmaceutical composition of appropriate dosage form. The dose may vary depending upon subject to be administered, target disease, conditions, route of administration, etc. For example, when the agent is used, e.g., for the purpose of improving postprandial hyperglycemia, it is advantageous to administer the antibody of the present invention by way of a dry powder inhaler normally in a single dose of about 0.01 to about 20 mg/kg body weight, preferably about 0.1 to about 10 mg/kg body weight, and more preferably about 0.1 to about 5 mg/kg body weight, approximately 1 to 5 times per day. In other parenteral administration and oral administration, the agent can be administered in a dose corresponding to the dose given above. When the condition is especially severe, the dose may be increased according to the condition.

The antibody of the present invention can be administered per se or in the form of an appropriate pharmaceutical composition. The pharmaceutical composition used for the administration above comprises the antibody described above or its salts, pharmacologically acceptable carriers and dilutes or excipients. Such a composition can be provided in the form of pharmaceutical preparations suited for oral or parenteral administration (e.g., intraarticular administration). Preferably, the composition is provided as an inhaler.

The composition described above may further contain other active components unless formulation with the aforesaid antibody causes any adverse interaction.

### (6) DNA transgenic animal

The present invention provides a non-human mammal bearing DNA encoding the protein of the present invention, which is exogenous (hereinafter abbreviated as the exogenous DNA of the present invention) or its variant DNA (sometimes simply referred to as the exogenous variant DNA of the present invention).

That is, the present invention provides:
(1) A non-human mammal bearing the exogenous DNA of the present invention or its variant DNA;
(2) The mammal according to (1), wherein the non-human mammal is a rodent;
(3) The mammal according to (2), wherein the rodent is mouse or rat; and,
(4) A recombinant vector containing the exogenous DNA of the present invention or its variant DNA and capable of expressing in a mammal; etc.

The non-human mammal bearing the exogenous DNA of the present invention or its variant DNA (hereinafter simply referred to as the DNA transgenic animal of the present invention) can be prepared by transfecting a desired DNA into an unfertilized egg, a fertilized egg, a spermatozoon, a germinal cell containing a primordial germinal cell thereof, or the like, preferably in the embryogenic stage in the development of a non-human mammal (more preferably in the single cell or fertilized cell stage and generally before the 8-cell phase), by standard means, such as the calcium phosphate method, the electric pulse method, the lipofection method, the agglutination method, the microinjection method, the particle gun method, the DEAE-dextran method, etc. Also, it is possible to transfect the exogenous DNA of the present invention into a somatic cell, a living organ, a tissue cell, or the like by the DNA transfection methods, and utilize the transformant for cell culture, tissue culture, etc. In addition, these cells may be fused with the above-described germinal cell by a publicly known cell fusion method to prepare the DNA transgenic animal of the present invention.

Examples of the non-human mammal that can be used include bovine, swine, sheep, goat, rabbits, dogs, cats, guinea pigs, hamsters, mice, rats, etc. Above all, preferred are rodents, especially mice (e.g., C57B1/6 strain, DBA2 strain, etc. for a pure line and for a cross line, B6C3F₁ strain, BDF₁ strain B6D2F₁ strain, BALB/c strain, ICR strain, etc.), rats (Wistar, SD, etc.) or the like, since they are relatively short in ontogeny and life cycle from a standpoint of creating model animals for human disease.

"Mammals" in a recombinant vector that can be expressed in the mammals include the aforesaid non-human mammals, human, etc.

The exogenous DNA of the present invention refers to the DNA of the present invention that is once isolated/extracted from mammals, not the DNA of the present invention inherently possessed by the non-human mammals.

The mutant DNA of the present invention includes mutants resulting from variation (e.g., mutation, etc.) in the base sequence of the original DNA of the present invention, specifically DNAs resulting from base addition, deletion, substitution with other bases, etc. and further including abnormal DNA.

The abnormal DNA is intended to mean DNA that expresses the protein of the present invention which is abnormal and exemplified by the DNA, etc. that expresses a protein for suppressing the function of the protein of the present invention which is normal.

The exogenous DNA of the present invention may be any one of those derived from a mammal of the same species as, or a different species from, the mammal as the target animal. In transfecting the DNA of the present invention into the target animal, it is generally advantageous to use the DNA as a DNA construct in which the DNA is ligated downstream a promoter capable of expressing the DNA in the target animal. For example, in the case of transfecting the human DNA of the present invention, a DNA transgenic mammal that expresses the DNA of the present invention to a high level, can be prepared by microinjecting a DNA construct (e.g., vector, etc.) ligated with the human DNA of the present invention into a fertilized egg of the target non-human mammal downstream various promoters which are capable of expressing the DNA derived from various mammals (e.g., rabbits, dogs, cats, guinea pigs, hamsters, rats, mice, etc.) bearing the DNA of the present invention highly homologous to the human DNA.

As expression vectors for the protein of the present invention, there are Escherichia coli-derived plasmids, Bacillus subtilis-derived plasmids, yeast-derived plasmids, bacteriophages such as λ phage, retroviruses such as Moloney leukemia virus, etc., and animal viruses such as vaccinia virus, baculovirus, etc. Of these vectors, Escherichia coli-derived plasmids, Bacillus subtilis-derived plasmids, or yeast-derived plasmids, etc. are preferably used.

Examples of these promoters for regulating the DNA expression described above include 1) promoters for DNA derived from viruses (e.g., simian virus, cytomegalovirus, Moloney leukemia virus, JC virus, breast cancer virus, poliovirus, etc.), and 2) promoters derived from various mammals (human, rabbits, dogs, cats, guinea pigs, hamsters, rats, mice, etc.), for example, promoters of albumin, insulin II, uroplakin II, elastase, erythropoietin, endothelin, muscular creatine kinase, glial fibrillary acidic protein, glutathione S-transferase, platelet-derived growth factor β, keratins K1, K10 and K14, collagen types I and II, cyclic AMP-dependent protein kinase βI subunit, dystrophin, tartarate-resistant alkaline phosphatase, atrial natriuretic factor, endothelial receptor tyrosine kinase (generally abbreviated as Tie2), sodium-potassium adenosine triphosphorylase (Na,K-ATPase), neurofilament light chain, metallothioneins I and IIA, metalloproteinase I tissue inhibitor, MHC class I antigen (H-2L), H-ras, renin, dopamine β-hydroxylase, thyroid peroxidase (TPO), protein chain elongation factor 1α (EF-1α), β actin, α and β myosin heavy chains, myosin light chains 1 and 2, myelin base protein, thyroglobulins, Thy-1, immunoglobulins, H-chain variable region (VNP), serum amyloid component P, myoglobin, troponin C, smooth muscle α actin, preproencephalin A, vasopressin, etc. Among them, cytomegalovirus promoters, human protein elongation factor 1α (EF-1α) promoters, human and fowl β actin promoters, etc., which are capable of high expression in the whole body are preferred.

Preferably, the vectors described above have a sequence that terminates the transcription of the desired messenger RNA in the DNA transgenic animal (generally termed a terminator); for example, a sequence of each DNA derived from viruses and various mammals, and SV40 terminator of the simian virus and the like are preferably used.

In addition, for the purpose of enhancing the expression of the desired exogenous DNA to a higher level, the splicing signal and enhancer region of each DNA, a portion of the intron of an eukaryotic DNA may also be ligated at the 5' upstream of the promoter region, or between the promoter region and the translational region, or at the 3' downstream of the translational region, depending upon purposes.

The translational region for the normal protein of the present invention can be obtained using as a starting material the entire genomic DNA or its portion of liver, kidney, thyroid cell or fibroblast origin from human or various mammals (e.g., rabbits, dogs, cats, guinea pigs, hamsters, rats, mice, etc.) or of various commercially available genomic DNA libraries, or using cDNA prepared by a publicly known method from RNA of liver, kidney, thyroid cell or fibroblast origin as a starting material. Also, an exogenous abnormal DNA can produce the translational region through variation of the translational region of normal protein obtained from the cells or tissues described above by point mutagenesis.

The translational region can be prepared by a conventional DNA engineering technique, in which the DNA is ligated downstream the aforesaid promoter and if desired, upstream the translation termination site, as a DNA construct capable of being expressed in the transgenic animal.

The exogenous DNA of the present invention is transfected at the fertilized egg cell stage in a manner such that the DNA is certainly present in all the germinal cells and somatic cells of the target mammal. The fact that the exogenous DNA of the present invention is present in the germinal cells of the animal prepared by DNA transfection means that all offspring of the prepared animal will maintain the exogenous DNA of the present invention in all of the germinal cells and somatic cells thereof. The offspring of the animal that inherits the exogenous DNA of the present invention also have the exogenous DNA of the present invention in all of the germinal cells and somatic cells thereof.

The non-human mammal in which the normal exogenous DNA of the present invention has been transfected can be passaged as the DNA-bearing animal under ordinary rearing environment, by confirming that the exogenous DNA is stably retained by crossing.

By the transfection of the exogenous DNA of the present invention at the fertilized egg cell stage, the DNA is retained to be excess in all of the germinal and somatic cells. The fact that the exogenous DNA of the present invention is excessively present in the germinal cells of the prepared animal after transfection means that the DNA of the present invention is excessively present in all of the germinal cells and somatic cells thereof. The offspring of the animal that inherits the exogenous DNA of the present invention have excessively the DNA of the present invention in all of the germinal cells and somatic cells thereof.

It is possible to obtain homozygotic animals having the transfected DNA in both homologous chromosomes and breed male and female of the animal so that all the progeny have this DNA in excess.

In a non-human mammal bearing the normal DNA of the present invention, the normal DNA of the present invention has expressed at a high level, and may eventually develop hyperfunction in the function of the protein of the present invention by accelerating the function of endogenous normal DNA. Therefore, the animal can be utilized as a pathologic model animal for such a disease. For example, using the normal DNA transgenic animal of the present invention, it is possible to elucidate the mechanism of hyperfunction in the function of the protein of the present invention and the pathological mechanism of the disease associated with the protein of the present invention and to investigate how to treat these diseases.

Furthermore, a mammal transfected with the exogenous normal DNA of the present invention exhibits a symptom of increasing the protein of the present invention liberated. Thus, the animal is usable for screening test of postprandial hyperglycemia-improving agents, glucose absorption promoters, or the like.

On the other hand, a non-human mammal having the exogenous abnormal DNA of the present invention can be passaged under normal breeding conditions as the DNA-bearing animal by confirming stable retention of the exogenous DNA via crossing. Furthermore, the exogenous DNA of interest can be utilized as a starting material by inserting the DNA into the plasmid described above. The DNA construct with a promoter can be prepared by conventional DNA engineering techniques. The transfection of the abnormal DNA of the present invention at the fertilized egg cell stage is preserved to be present in all of the germinal and somatic cells of the target mammal. The fact that the abnormal DNA of the present invention is present in the germinal cells of the animal after DNA transfection means that all of the offspring of the prepared animal have the abnormal DNA of the present invention in all of the germinal and somatic cells. Such an offspring that passaged the exogenous DNA of the present invention will have the abnormal DNA of the present invention in all of the germinal and somatic cells. A homozygous animal having the introduced DNA on both of homologous chromosomes can be acquired, and by crossing these male and female animals, all the offspring can be bred to retain the DNA.

In a non-human mammal bearing the abnormal DNA of the present invention, the abnormal DNA of the present invention has expressed to a high level, and may eventually develop the function inactive type inadaptability to the protein of the present invention by inhibiting the functions of endogenous normal DNA. Therefore, the animal can be utilized as a pathologic model animal for such a disease. For example, using the abnormal DNA transgenic animal of the present invention, it is possible to elucidate the mechanism of the function inactive type inadaptability to the protein of the present invention and the pathological mechanism of the disease associated with the protein of the present invention and to investigate how to treat the disease.

More specifically, the transgenic animal of the present invention expressing the abnormal DNA of the present invention at a high level is expected to serve as an experimental model to elucidate the mechanism of the functional inhibition (dominant negative effect) of a normal protein by the abnormal protein of the present invention in the function inactive type inadaptability of the protein of the present invention.

Since a mammal bearing the abnormal exogenous DNA of the present invention shows a symptom of increasing the protein of the present invention liberated, the animal is also expected to serve for screening tests of, e.g., postprandial hyperglycemia-improving agents or glucose absorption promoters, preferably, for the screening test of postprandial hyperglycemia-improving agents.

Other potential applications of two kinds of the DNA transgenic animals of the present invention described above further include:
1) Use as a cell source for tissue culture;
2) Elucidation of the relation to a peptide that is specifically expressed or activated by the protein of the present invention, by direct analysis of DNA or RNA in tissues of the DNA transgenic animal of the present invention or by analysis of the peptide tissues expressed by the DNA;
3) Research on the function of cells derived from tissues that are usually cultured only with difficulty, using cells in tissues bearing the DNA cultured by a standard tissue culture technique;
4) Screening a drug that enhances the functions of cells using the cells described in 3) above; and,
5) Isolation and purification of the variant protein of the present invention and preparation of an antibody thereto; etc.

Furthermore, clinical conditions of a disease associated wit the protein of the present invention, including the function inactive type inadaptability to the protein of the present invention can be determined by using the DNA transgenic animal of the present invention. Also, pathological findings on each organ in a disease model associated with the protein of the present invention can be obtained in more detail, leading to the development of a new method for treatment as well as the research and therapy of any secondary diseases associated with the disease.

It is also possible to obtain a free DNA-transfected cell by withdrawing each organ from the DNA transgenic animal of the present invention, mincing the organ and degrading with a proteinase such as trypsin, etc., followed by establishing the line of culturing or cultured cells. Furthermore, the DNA transgenic animal of the present invention can serve to identify cells capable of producing the protein of the present invention, and to study in association with apoptosis, differentiation or propagation or on the mechanism of signal transduction in these properties to inspect any abnormality therein. Accordingly, the DNA transgenic animal can provide an effective research material for the protein of the present invention and for investigation of the function and effect thereof.

To develop a drug for the treatment of diseases associated with the protein of the present invention, including the function inactive type inadaptability to the protein of the present invention, using the DNA transgenic animal of the present invention, an effective and rapid method for screening can be provided by using the method for inspection and the method for quantification, etc. described above. It is also possible to investigate and develop a method for DNA therapy for the treatment of diseases associated with the protein of the present invention, using the DNA transgenic animal of the present invention or a vector capable of expressing the exogenous DNA of the present invention.

### (7) Knockout animal

The present invention provides a non-human mammal embryonic stem cell bearing the DNA of the present invention inactivated and a non-human mammal deficient in expressing the DNA of the present invention.

Thus, the present invention provides:
(1) A non-human mammal embryonic stem cell in which the DNA of the present invention is inactivated;
(2) The embryonic stem cell according to (1), wherein the DNA is inactivated by introducing a reporter gene (e.g., β-galactosidase gene derived from Escherichia coli);
(3) The embryonic stem cell according to (1), which is resistant to neomycin;
(4) The embryonic stem cell according to (1), wherein the non-human mammal is a rodent;
(5) The embryonic stem cell according to (4), wherein the rodent is mouse;
(6) A non-human mammal deficient in expressing the DNA of the present invention, wherein the DNA is inactivated;
(7) The non-human mammal according to (6), wherein the DNA is inactivated by inserting a reporter gene (e.g., β-galactosidase derived from Escherichia coli) therein and the reporter gene is capable of being expressed under control of a promoter for the DNA of the present invention;
(8) The non-human mammal according to (6), which is a rodent;
(9) The non-human mammal according to (8), wherein the rodent is mouse; and,
(10) A method of screening a compound that promotes or inhibits (preferably inhibits) the promoter activity to the DNA of the present invention, which comprises administering a test compound to the mammal of (7) and detecting expression of the reporter gene.

The non-human mammal embryonic stem cell in which the DNA of the present invention is inactivated refers to a non-human mammal embryonic stem cell that suppresses the ability of the non-human mammal to express the DNA by artificially mutating the DNA of the present invention, or the DNA has no substantial ability to express the protein of the present invention (hereinafter sometimes referred to as the knockout DNA of the present invention) by substantially inactivating the activities of the protein of the present invention encoded by the DNA (hereinafter merely referred to as ES cell).

As the non-human mammal, the same examples as described above apply.

Techniques for artificially mutating the DNA of the present invention include deletion of a part or all of the DNA sequence and insertion of or substitution with other DNA, by genetic engineering. By these variations, the knockout DNA of the present invention may be prepared, for example, by shifting the reading frame of a codon or by disrupting the function of a promoter or exon.

Specifically, the non-human mammal embryonic stem cell in which the DNA of the present invention is inactivated (hereinafter merely referred to as the ES cell with the DNA of the present invention inactivated or the knockout ES cell of the present invention) can be obtained by, for example, isolating the DNA of the present invention that the desired non-human mammal possesses, inserting a DNA fragment having a DNA sequence constructed by inserting a drug resistant gene such as a neomycin resistant gene or a hygromycin resistant gene, or a reporter gene such as lacZ (β-galactosidase gene) or cat (chloramphenicol acetyltransferase gene), etc. into its exon site thereby to disable the functions of exon, or integrating to a chromosome of the target animal by, e.g., homologous recombination, a DNA sequence that terminates gene transcription (e.g., polyA additional signal, etc.) in the intron between exons, thus inhibiting the synthesis of complete messenger RNA and eventually destroying the gene (hereinafter simply referred to as a targeting vector). The thus-obtained ES cells to the southern hybridization analysis with a DNA sequence on or near the DNA of the present invention as a probe, or to PCR analysis with a DNA sequence on the targeting vector and another DNA sequence near the DNA of the present invention which is not included in the targeting vector as primers, to select the knockout ES cell of the present invention.

The parent ES cells to inactivate the DNA of the present invention by homologous recombination, etc. may be of a strain already established as described above, or may originally be established in accordance with a modification of the known method by Evans and Kaufman described above. For example, in the case of mouse ES cells, currently it is common practice to use ES cells of the 129 strain. However, since their immunological background is obscure, the C57BL/6 mouse or the BDF₁ mouse (F₁ between C57BL/6 and DBA/2), wherein the low ovum availability per C57BL/6 in the C57BL/6 mouse has been improved by crossing with DBA/2, may be preferably used, instead of obtaining a pure line of ES cells with the clear immunological genetic background and for other purposes. The BDF₁ mouse is advantageous in that, when a pathologic model mouse is generated using ES cells obtained therefrom, the genetic background can be changed to that of the C57BL/6 mouse by back-crossing with the C57BL/6 mouse, since its background is of the C57BL/6 mouse, as well as being advantageous in that ovum availability per animal is high and ova are robust.

In establishing ES cells, blastocytes at 3.5 days after fertilization are commonly used. In the present invention, embryos are preferably collected at the 8-cell stage, after culturing until the blastocyte stage; the embryos are used to efficiently obtain a large number of early stage embryos.

Although the ES cells used may be of either sex, male ES cells are generally more convenient for generation of a germ cell line chimera. It is also desirable that sexes are identified as soon as possible to save painstaking culture time.

Methods for sex identification of the ES cell include the method in which a gene in the sex-determining region on the Y-chromosome is amplified by the PCR process and detected. When this method is used, one colony of ES cells (about 50 cells) is sufficient for sex-determination analysis, which karyotype analysis, for example G-banding method, requires about 10⁶ cells; therefore, the first selection of ES cells at the early stage of culture can be based on sex identification, and male cells can be selected early, which saves a significant amount of time at the early stage of culture.

Also, second selection can be achieved by, for example, confirmation of the number of chromosomes by the G-banding method. It is usually desirable that the chromosome number of the obtained ES cells be 100% of the normal number. However, when it is difficult to obtain the cells having the normal number of chromosomes due to physical operations, etc. in the cell establishment, it is desirable that the ES cell is again cloned to a normal cell (e.g., in a mouse cell having the number of chromosomes being 2n = 40) after knockout of the gene of the ES cells.

Although the embryonic stem cell line thus obtained shows a very high growth potential, it must be subcultured with great care, since it tends to lose its ontogenic capability. For example, the embryonic stem cell line is cultured at about 37°C in a carbon dioxide incubator (preferably 5 % carbon dioxide and 95 % air, or 5% oxygen, 5% carbon dioxide and 90% air) in the presence of LIF (1 to 10000 U/ml) on appropriate feeder cells such as STO fibroblasts, treated with a trypsin/EDTA solution (normally 0.001 to 0.5% trypsin/0.1 to about 5 mM EDTA, preferably about 0.1 % trypsin/1 mM EDTA) at the time of passage to obtain separate single cells, which are then plated on freshly prepared feeder cells. This passage is normally conducted every 1 to 3 days; it is desirable that cells be observed at the passage and cells found to be morphologically abnormal in culture, if any, be abandoned.

Where ES cells are allowed to reach a high density in mono-layers or to form cell aggregates in suspension under appropriate conditions, it is possible to differentiate the ES cells to various cell types, for example, pariental and visceral muscles, cardiac muscle or the like [M. J. Evans and M. H. Kaufman, Nature, 292, 154, 1981; G. R. Martin, Proc. Natl. Acad. Sci. U.S.A., 78, 7634, 1981; T. C. Doetschman et al., Journal of Embryology Experimental Morphology, 87, 27, 1985]. The cells deficient in expression of the DNA of the present invention, which are obtained from the differentiated ES cells of the present invention, are useful for cytological study of the protein of the present invention in vitro.

The non-human mammal deficient in expression of the DNA of the present invention can be identified from a normal animal by measuring the mRNA level in the subject animal by a publicly known method, and indirectly comparing the degrees of expression.

As the non-human mammal, the same examples given above apply.

With respect to the non-human mammal deficient in expression of the DNA of the present invention, the DNA of the present invention can be knockout by transfecting a targeting vector, prepared as described above, to mouse embryonic stem cells or mouse oocytes, and conducting homologous recombination in which a targeting vector DNA sequence, wherein the DNA of the present invention is inactivated by the transfection, is replaced with the DNA of the present invention on a chromosome of a mouse embryonic stem cell or mouse embryo.

The knockout cells with the disrupted DNA of the present invention can be identified by the southern hybridization analysis using as a probe a DNA fragment on or near the DNA of the present invention, or by the PCR analysis using as primers a DNA sequence on the targeting vector and another DNA sequence at the proximal region of other than the DNA of the present invention derived from mouse used in the targeting vector. When non-human mammal stem cells are used, a cell line wherein the DNA of the present invention is inactivated by homologous recombination is cloned; the resulting clones are injected to, e.g., a non-human mammalian embryo or blastocyst, at an appropriate stage such as the 8-cell stage. The resulting chimeric embryos are transplanted to the uterus of the pseudopregnant non-human mammal. The resulting animal is a chimeric animal constructed with both cells having the normal locus of the DNA of the present invention and those having an artificially mutated locus of the DNA of the present invention.

When some germ cells of the chimeric animal have a mutated locus of the DNA of the present invention, an individual, which entire tissue is composed of cells having a mutated locus of the DNA of the present invention can be selected from a series of offspring obtained by crossing between such a chimeric animal and a normal animal, e.g., by coat color identification, etc. The individuals thus obtained are normally deficient in heterozygous expression of the protein of the present invention. The individuals deficient in homozygous expression of the protein of the present invention can be obtained from offspring of the intercross between those deficient in heterozygous expression of the protein of the present invention.

When an oocyte is used, a DNA solution may be injected, e.g., into the prenucleus by microinjection thereby to obtain a transgenic non-human mammal having a targeting vector introduced in its chromosome. From such transgenic non-human mammals, those having a mutation at the locus of the DNA of the present invention can be obtained by selection based on homologous recombination.

As described above, the individuals in which the DNA of the present invention is knockout permit passage rearing under ordinary rearing conditions, after the individuals obtained by their crossing have proven to have been knockout.

Furthermore, the genital system may be obtained and retained by conventional methods. That is, by crossing male and female animals each having the inactivated DNA, homozygote animals having the inactivated DNA in both loci can be obtained. The homozygotes thus obtained may be reared so that one normal animal and two or more homozygotes are produced from a mother animal to efficiently obtain such homozygotes. By crossing male and female heterozygotes, homozygotes and heterozygotes having the inactivated DNA are proliferated and passaged.

The non-human mammal embryonic stem cell, in which the DNA of the present invention is inactivated, is very useful for preparing a non-human mammal deficient in expression of the DNA of the present invention.

Since the non-human mammal, in which the DNA of the present invention is inactivated, lacks various biological activities derived from the protein of the present invention, such an animal can be a disease model suspected of inactivated biological activities of the protein of the present invention and thus, offers an effective study to investigate the causes for and therapy for these diseases.

### (8a) Method of screening the compound having therapeutic/prophylactic effects on diseases caused by deficiency, damages, etc. of the DNA of the present invention

The non-human mammal deficient in expression of the DNA of the present invention can be employed for screening the compound having therapeutic/prophylactic effects on diseases caused by deficiency, damages, etc. of the DNA of the present invention.

As the non-human mammal deficient in expression of the DNA of the present invention, which can be employed for the screening method, the same examples as described above apply.

Examples of the test compound include peptides, proteins, non-peptide compounds, synthetic compounds, fermentation products, cell extracts, plant extracts, animal tissue extracts, plasma, etc. These compounds may be novel compounds or publicly known compounds.

Specifically, the non-human mammal deficient in expression of the DNA of the present invention is treated with a test compound, comparison is made with an intact animal for control and a change in each organ, tissue, disease conditions, etc. of the animal is used as an indicator to assess the therapeutic/prophylactic effects of the test compound.

For treating an animal to be tested with a test compound, for example, oral administration, intravenous injection, etc. are applied, and the treatment can be appropriately selected depending on conditions of the test animal, properties of the test compound, etc. Furthermore, a dose of the test compound to be administered can be appropriately chosen depending on the administration route, nature of the test compound, etc.

The compound obtained by the screening method above may form salts, and may be used in the form of salts with physiologically acceptable acids (e.g., inorganic acids, organic acids, etc.) or bases (e.g., alkali metal salts), preferably in the form of physiologically acceptable acid addition salts. Examples of such salts are salts with inorganic acids (e.g., hydrochloric acid, phosphoric acid, hydrobromic acid, sulfuric acid, etc.), salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid, etc.) and the like.

A pharmaceutical comprising the compound obtained by the above screening method or salts thereof can be manufactured in a manner similar to the method for preparing the pharmaceutical comprising the protein of the present invention described hereinabove.

Since the pharmaceutical preparation thus obtained is safe and low toxic, it can be administered to human or mammal (e.g., rat, mouse, guinea pig, rabbit, sheep, swine, bovine, horse, cat, dog, monkey, etc.).

The dose of the compound or its salt may vary depending upon target disease, subject to be administered, route of administration, etc. For example, when the compound is orally administered, the compound is administered to the adult patient with postprandial hyperglycemia (as 60 kg body weight) generally in a dose of about 0.1 to 100 mg, preferably about 1.0 to 50 mg and more preferably about 1.0 to 20 mg. In parenteral administration, a single dose of the compound may vary depending upon subject to be administered, target disease, etc. When the compound is administered to the adult patient with postprandial hyperglycemia (as 60 kg body weight) in the form of an injectable preparation, it is advantageous to administer the compound in a single dose of about 0.01 to about 30 mg, preferably about 0.1 to about 20 mg and more preferably about 0.1 to about 10 mg a day. For other animal species, the corresponding dose as converted per 60 kg weight can be administered.

### (8b) Method of screening a compound that promotes or inhibits the activity of a promoter to the DNA of the present invention

The present invention provides a method of screening a compound or its salts that promote or inhibit the activity of a promoter to the DNA of the present invention, which comprises administering a test compound to a non-human mammal deficient in expression of the DNA of the present invention and detecting the expression of a reporter gene.

In the screening method described above, an animal in which the DNA of the present invention is inactivated by introducing a reporter gene and the reporter gene is expressed under control of a promoter to the DNA of the present invention is used as the non-human mammal deficient in expression of the DNA of the present invention, which is selected from the aforesaid non-human mammals deficient in expression of the DNA of the present invention.

The same examples of the test compound apply to specific compounds described above.

As the reporter gene, the same specific examples apply to this screening method. Preferably, there are used β-galactosidase (lacZ), soluble alkaline phosphatase gene, luciferase gene and the like.

Since the reporter gene is present under control of a promoter to the DNA of the present invention in the non-human mammal deficient in expression of the DNA of the present invention wherein the DNA of the present invention is substituted with the reporter gene, the activity of the promoter can be detected by tracing the expression of a substance encoded by the reporter gene.

When a part of the DNA region encoding the protein of the present invention is substituted with, e.g., β-galactosidase gene (lacZ) derived from *Esclzerichia coli,* β-galactosidase is expressed in a tissue where the protein of the present invention should originally be expressed, instead of the protein of the present invention. Thus, the state of expression of the protein of the present invention can be readily observed in vivo of an animal by staining with a reagent, e.g., 5-bromo-4-chloro-3-indolyl-β-galactopyranoside (X-gal) which is substrate for β-galactosidase. Specifically, a mouse deficient in the protein of the present invention, or its tissue section is fixed with glutaraldehyde, etc. After washing with phosphate buffered saline (PBS), the system is reacted with a staining solution containing X-gal at room temperature or about 37°C for approximately 30 minutes to an hour. After the β-galactosidase reaction is terminated by washing the tissue preparation with 1 mM EDTA/PBS solution, the color formed is observed. Alternatively, mRNA encoding lacZ may be detected in a conventional manner.

The compound or salts thereof obtained using the screening method described above are compounds that are selected from the test compounds described above and that promote or inhibit the promoter activity to the DNA of the present invention.

The compound obtained by the screening method above may form salts, and may be used in the form of salts with physiologically acceptable acids (e.g., inorganic acids, etc.) or bases (e.g., alkali metals, etc.) or the like, especially in the form of physiologically acceptable acid addition salts. Examples of such salts are salts with inorganic acids (e.g., hydrochloric acid, phosphoric acid, hydrobromic acid, sulfuric acid, etc.), salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid, etc.) and the like.

The compound or its salts that promote or inhibit the promoter activity to the DNA of the present invention can regulate the expression of the protein of the present invention and can regulate the functions of the protein. Thus, the compound or its salt is useful as postprandial hyperglycemia-improving agents or glucose absorption promoters, preferably as postprandial hyperglycemia-improving agents.

In addition, compounds derived from the compound obtained by the screening described above may also be used as well.

A pharmaceutical comprising the compound obtained by the above screening method or salts thereof can be manufactured in a manner similar to the method for preparing the pharmaceutical comprising the protein of the present invention described above.

Since the pharmaceutical preparation thus obtained is safe and low toxic, it can be administered to human or mammal (e.g., rat, mouse, guinea pig, rabbit, sheep, swine, bovine, horse, cat, dog, monkey, etc.).

A dose of the compound or salts thereof may vary depending on target disease, subject to be administered, route for administration, etc.; when the compound that inhibits the promoter activity to the DNA of the present invention is orally administered, the compound is administered to the adult patient with postprandial hyperglycemia (as 60 kg body weight) normally in a daily dose of about 0.1 to 100 mg, preferably about 1.0 to 50 mg and more preferably about 1.0 to 20 mg. In parenteral administration, a single dose of the compound varies depending on subject to be administered, target disease, etc. but when the compound of inhibiting the promoter activity to the DNA of the present invention is administered to the adult patient with postprandial hyperglycemia (as 60 kg body weight) in the form of injectable preparation, it is advantageous to administer the compound intravenously to the patient in a daily dose of about 0.01 to about 30 mg, preferably about 0.1 to about 20 mg and more preferably about 0.1 to about 10 mg. For other animal species, the corresponding dose as converted per 60 kg weight can be administered.

As stated above, the non-human mammal deficient in expression of the DNA of the present invention is extremely useful for screening the compound or its salt that promotes or inhibits the promoter activity to the DNA of the present invention and, can greatly contribute to elucidation of causes for various diseases suspected of deficiency in expression of the DNA of the present invention and for the development of prophylactic/therapeutic agents for these diseases.

In addition, a so-called transgenic animal (gene transgenic animal) can be prepared by using a DNA containing the promoter region of the protein of the present invention, ligating genes encoding various proteins at the downstream and injecting the same into oocyte of an animal. It is thus possible to synthesize the protein therein specifically and study its activity in vivo. When an appropriate reporter gene is ligated to the promoter site described above and a cell line that expresses the gene is established, the resulting system can be utilized as the search system for a low molecular compound having the action of specifically promoting or inhibiting the in vivo productivity of the protein itself of the present invention.

In the specification and drawings, the codes of bases, amino acids, etc. are denoted in accordance with the IUPAC-IUB Commission on Biochemical Nomenclature or by the common codes in the art, examples of which are shown below. For amino acids that may have the optical isomer, L form is presented unless otherwise indicated.

| | |
|---|---|
| DNA | deoxyribonucleic acid |
| cDNA | complementary deoxyribonucleic acid |
| A | adenine |
| T | thymine |
| G | guanine |
| C | cytosine |
| RNA | ribonucleic acid |
| mRNA | messenger ribonucleic acid |
| dATP | deoxyadenosine triphosphate |
| dTTP | deoxythymidine triphosphate |
| dGTP | deoxyguanosine triphosphate |
| dCTP | deoxycytidine triphosphate |
| ATP | adenosine triphosphate |
| EDTA | ethylenediaminetetraacetic acid |
| SDS | sodium dodecyl sulfate |
| Gly | glycine |
| Ala | alanine |
| Val | valine |
| Leu | leucine |
| Ile | isoleucine |
| Ser | serine |
| Thr | threonine |
| Cys | cysteine |
| Met | methionine |
| Glu | glutamic acid |
| Asp | aspartic acid |
| Lys | lysine |
| Arg | arginine |
| His | histidine |
| Phe | phenylalanine |
| Tyr | tyrosine |
| Trp | tryptophan |
| Pro | proline |
| Asn | asparagine |
| Gln | glutamine |
| pGlu | pyroglutamic acid |
| Sec | selenocysteine |

Substituents, protecting groups and reagents generally used in this specification are presented as the codes below.

| | |
|---|---|
| Me | methyl group |
| Et | ethyl group |
| Bu | butyl group |
| Ph | phenyl group |
| TC | thiazolidine-4(R)-carboxamido group |
| Tos | p-toluenesulfonyl |
| CHO | formyl |
| Bzl | benzyl |
| Cl₂-Bzl | 2,6-dichlorobenzyl |
| Bom | benzyloxymethyl |
| Z | benzyloxycarbonyl |
| Cl-Z | 2-chlorobenzyloxycarbonyl |
| Br-Z | 2-bromobenzyl oxycarbonyl |
| Boc | t-butoxycarbonyl |
| DNP | dinitrophenol |
| Trt | trityl |
| Bum | t-butoxymethyl |
| Fmoc | N-9-fluorenyl methoxycarbonyl |
| HOBt | 1-hydroxybenztriazole |
| HOOBt | 3,4-dihydro-3-hydroxy-4-oxo-1,2,3-benzotriazine |
| HONB | 1-hydroxy-5-norbornene-2,3-dicarboxyimide |
| DCC | N,N'-dicyclohexylcarbodiimide |

The sequence identification numbers in the sequence listing of the specification indicate the following sequences.

### [SEQ ID NO: 1]

This shows the amino acid sequence of human SGLT homolog protein.

### [SEQ ID NO: 2]

This shows the base sequence of DNA encoding the human SGLT homolog protein having the amino acid sequence represented by SEQ ID NO: 1.

### [SEQ ID NO: 3]

This shows the amino acid sequence of mouse SGLT homolog protein.

### [SEQ ID NO: 4]

This shows the base sequence of DNA encoding the mouse SGLT homolog protein having the amino acid sequence represented by SEQ ID NO: 3.

### [SEQ ID NO: 5]

This shows the amino acid sequence of rat SGLT homolog protein.

### [SEQ ID NO: 6]

This shows the base sequence of DNA encoding the rat SGLT homolog protein having the amino acid sequence represented by SEQ ID NO: 5.

### [SEQ ID NO: 7]

This shows the base sequence of the primer used in EXAMPLE 2(1).

### [SEQ ID NO: 8]

This shows the base sequence of the primer used in EXAMPLE 2(1).

### [SEQ ID NO: 9]

This shows the base sequence of the probe used in EXAMPLE 2(1).

### [SEQ ID NO: 10]

This shows the base sequence of the primer used in EXAMPLE 2(1).

### [SEQ ID NO: 11]

This shows the base sequence of the primer used in EXAMPLE 2(1).

### [SEQ ID NO: 12]

This shows the base sequence of the primer used in EXAMPLE 2(2).

### [SEQ ID NO: 13]

This shows the base sequence of the primer used in EXAMPLE 2(2).

### [SEQ ID NO: 14]

This shows the base sequence of the probe used in EXAMPLE 2(2).

### [SEQ ID NO: 15]

This shows the base sequence of the primer used in EXAMPLE 2(2).

### [SEQ ID NO: 16]

This shows the base sequence of the primer used in EXAMPLE 2(2).

### [SEQ ID NO: 17]

This shows the amino acid sequence of the peptide used in EXAMPLE 4.

### [SEQ ID NO: 18]

This shows the base sequence of the primer used in EXAMPLE 5(1).

### [SEQ ID NO: 19]

This shows the base sequence of the primer used in EXAMPLE 5(1).

### [SEQ ID NO: 20]

This shows the base sequence of the probe used in EXAMPLE 5(1).

### [SEQ ID NO: 21]

This shows the base sequence of the primer used in EXAMPLE 5(1).

### [SEQ ID NO: 22]

This shows the base sequence of the primer used in EXAMPLE 5(1).

### [SEQ ID NO: 23]

This shows the base sequence of the primer used in EXAMPLE 5(2).

### [SEQ ID NO: 24]

This shows the base sequence of the primer used in EXAMPLE 5(2).

### [SEQ ID NO: 25]

This shows the base sequence of the probe used in EXAMPLE 5(2).

### [SEQ ID NO: 26]

This shows the base sequence of the primer used in EXAMPLE 5(2).

### [SEQ ID NO: 27]

This shows the base sequence of the primer used in EXAMPLE 5(2).

### [SEQ ID NO: 28]

This shows the base sequence of the primer used in EXAMPLE 6.

### [SEQ ID NO: 29]

This shows the base sequence of the primer used in EXAMPLE 6.

### [SEQ ID NO: 30]

This shows the base sequence of the primer used in EXAMPLE 6.

### [SEQ ID NO: 31]

This shows the base sequence of the primer used in EXAMPLE 6.

### [SEQ ID NO: 32]

This shows the base sequence of the primer used in EXAMPLE 6.

### [SEQ ID NO: 33]

This shows the base sequence of the primer used in EXAMPLE 6.

### [SEQ ID NO: 34]

This shows the base sequence of the primer used in EXAMPLE 6.

### [SEQ ID NO: 35]

This shows the base sequence of the primer used in EXAMPLE 6.

### [SEQ ID NO: 36]

This shows the base sequence of the primer used in EXAMPLE 6.

### [SEQ ID NO: 37]

This shows the base sequence of the primer used in EXAMPLE 6.

### [SEQ ID NO: 38]

This shows the base sequence of the primer used in EXAMPLE 6.

### [SEQ ID NO: 39]

This shows the base sequence of the primer used in EXAMPLE 6.

### [SEQ ID NO: 40]

This shows the base sequence of the primer used in EXAMPLE 6.

### [SEQ ID NO: 41]

This shows the base sequence of the primer used in EXAMPLE 6.

### [SEQ ID NO: 42]

This shows the base sequence of the primer used in EXAMPLE 6.

### [SEQ ID NO: 43]

This shows the base sequence of the primer used in EXAMPLE 6.

### [SEQ ID NO: 44]

This shows the base sequence of the primer used in EXAMPLE 6.

### [SEQ ID NO: 45]

This shows the base sequence of the primer used in EXAMPLE 6.

### [SEQ ID NO: 46]

This shows the base sequence of the primer used in EXAMPLE 6.

### [SEQ ID NO: 47]

This shows the base sequence of the primer used in EXAMPLE 6.

### [SEQ ID NO: 48]

This shows the base sequence of the primer used in EXAMPLE 6.

### [SEQ ID NO: 49]

This shows the base sequence of the primer used in EXAMPLE 6.

### [SEQ ID NO: 50]

This shows the amino acid sequence of the hamster SGLT homolog protein.

### [SEQ ID NO: 51]

This shows the base sequence of DNA encoding the hamster SGLT homolog protein having the amino acid sequence represented by SEQ ID NO: 50.

### [SEQ ID NO: 52]

This shows the amino acid sequence of the hamster SGLT1 protein.

### [SEQ ID NO: 53]

This shows the base sequence of DNA encoding the hamster SGLT1 protein having the amino acid sequence represented by SEQ ID NO: 53.

### [SEQ ID NO: 54]

This shows the base sequence of the primer used in EXAMPLE 10.

### [SEQ ID NO: 55]

This shows the base sequence of the primer used in EXAMPLE 10.

### [SEQ ID NO: 56]

This shows the base sequence of the primer used in EXAMPLE 10.

### [SEQ ID NO: 57]

This shows the base sequence of the primer used in EXAMPLE 10.

### EXAMPLES

Hereinafter, the present invention will be described specifically with reference to EXAMPLES but is not deemed to be limited thereto.

### EXAMPLE 1

### Determination of glucose uptake-suppressing action by phlorizin

In accordance with the procedures described in Example 4 of WO 02/53738, the CHO cell lines expressing the human SGLT homolog (hSGLTh), mouse SGLT homolog (mSGLTh), rat SGLT homolog (rSGLTh), human SGLT1(hSGLT1) and human SGLT2 (hSGLT2) were prepared, respectively, and used for experiments. The experimental uptake of α-methyl glucose, which is a glucose analog selectively taken up into the cells by SGLT, was carried out according to the method described in Am. J. Physiol., 270: G833-G843, 1996 and J. Clin. Invest., 93: 397-404, 1994. The cells were plated on 100 µl of DMEM containing 10% FBS charged in a 96-well plate in a cell density of 1 x 10⁵ cells/well, followed by culturing overnight at 37°C. The cells were washed 3 times with 150 µl of a buffer solution (125 mM N-Methyl-D-Glucamine, 1.2 mM KH₂PO₄, 2.5 mM CaCl₂, 1.2 mM MgSO₄, 4 mM Glutamine, 10 mM HEPES (pH 7.2), 0.1 mg/ml BSA) and cultured for an hour in the same buffer solution to remove the remaining glucose. The buffer solution was removed and replaced with 90 µl of the same buffer solution or the buffer solution containing Nacl or NaCl + Phlorizin (Sigma) in place of N-Methyl-D-Glucamine (NMDG). After one-hour incubation with 10 µl of 1 mM α-methyl glucose (containing 0.02 µCi of [¹⁴C]-α-Methyl Glucose (Amersham Pharmacia Biotech)) per well, the cells were washed 3 times with 200 µl of chilled PBS buffer. The radioactivity of ¹⁴C taken up into the cells was counted with 100 µl of a liquid scintillator added per well.

The results are shown in TABLES 1 and 2.

**[TABLE 1]**

| Comparison of hSGLT1, hSGLT2 and hSGLTh in phlorizin resistance | | | |
|---|---|---|---|
| | Uptake of α-Methyl Glucose (% based on Control Group) | | |
| | hSGLT1 | hSGLT2 | hSGLTh |
| Control Group (added with NMDG/not with NaCl) | 100± 18 | 100±15 | 100± 12 |
| Group added with NaCl | 1039± 186 | 830±99 | 767± 85 |
| Group added with NaCl + 3 µM phlorizin | 244± 48 | 139±23 | 570± 142 |
| Group added with NaCl + 100 µM phlorizin | 60± 14 | 48± 8 | 124± 19 |

**[TABLE 2]**

| Comparison of mSGLTh and rSGLTh in phloridzin resistance | | |
|---|---|---|
| | Uptake of α-Methyl Glucose (% based on Control Group) | |
| | mSGLTh | rSGLTh |
| Control Group (added with NMDG not added with NaCl) | 100± 6 | 100± 1 |
| Group added with NaCl | 819±54 | 953± 0 |
| Group added with NaCl + 15 µM phlorizin | 521±35 | 643±76 |
| Group added with NaCl + 500 µM phlorizin NaCl | 78± 8 | 118± 6 |

As is clear from TABLES 1 and 2, α-methyl glucose was Na⁺-dependently taken up in the human, mouse and rat SGLT homologs, as in hSGLT1 and hSGLT2, but it was demonstrated that resistance to phlorizin was more potent than in SGLT1 and SGLT2.

### EXAMPLE 2

### Analysis of the distribution of expressed SGLT homolog in human gastrointestinal tract

### (1) Analysis of expression level of the human SGLT homolog by TaqMan PCR

The primers and probe used for TaqMan PCR were searched using Primer Express ver. 1.0 (PE Biosystems, Japan) to choose Primer cccgatgctttccacatgcttc (SEQ ID NO: 7), Primer acaatgacctggtctgtgcacc (SEQ ID NO: 8) and Probe acatcccttggccaggtctcattttcgg (SEQ ID NO: 9). As a reporter dye for the probe, FAM (6-carboxyfluorescein) was added thereto.

The PCR fragment of the human SGLT homolog was used as a standard DNA. The reaction solution for the PCR contained 1 µl of the human SGLT homolog DNA as the template, 1 µl of Pfu Turbo DNA Polymerase (STRATAGENE), 0.5 µM each of Primer gggggccagaggatccaggtgta (SEQ ID NO: 10) and Primer gcaatcatcagcccccgcagac (SEQ ID NO: 11), 200 µM dNTPs and 5 µl of the buffer solution attached to the enzyme to make the total volume 50 µl. The PCR was carried out by reacting at 94°C for 1 minute and then repeating 35 cycles of the reactions at 96°C for 20 seconds, 60°C for 30 seconds and 72°C for 1 minute, and a final elongation reaction at 72°C for 7 minutes. The PCR product was subjected to electrophoresis on 1 % agarose gel, and 0.7 Kbp DNA fragment was excised and extracted with Gel Extraction Kit (Qiagen). The PCR fragment, adjusted in concentrations of 10⁰ to 10⁶ copies/µl, was used as the standard DNA.

### (2) Analysis of the expression level of human SGLT1 homolog by TaqMan PCR

The primers and probe used for TaqMan PCR were searched using Primer Express ver. 1.0 (PE Biosystems, Japan) to choose Primer agcaccctcttcaccatgga (SEQ ID NO: 12), Primer aaacaaccttccggcaatcat (SEQ ID NO: 13) and Probe ccaaggtccgcaagagagcatctga (SEQ ID NO: 14). As a reporter dye for the probe, FAM (6-carboxyfluorescein) was added thereto.

The PCR fragment of the human SGLT1 homolog was used as a standard DNA. The reaction solution for the PCR contained 1 µl of human SGLT1 homolog DNA as the template, 1 µl of Pfu Turbo DNA Polymerase (STRATAGENE), 0.5 µM each of tgtgtcgtcccttcagaatgtg (SEQ ID NO: 15) and Primer agaactagttcaggcaaaatatgcatg (SEQ ID NO: 16), 200 µM dNTPs and 5 µl of the buffer solution attached to the enzyme to make the total volume 50 µl. The PCR was carried out by reacting at 94°C for 1 minute and then repeating 35 cycles of the reactions at 96°C for 20 seconds, 60°C for 30 seconds and 72°C for 1 minute, and a final elongation reaction at 72°C for 7 minutes. The PCR product was subjected to electrophoresis on 1 % agarose gel, and 1.0 Kbp DNA fragment was excised and extracted with Gel Extraction Kit (Qiagen). The extracted DNA was adjusted in concentrations of 10⁰ to 10⁶ copies/µl and used as the standard DNA.

Human gastrointestinal tract MTC panels (CLONTECH) were used as cDNA sources of various tissues. PCR was carried out on ABI PRISM 7700 Sequence Detection System (PE Biosystems, Japan) by adding TaqMan Universal PCR Master Mix (PE Biosystems, Japan) to 200 nM of the above primer (SEQ ID NO: 7), 100 nM of the above primer (SEQ ID NO: 8), 50 nM of the above probe (SEQ ID NO: 9) and the template DNA in given amounts described in the attached brochure, followed by analysis.

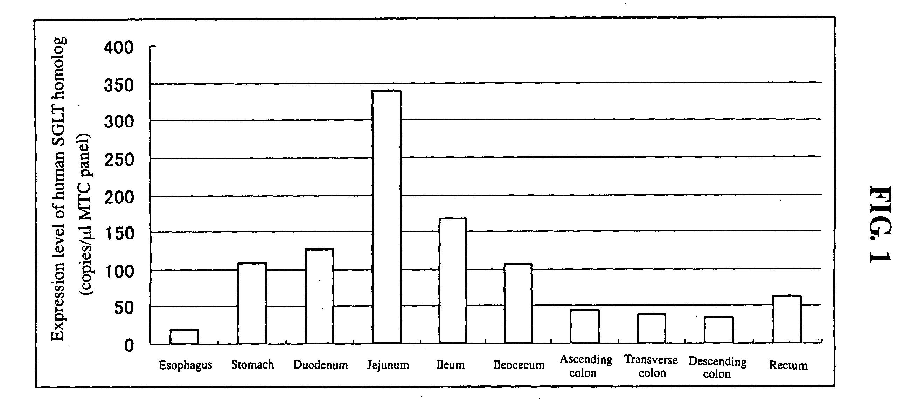

Using human gastrointestinal tract MTC panels (CLONTECH), distribution of the expressed human SGLT homolog was examined by TaqMan PCR. As shown in FIG. 1, the highest expression was observed in the jejunum, which was the main absorption site for dietary glucose. It is considered that polysaccharides would undergo degradation with pancreatic amylase in the duodenum and absorbed mainly in the jejunum.

### EXAMPLE 3

### Expression analysis of SGLT1 and the SGLT homolog in normal human small intestine epithelial cells in primary culture

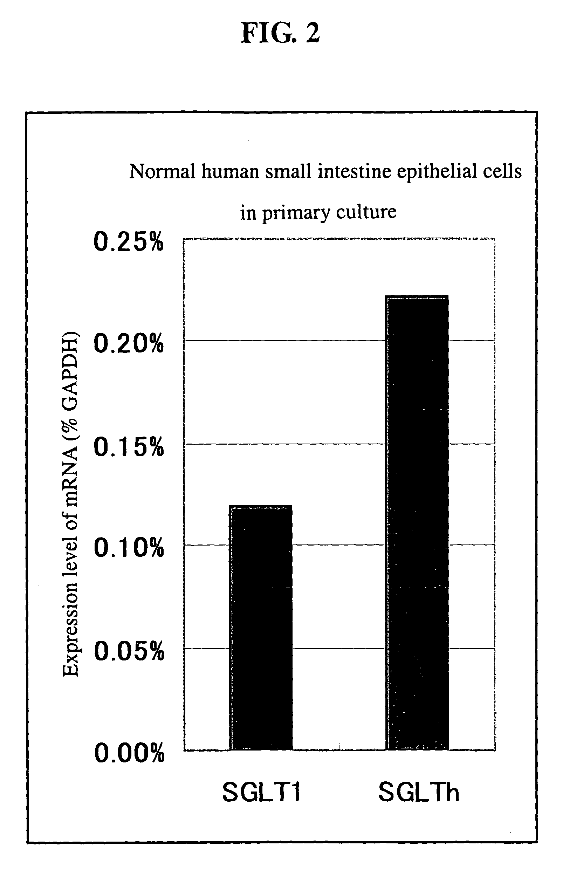

Normal human small intestine epithelial cells (Cell System-IE Cells) were purchased from Dainippon Pharmaceutical Co., Ltd. The cells were plated on CS-2.0 medium (supplemented with 25 mM glucose, 10% FBS and an antibiotic) charged in a collagen-coated plate (24-well plate) in 2 x 10⁵ cells/well. While the medium was exchanged every 2 other days, incubation was continued for 13 days. Using RNAeasy mini kit (Qiagen), the total RNA was extracted. Expression levels of human SGLT (hSGLT) homolog and hSGLT1 were determined by TaqMan PCR using TaqMan Gold RT-PCR Kit (PE Biosystems). As shown in FIG. 2, the hSGLT homolog (hSGLTh) was expressed in normal human small intestine epithelial cells (Cell System-IE Cells) on a level equal to or more than hSGLT1.

### EXAMPLE 4

### Immunostaining of human small intestine slices with anti-human SGLT homolog antibody

### (1) Production of anti-human SGLT homolog peptide antibody

A 261-275 peptide in the human SGLT homolog in which cysteine was bound to the 275th amino residue (Kurabo Industries Ltd.) was used as an immunogen peptide [H-HisAsnLeuArgAspProValSerGlyAspIleProTrpGlyCys (SEQ ID NO: 17)-NH₂ = H-HNLRDPVSGDIPWGC-NH₂]. After N-(γ-maleimidobutyryloxy)succinimide (GMBS) was mixed with Keyhole Limpet Hemocyanin (KLH), the mixture was reacted at room temperature for 40 minutes. The reaction mixture was fractionated on Sephadex G-25 column to give the maleimide-introduced KLH. The maleimide-introduced KLH was equally mixed with 5 mg of the immunogen peptide, followed by reacting at 4°C for a day. The reaction mixture was dialyzed to PBS buffer for 2 days and the dialysate was suspended in PBS buffer in a concentration of 1 mg/ml.

Equal volumes of Freund's complete adjuvant and the antigen were mixed (0.6 ml in total) and the mixture was subcutaneously injected to New Zealand white rabbits of 3 months old for immunization. Subsequently, the animal was boostered 3 times every 2 or 3 other weeks with the equal amount of the immunogen, together with incomplete Freund's adjuvant.

The synthetic peptide (5 mg) was coupled to 5 ml of Sulfo-Link gel via cysteine bond, and equilibrated with PBS buffer. The antiserum (5 ml) was passed through the gel coupled to the peptide, and then washed 3 times with PBS buffer (5 ml). The antibody bound to the peptide was eluted with 8 ml of 0.1N glycine/HCl buffer solution (pH 2.5). The eluate was neutralized with 2.4 ml of Tris buffer to give the anti-human SGLT homolog peptide antibody.

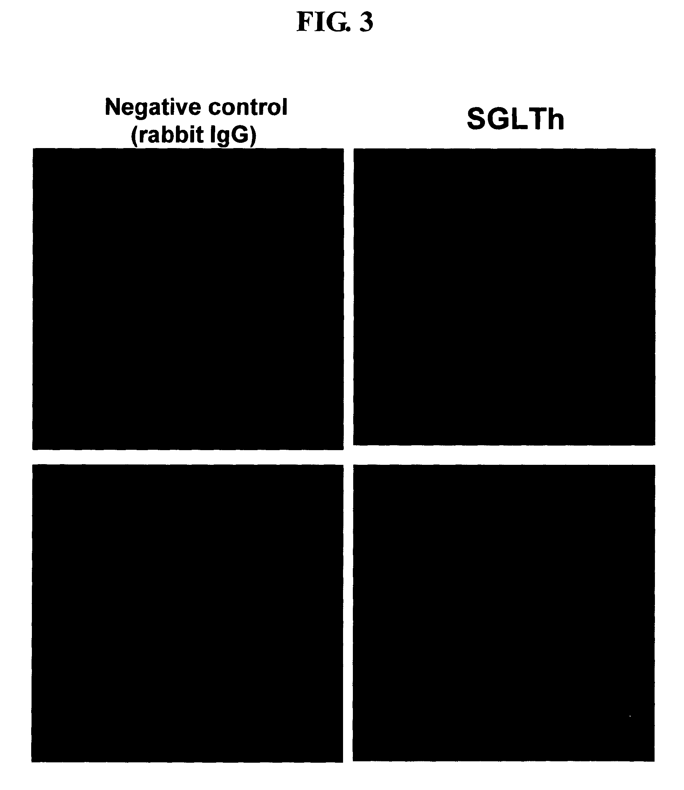

When human small intestine slices were immunostained using this antibody, it was confirmed on a protein level that the homolog was expressed over the glucose absorption site from the basal part to the top of the Villi in epithelial cells (SGLT1 is expressed there (Eur. J. Physiol., 430:151, 1995)). The results are shown in FIG 3.

### EXAMPLE 5

### Change in expression of the SGLT homolog in diabetic animal

It is noted that in the patient with diabetes mellitus, glucose absorption increased in the small intestine (Am. J. Physiol. Gastrointest. Liver Physiol., 282: G241-G248 2002). By TaqMan PCR, the expression level of the SGLT homolog in the small intestine was compared between diabetic KKA^{y} model mice and normal mice C57/BL6 and between diabetic Wistar fatty rats and normal Wistar lean rats.

### (1) Analysis of expression level of the mouse SGLT homolog by TaqMan PCR

The primers and probe used for TaqMan PCR were searched using Primer Express ver.1.0 (PE Biosystems, Japan) and chosen as below: Primer (5'-tgcacagaccaggtgattgtg-3') [SEQ ID NO: 18] Primer (5'-gcacggagcctcccttg-3') [SEQ ID NO: 19] and Probe (5'-ctcgcagccaacaatctttcacatg-3') [SEQ ID NO: 20]. As a reporter dye for the probe, FAM (6-carboxyfluorescein) was added thereto.

The PCR fragment of the mouse SGLT homolog was used as a standard DNA. The reaction solution for the PCR contained 1 µl of mouse SGLT homolog DNA as the template, 1 µl of Pfu Turbo DNA Polymerase (STRATAGENE), 0.5 µM each of the primer (5'-atctctaatgtccagcaatgtg-3') [SEQ ID NO: 21] and the primer (5'-accagcttggggtaggcaat-3') [SEQ ID NO: 22], 200 µM dNTPs and 5 µl of the buffer solution attached to the enzyme to make the total volume 50 µl. The PCR was carried out by reacting at 94°C for 1 minute and then repeating 40 cycles of the reactions at 96°C for 20 seconds, 62°C for 30 seconds and 72°C for 30 seconds, and a final elongation reaction at 72°C for 7 minutes. The PCR product was subjected to electrophoresis on 2% agarose gel, and 0.9 kbp DNA fragment was excised and extracted with Gel Extraction Kit (Qiagen). The PCR fragment, adjusted in concentrations of 10⁰ to 10⁶ copies/µl, was used as the standard DNA.

The duodenum and jejunoileum were isolated from diabetic KKA^{y} model mice and normal mice C57/BL6 and total RNA was extracted from the organs. The total RNA was extracted in accordance with the procedures of ISOGEN (Nippon Gene Co., Ltd.). Using 0.1 µg of the obtained RNA as the template, cDNA was synthesized by the procedures of TaqMan Reverse Transcription Reagents (Roche, Inc.). Using 1 µl of this cDNA as the template, PCR was carried out on ABI PRISM 7700 Sequence Detection System (PE Biosystems, Japan) by adding TaqMan Universal PCR Master Mix (PE Biosystems, Japan) to 200 nM of the above primer (5'-tgcacagaccaggtgattgtg-3') [SEQ ID NO: 18], 200 nM of the above primer (5'-gcacggagcctcccttg-3') [SEQ ID NO: 19] and 50 nM of the above probe (5'-ctcgcagccaacaatctttcacatg-3') [SEQ ID NO: 20] in given amounts described in the attached brochure, followed by analysis.

### (2) Analysis of expression level of the rat SGLT homolog by TaqMan PCR

The primers and probe used for TaqMan PCR were searched using Primer Express ver. 1.0 (PE Biosystems, Japan) and chosen as below. Primer (5'-ctcacagtcttggccacctg-3') [SEQ ID NO: 23] Primer (5'-agaaccggctctctctggag-3') [SEQ ID NO: 24] and Probe (5'-tgcacggaccaggtgattgtgc-3') [SEQ ID NO: 25]. As a reporter dye for the probe, FAM (6-carboxyfluorescein) was added thereto.

The PCR fragment of the rat SGLT homolog was used as a standard DNA. The reaction solution for the PCR contained 1 µl of rat SGLT homolog DNA as the template, 1 µl of Pfu Turbo DNA Polymerase (STRATAGENE), 0.5 µM each of the primer (5'-tctggagtcagcctgcacacct-3') [SEQ ID NO: 26] and the primer (5'-cagccttctcagctgggctcag-3') [SEQ ID NO: 27], 200 µM dNTPs and 5 µl of the buffer solution attached to the enzyme to make the total volume 50 µl. The PCR was carried out by reacting at 94°C for 1 minute and then repeating 40 cycles of the reactions at 96°C for 20 seconds, 62°C for 30 seconds and 72°C for 30 seconds, and a final elongation reaction at 72°C for 7 minutes. The PCR product was subjected to electrophoresis on 2% agarose gel, and 0.9 kbp DNA fragment was excised and extracted with Gel Extraction Kit (Qiagen). The PCR fragment, adjusted in concentrations of 10⁰ to 10⁶ copies/µl, was used as the standard DNA.

The small intestine from diabetic Wistar fatty rats and the small intestine from Wistar lean normal rats were divided into the upper, middle and lower parts, from which total RNA was extracted. The total RNA was extracted in accordance with the procedures of ISOGEN (Nippon Gene Co., Ltd.). Using 0.1 µg of this RNA as the template, cDNA was synthesized by the procedures of TaqMan Reverse Transcription Reagents (Roche, Inc.) Using 1 µl of this cDNA as the template, PCR was carried out on ABI PRISM 7700 Sequence Detection System (PE Biosystems, Japan) by adding TaqMan Universal PCR Master Mix (PE Biosystems, Japan) to 200 nM of the primer (5'-ctcacagtcttggccacctg-3') [SEQ ID NO: 23], 200 nM of the primer (5'-agaaccggctctctctggag-3') [SEQ ID NO: 24] and 50 nM of the probe (5'-tgcacggaccaggtgattgtgc-3') [SEQ ID NO: 25] in given amounts described in the attached brochure, followed by analysis.

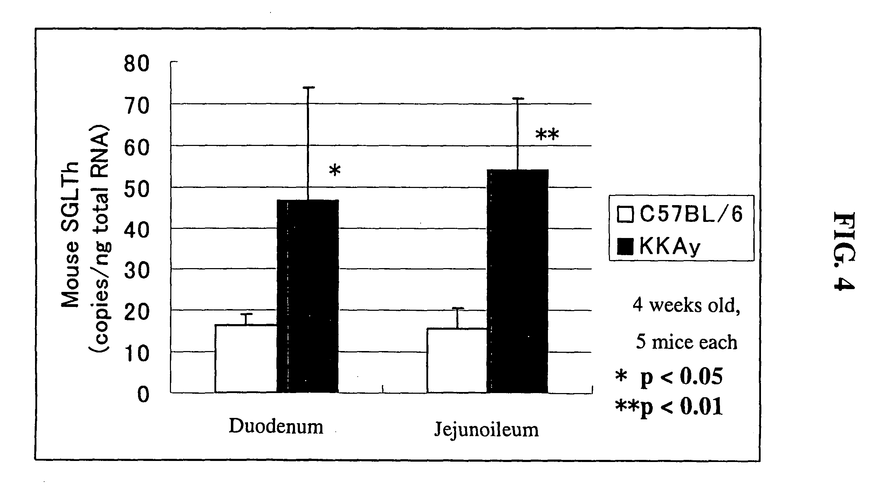

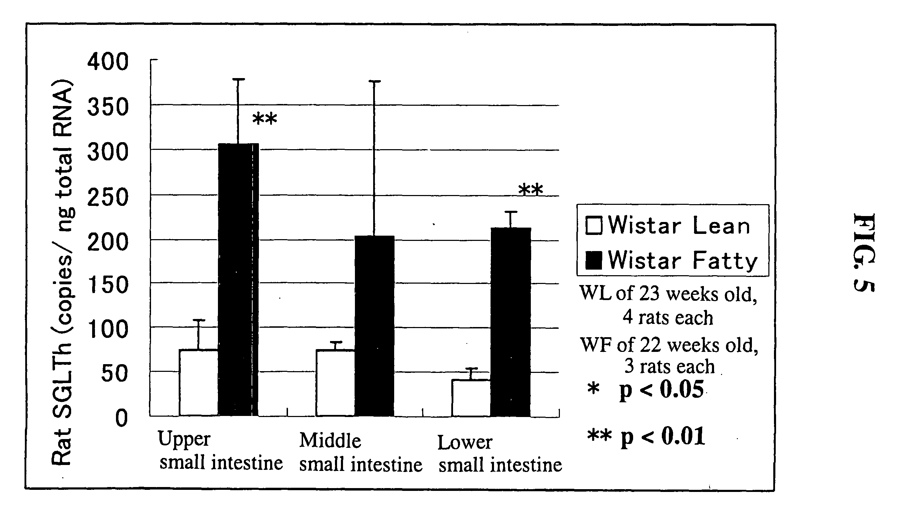

The results are shown in FIG. 4 and FIG. 5. In the small intestine from diabetic KKA^{y} mice and diabetic Wistar fatty rats, the SGLT homolog was more expressed than in the respective normal animals for control, suggesting that the increased expression would be a cause for increased glucose absorption in the small intestine or postprandial hyperglycemia in diabetes mellitus.

### EXAMPLE 6

### Expression of the SGLT homolog in the small intestines from human, mouse, rat, hamster and monkey

The expression levels of SGLT1 and the SGLT homolog in the small intestines from human, mouse, rat, hamster and monkey were compared by RT-PCR.

### Expression analysis of the SGLT homolog in the small intestine by RT-PCR

As to human SGLT homolog, human jejunum cDNA (DCA, Inc.) was used as a template and PCR was carried out using Primer 1 gggggccagaggatccaggtgta [SEQ ID NO: 28] and Primer 2 aaaatagccccagaggaagatgttga [SEQ ID NO: 29]. The reaction solution for the PCR contained 1 µl of Pfu Turbo DNA Polymerase (STRATAGENE), 0.5 µM each of Primer 1 and Primer 2, 200 µM dNTPs and 5 µl of the buffer solution attached to the enzyme to make the total volume 50 µl. The PCR was carried out by reacting at 94°C for 1 minute and then repeating 40 cycles of the reactions at 96°C for 20 seconds, 62°C for 30 seconds and 72°C for 1.5 minutes, and a final elongation reaction at 72°C for 7 minutes. Likewise, PCR was carried on human SGLT1 (NM_000343) using Primer 3 atcctgactgggtttgcttt [SEQ ID NO: 30] and Primer 4 atgctgatgccaatcagcac [SEQ ID NO: 31]. The reaction was carried out under the conditions repeating 40 cycles of the reactions at 96°C for 20 seconds, 55°C for 30 seconds and 72°C for 30 seconds, otherwise the same as the conditions for the human SGLT homolog.

As to human actin, PCR was carried out using Primer 5 agagctacgagctgcctgac [SEQ ID NO: 32] and Primer 6 acatctgctggaaggtggac [SEQ ID NO: 33], under the reaction conditions by repeating 40 cycles of the reactions at 96°C for 20 seconds, 64°C for 30 seconds and 72°C for 30 seconds, otherwise the same as the conditions for the human SGLT homolog.

The reaction conditions for RT-PCR used for the hamster SGLT homolog and SGLT1, the monkey SGLT homolog and SGLT1, the mouse SGLT homolog and SGLT1 as well as the rat SGLT homolog and SGLT1are described below.

Turning to the hamster SGLT homolog and SGLT1, cDNA was synthesized from random primers using the RNA extracted from the small intestine of Syrian male hamster of 9 weeks old as the template and using TaqMan reverse transcription reagents (Applied Biosystems, Inc.). The PCR was carried out under the reaction conditions described below, using this cDNA as the template.
Hamster SGLT homolog: Forward: tgccacagtacttgaagaaacgat [SEQ ID NO: 34] Reverse: tgaagaacattgggaggatt [SEQ ID NO: 35] Repeating 40 cycles of the reactions at 95°C for 20 seconds, 50°C for 30 seconds and 72°C for 30 seconds
Hamster SGLT1: Forward: caatgaagtaggagggtatgagg [SEQ ID NO: 36] Reverse: tggcgctgttgaagatggaggtc [SEQ ID NO: 37] Repeating 40 cycles of the reactions at 95°C for 20 seconds, 56°C for 30 seconds and 72°C for 1 minute

Turning to the monkey SGLT homolog and SGLT1, cDNA was synthesized from random primers using the RNA extracted from the small intestine of cynomolgus monkeys as the template and using TaqMan reverse transcription reagents. The PCR was carried out under the reaction conditions described below, using this cDNA as the template.
Monkey SGLT homolog: Forward: atctctaatgtccagcaatgtg [SEQ ID NO: 38] Reverse: gcaatcatcagcccccgcagac [SEQ ID NO: 39] Repeating 40 cycles of the reactions at 95°C for 20 seconds, 57°C for 30 seconds and 72°C for 1 minute
Monkey SGLT1 Forward: atcctgactgggtttgcttt [SEQ ID NO: 40] Reverse: atgctgatgccaatcagcac [SEQ ID NO: 41] Repeating 40 cycles of the reactions at 95°C for 20 seconds, 55°C for 30 seconds and 72°C for 1 minute

Turning to the mouse SGLT homolog and SGLT1, cDNA was synthesized from random primers using the RNA extracted from the small intestine of male KKA^{y} mouse of 8 weeks old as the template and using TaqMan reverse transcription reagents. The PCR was carried out under the reaction conditions described below, using this cDNA as the template.
Mouse SGLT homolog: Forward: ggcaatggaaccaggagtgtc [SEQ ID NO: 42] Reverse: tcacgcaaaatagccccagagaaag [SEQ ID NO: 43] Repeating 40 cycles of the reactions at 95°C for 20 seconds, 60°C for 30 seconds and 72°C for 2.5 minutes
Mouse SGLT1 (NM_019810): Forward: atggacagtagcaccttgagcc [SEQ ID NO: 44] Reverse: tcaggcaaaataggcatggcag [SEQ ID NO: 45] Repeating 40 cycles of the reactions at 95°C for 20 seconds, 55°C for 30 seconds and 72°C for 2.5 minutes

Turning to the rat SGLT homolog and SGLT1, cDNA was synthesized from random primers using the RNA extracted from the small intestine of male Wistar fatty rat of 22 weeks old as the template and using TaqMan reverse transcription reagents. The PCR was carried out under the reaction conditions described below, using this cDNA as the template.
Rat SGLT homolog: Forward: caatggaacctggagcttcaag [SEQ ID NO: 46] Reverse: tcacgcaaaatagccccagagaaag [SEQ ID NO: 47] Repeating 40 cycles of the reactions at 95°C for 20 seconds, 60°C for 30 seconds and 72°C for 2.5 minutes
Rat SGLT1 (NM_013033): Forward: atggacagtagcaccttgagcc [SEQ ID NO: 48] Reverse: tcaggcaaaataggcgtggcag [SEQ ID NO: 49] Repeating 40 cycles of the reactions at 95°C for 20 seconds, 55°C for 30 seconds and 72°C for 2.5 minutes

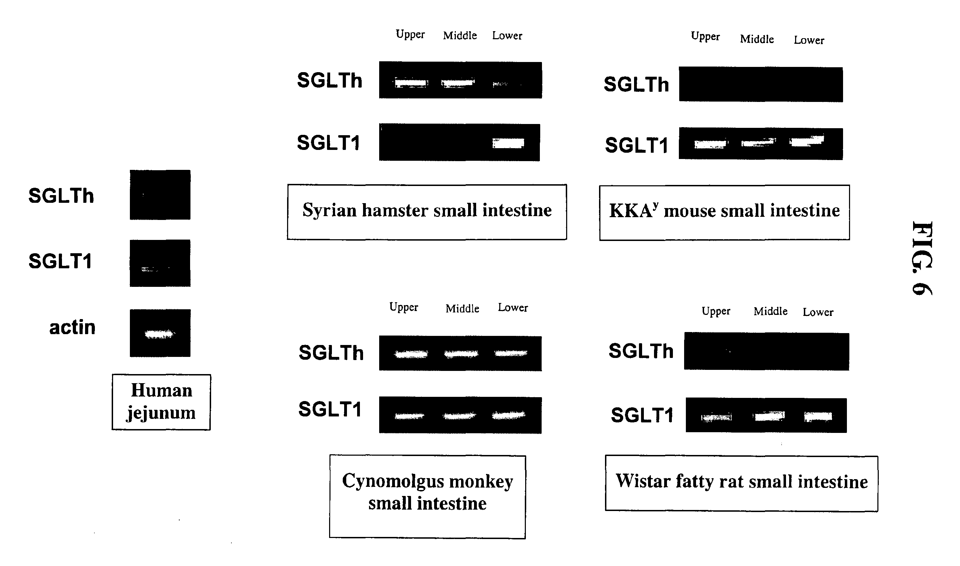

As shown by the results in FIG. 6, the expression of SGLT1 was higher in mice and rats than the SGLT homolog, whereas in monkeys both SGLT1 and the SGLT homolog were equally expressed as in human, and in hamster the expression of the SGLT homolog was higher than SGLT1.

### EXAMPLE 7

### Determination of glucose uptake level in the mouse, rat and hamster small intestines by the organ culture system

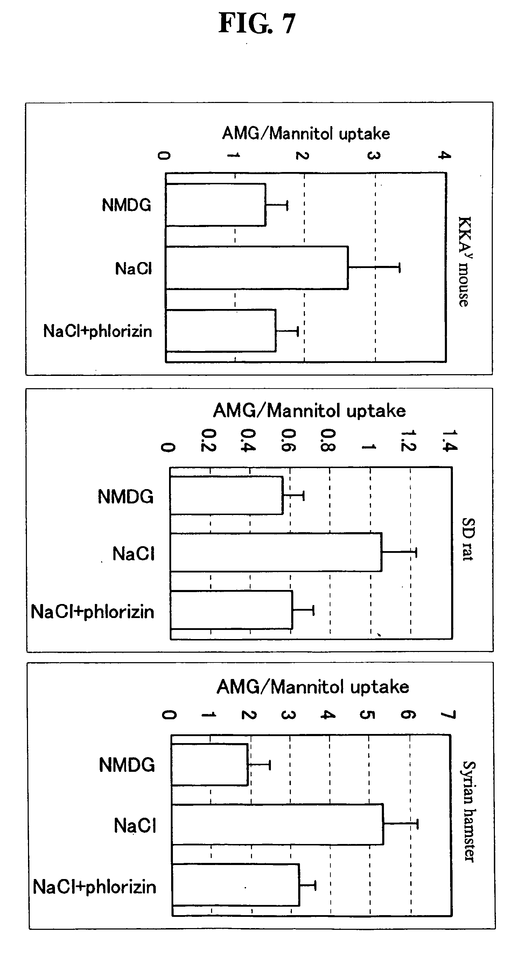

The glucose uptake level was determined by the intestinal organ culture system in accordance with the procedures described in Peptides, 19: 1249-1253, 1998 and J. Agric. Food Chem., 48: 5618-5623, 2000. The carotid was cut in mice, rats, guinea pigs and hamsters under ether anesthesia to put them to death. The jejunal region was excised, mesenteric fats were removed, the tract was dissected and the contents were washed and cut in 1 cm length. The jejunal slices were washed 3 times with a buffer solution (125 mM N-Methyl-D-Glucamine (NMDG), 1.2 mM KH₂PO₄, 2.5 mM CaCl₂, 1.2 mM MgSO₄, 4 mM Glutamine, 10 mM HEPES (pH 7.2), 0.1 mg/ml BSA) and then transferred to a 48-well plate by one slice/well. The buffer solution was replaced with 270 µl of the same buffer solution or the buffer solution containing NaCl or NaCl + 30 µM Phlorizin in place of NMDG. After 30 µl each of 10 mM α-methyl glucose and 10 mM D-mannitol were added to the well (containing 0.12 µCi of [¹⁴C] α-methyl glucose (AMG) (Amersham Pharmacia Biotech, Inc.) and 0.3µCi of [³H] D-mannitol (Perkin Elmer LifeSciences)), the mixture was incubated at 37°C for 10 minutes, followed by washing 3 times with 300 µl of chilled PBS buffer solution. The jejunal slices were transferred to a mini-vial for liquid scintillation counting. After 500 µl of tissue solubilizer Solvable (Perkin Elmer LifeSciences) was added thereto, the mixture was treated at 50°C for 2 hours to solubilize the tissues. After 5 ml of liquid scintillator Ultima Gold-XR (Perkin Elmer LifeSciences) was added, ¹⁴C and ³H taken up into the jejunal slices were counted. As shown by the results in FIG. 7, the SGLT activity strongly resistant to phloridzin was noted in the hamster small intestine, whereby the glucose uptake activity of the SGLT homolog was demonstrated.

### EXAMPLE 8

### Study of substrate specificity of SGLT1 and the SGLT homolog

The human SGLT homolog or human SGLT1-transfected COS7 cells were prepared and the respective substrate specificities were examined. The α-methyl glucose uptake test was carried out in accordance with the procedures described in Am. J. Physiol., 270: G833-G843, 1996 and J. Clin. Invest., 93: 397-404, 1994. The cells were plated on 100 µl of DMEM medium containing 10% FBS charged in a 96-well plate in a cell density of 3 x 10⁴ cells/well, followed by culturing overnight at 37°C. Each well was washed 3 times with a reaction buffer solution supplemented with 125 mM N-Methyl-D-Glucamine (1.2 mM KH₂PO₄, 2.5 mM CaCl₂, 1.2 mM MgSO₄, 4 mM Glutamine, 10 mM HEPES (pH 7.2), 0.1 mg/ml BSA) and further cultured for an hour in the same buffer solution to remove the glucose remained in the cells. Next, 150 mM NaCl was added to the reaction buffer solution above and glucose or galactose was added to the mixture in 0, 1 and 10 mM, respectively. Then 90 µl each of the thus prepared mixtures was added per well. In addition, 10 µl each of α-methyl glucose in a final concentration of 1 mM, which contained 0.02 µCi of [¹⁴C]-α-methyl glucose (Amersham Pharmacia Biotech, Inc.), was added per well to carry out a glucose uptake reaction for an hour. After washing 3 times with 200 µl of chilled PBS, 100 µl each of liquid scintillator was added per well and the radioactivity of ¹⁴C taken up into the cells was counted with a scintillation counter. The results indicate that SGLT1 had affinity to glucose and galactose, whereas the SGLT homolog showed little affinity to galactose, though the homolog showed affinity to glucose.

### EXAMPLE 9

### Kinetic analysis of glucose uptake mediated by SGLTlor the SGLT homolog

The human SGLT homolog or the human SGLT1-transfected COS7 cells were prepared and subjected to kinetic analysis of glucose uptake, respectively. The α-methyl glucose uptake test was carried out in accordance with the procedures described in Am. J. Physiol., 270: G833-G843, 1996 and J. Clin. Invest., 93: 397-404, 1994. The cells were plated on 100 µl of DMEM medium containing 10% FBS charged in a 96-well plate in a cell density of 3 x 10⁴ cells/well, followed by culturing overnight at 37°C. Each well was washed 3 times with a reaction buffer solution supplemented with 125 mM N-Methyl-D-Glucamine (1.2 mM KH₂PO₄, 2.5 mM CaCl₂, 1.2 mM MgSO₄, 4 mM Glutamine, 10 mM HEPES (pH 7.2), 0.1 mg/ml BSA) and further cultured for an hour in the same buffer solution to remove the glucose remained in the cells. Next, 150 mM Nacl was added to the reaction buffer solution above and α-methyl glucose was added to the mixture in 0 to 20 mM. Then 90 µl each of the thus prepared mixtures was added per well. In addition, 10 µl each of α-methyl glucose in a final concentration of 1 mM, which contained 0.02 µCi of [¹⁴C]-α-methyl glucose (Amersham Pharmacia Biotech, Inc.), was added per well to carry out a glucose uptake reaction for an hour. After washing 3 times with 200 µl of chilled PBS, 100 µl each of liquid scintillator was added per well and the radioactivity of ¹⁴C taken up into the cells was counted with a scintillation counter to determine Km and Vmax in the glucose uptake. As a result, SGLT1 had Km = 1.8 mM and Vmax = 3.9 nmol/hour/106 cells, whereas the SGLT homolog showed Km = 7.9 mM and Vmax = 8.0 nmol/hour/106 cells. These results indicate that SGLT1 is a transporter having a high affinity and low transport capacity, whereas the SGLT homolog is a transporter having a low affinity but a high transport capacity.

### EXAMPLE 10

### Cloning of cDNA encoding Na⁺/glucose transporter protein derived from Syrian hamster

Using TaqMan reverse transcription reagents (Applied Biosystems, Inc.), cDNA used as the template was prepared from random primers, which were acquired from RNA extracted from the small intestine of male Syrian hamsters, 9 weeks old, according to the manual of RNeasy Kit (QIAGEN). This cDNA from the hamster small intestine was used as the template, and PCR was carried out using the set of Primer A (5'-gcaatggagcctggagattcag-3') [SEQ ID NO: 54] and Primer B (5'-agcctgcctctggtcttg-3') [SEQ ID NO: 55] for cloning of the SGLT homolog and for cloning of SGLT1, using the set of Primer C (5'-atggacagtagcaccttgagccccgcggtca-3') [SEQ ID NO: 56] and Primer D (5'-gattcaagcaaaatatccgtggcaaaaga-3') [SEQ ID NO: 57]. The reaction solution for the PCR contained 10 ng of the above cDNA for use as the template, 1 µl of Pfu Turbo DNA Polymerase (STRATAGENE), 0.5 µM each of the primers, 200 µM dNTPs and 5 µl of the buffer solution attached to the enzyme to make the total volume 50 µl. The reaction was carried out by reacting at 94°C for 1 minute and then repeating 40 cycles of the reactions at 96°C for 20 seconds, 58°C for 30 seconds and 72°C for 2 minutes, and a final elongation reaction at 72°C for 2.5 minutes. The reaction product was subjected to electrophoresis on 1 % agarose gel, and the DNA fragment was excised and extracted from the gel with Gel Extraction Kit (Qiagen). The extracted DNA was subcloned onto pCR Blunt II vector in accordance with the formulation of TOPO Ligation Kit (Invitrogen Corp.) and transfected to Escherichia coli TOP10 to acquire cDNA clones. The amino acid sequence deduced from the hamster SGLT homolog cDNA is shown by SEQ ID NO: 50 and the base sequence of the region encoding the amino acid sequence represented by SEQ ID NO: 50 is shown by SEQ ID NO: 51. The homology of the amino acid sequence of hamster SGLT homolog to the amino acid sequence of human, mouse and rat SGLT homologs was 86.4%, 88.4% and 88.3%, respectively. The amino acid sequence deduced from the hamster SGLT1 cDNA is shown by SEQ ID NO: 52 and the base sequence of the region encoding the amino acid sequence represented by SEQ ID NO: 52 is shown by SEQ ID NO: 53. The homology of the amino acid sequence of hamster SGLT1 to the amino acid sequence of human, mouse and rat SGLT1 was 83.9%, 89.7% and 90.6%, respectively.

### EXAMPLE 11

### Determination of glucose uptake level mediated by the hamster SGLT homolog or SGLT1

The glucose uptake level of the hamster SGLT homolog can be determined by the following procedures. The hamster SGLT homolog or SGLTlclosed from hamster intestinal cDNA was subcloned onto animal cell expression vector pcDNA3.1 (Invitrogen Corp.) to construct a vector expressing the hamster SGLT homolog or SGLT1 in animal cells. Using FuGENE6 reagent (Roche), 1 µg each of the expression vectors was transfected to COS7 cells (5 x 10⁵ cells) to give the respective expression cells. The experiment for α-methyl glucose uptake in the hamster SGLT homolog or the SGLT1-transfected COS7 was carried out in accordance with the procedures described in Am. J. Physiol., 270: G833-G843, 1996 and J. Clin. Invest., 93: 397-404, 1994. The cells were plated on 100 µl of DMEM medium containing 10% FBS charged in a 96-well plate in a cell density of 3 x 10⁴ cells/well, followed by culturing overnight at 37°C. After the cells were washed 3 times with a reaction buffer solution supplemented with 125 mM N-Methyl-D-Glucamine (1.2 mM KH₂PO₄, 2.5 mM CaCl₂, 1.2 mM MgSO₄, 4 mM Glutamine, 10 mM HEPES (pH 7.2), 0.1 mg/ml BSA), the cells were cultured for further an hour in the same buffer solution to remove the glucose remained in the cells. Next, 150 mM NaCl was added to the reaction buffer solution above and phloridzin (Sigma Inc.) was added to the mixture in 0, 0.1 and 1 mM, respectively. Then 90 µl each of the thus prepared mixtures was added per well. In addition, 10 µl each of α-methyl glucose in a final concentration of 1 mM, which contained 0.02 µCi of [¹⁴C]-α-methyl glucose (Amersham Pharmacia Biotech, Inc.), was added per well to carry out a glucose uptake reaction for an hour. After washing 3 times with 200 µl of chilled PBS, 100 µl each of liquid scintillator was added per well and the radioactivity of ¹⁴C taken up into the cells was counted with a scintillation counter.

### INDUSTRIAL APPLICABILITY

The compound or its salts that regulate the activity of the protein used in the present invention or the expression of a gene for the protein, and the neutralizing antibody that regulates the activity of the protein can be used as, e.g., postprandial hyperglycemia-improving agents, glucose absorption promoters, etc. In addition, the antisense polynucleotide of the present invention can regulate the expression of the protein used in the present invention and can be used as, e.g., postprandial hyperglycemia-improving agents, etc.

## Claims

1. A glucose uptake inhibitor in the small intestine comprising a compound or a salt thereof that inhibits the activity of a Na⁺/glucose transporter (SGLT) homolog.

2. A glucose uptake inhibitor in the small intestine comprising a compound or a salt thereof that inhibits the expression of a gene for Na⁺/glucose transporter (SGLT) homolog.

3. The inhibitor according to claim 1 or 2, which is a postprandial hyperglycemia-improving agent.

4. The inhibitor according to claim 1 through 3, which is an agent for the prevention/treatment of diabetes, obesity or hyperlipemia.

5. A glucose uptake promoter in the small intestine comprising a compound or a salt thereof that promotes the activity of a Na⁺/glucose transporter (SGLT) homolog.

6. A glucose uptake promoter in the small intestine comprising a compound or a salt thereof that promotes the expression of a gene for Na⁺/glucose transporter (SGLT) homolog.

7. The promoter according to claim 5 or 6, which is a glucose absorption promoter.

8. The agent according to claim 1 through 7, wherein the Na⁺/glucose transporter (SGLT) homolog is a protein comprising the same or substantially the same amino acid sequence as the amino acid sequence represented by SEQ ID NO: 1, its partial peptide, or a salt thereof.

9. The agent according to claim 1 through 7, wherein the Na⁺/glucose transporter (SGLT) homolog is a protein comprising the same or substantially the same amino acid sequence as the amino acid sequence represented by SEQ ID NO: 3, its partial peptide, or a salt thereof.

10. The agent according to claim 1 through 7, wherein the Na⁺/glucose transporter (SGLT) homolog is a protein comprising the same or substantially the same amino acid sequence as the amino acid sequence represented by SEQ ID NO: 5, its partial peptide, or a salt thereof.

11. The agent according to claim 1 through 7, wherein the Na⁺/glucose transporter (SGLT) homolog is a protein comprising the same or substantially the same amino acid sequence as the amino acid sequence represented by SEQ ID NO: 50, its partial peptide, or a salt thereof.

12. A glucose uptake inhibitor in the small intestine comprising an antisense polynucleotide comprising the entire or part of a base sequence complementary or substantially complementary to a base sequence of a polynucleotide encoding a Na⁺/glucose transporter (SGLT) homolog.

13. The inhibitor according to claim 12, which is a postprandial hyperglycemia-improving agent.

14. The inhibitor according to claim 12 or 13, which is an agent for the prevention/treatment of diabetes, obesity or hyperlipemia.

15. The inhibitor according to claim 12 through 14, wherein the polynucleotide encoding the Na⁺/glucose transporter (SGLT) homolog is a polynucleotide comprising the same or substantially the same base sequence as the base sequence represented by SEQ ID NO: 2, SEQ ID NO: 4, SEQ ID NO: 6 or SEQ ID NO: 51.

16. A glucose uptake inhibitor in the small intestine comprising an antibody to a Na⁺/glucose transporter (SGLT) homolog.

17. The inhibitor according to claim 16, which is a postprandial hyperglycemia-improving agent.

18. The inhibitor according to claim 16 or 17, which is an agent for the prevention/treatment of diabetes, obesity or hyperlipemia.

19. The inhibitor according to claim 16 through 18, wherein the Na⁺/glucose transporter (SGLT) homolog is a protein comprising the same or substantially the same amino acid sequence as the amino acid sequence represented by SEQ ID NO: 1, SEQ ID NO: 3, SEQ ID NO: 5 or SEQ ID NO: 50, its partial peptide, or a salt thereof.

20. A diagnostic agent for postprandial hyperglycemia comprising an antibody to a Na⁺/glucose transporter (SGLT) homolog.

21. A diagnostic agent for postprandial hyperglycemia comprising a polynucleotide encoding a Na⁺/glucose transporter (SGLT) homolog.

22. A method of screening a compound or its salt that regulates the glucose uptake activity of a Na⁺/glucose transporter (SGLT) homolog in the small intestine, which comprises using the homolog.

23. The screening method according to claim 22, wherein the Na⁺/glucose transporter (SGLT) homolog is a protein comprising the same or substantially the same amino acid sequence as the amino acid sequence represented by SEQ ID NO: 1, SEQ ID NO: 3, SEQ ID NO: 5 or SEQ ID NO: 50, its partial peptide, or a salt thereof.

24. A kit for screening a compound or its salt that regulates the glucose uptake activity of a Na⁺/glucose transporter (SGLT) homolog in the small intestine, comprising the homolog.

25. A method of screening a compound or its salt that regulates the glucose uptake activity of a Na⁺/glucose transporter (SGLT) homolog in the small intestine, which comprises using a polynucleotide encoding the homolog.

26. The screening method according to claim 25, wherein the polynucleotide encoding the Na⁺/glucose transporter (SGLT) homolog is a polynucleotide comprising the same or substantially the same base sequence as the base sequence represented by SEQ ID NO: 2, SEQ ID NO: 4, SEQ ID NO: 6 or SEQ ID NO: 51.

27. A kit for screening comprising a compound or its salt that regulates the glucose uptake activity of a Na⁺/glucose transporter (SGLT) homolog in the small intestine, which comprises using a polynucleotide encoding the homolog.

28. A method of inhibiting glucose uptake in the small intestine, which comprises inhibiting the activity of a Na⁺/glucose transporter (SGLT) homolog.

29. A method of inhibiting glucose uptake in the small intestine, which comprises inhibiting the expression of a gene for Na⁺/glucose transporter (SGLT) homolog.

30. The method according to claim 28 or 29, which is a method of improving postprandial hyperglycemia.

31. The method according to claim 28 through 30, which is a method for the prevention/treatment of diabetes, obesity or hyperlipemia.

32. A method of promoting glucose uptake in the small intestine, which comprises promoting the activity of a Na⁺/glucose transporter (SGLT) homolog.

33. A method of promoting glucose uptake in the small intestine, which comprises promoting the expression of a gene for Na⁺/glucose transporter (SGLT) homolog.

34. The method according to claim 32 or 33, which is a method of promoting glucose absorption.

35. A method of inhibiting glucose uptake in the small intestine, which comprises administering to a mammal an effective dose of a compound or its salt that inhibits the activity of a Na⁺/glucose transporter (SGLT) homolog.

36. A method of inhibiting glucose uptake in the small intestine, which comprises administering to a mammal an effective dose of a compound or its salt that inhibits the expression of a gene for Na⁺/glucose transporter (SGLT) homolog.

37. The method according to claim 35 or 36, which is a method of improving postprandial hyperglycemia.

38. The method according to claim 35 through 37, which is a method for the prevention/treatment of diabetes, obesity or hyperlipemia.

39. A method of promoting glucose uptake in the small intestine, which comprises administering to a mammal an effective dose of a compound or its salt that promotes the activity of a Na⁺/glucose transporter (SGLT) homolog.

40. A method of promoting glucose uptake in the small intestine, which comprises administering to a mammal an effective dose of a compound or its salt that promotes the expression of a gene for Na⁺/glucose transporter (SGLT) homolog.

41. The method according to claim 39 or 40, which is a method of promoting glucose absorption.

42. Use of a compound or its salt that inhibits the activity of a Na⁺/glucose transporter (SGLT) homolog to manufacture a glucose uptake inhibitor in the small intestine.

43. Use of a compound or its salt that inhibits the activity of a Na⁺/glucose transporter (SGLT) homolog to manufacture a glucose uptake inhibitor in the small intestine.

44. The use according to claim 42 or 43, wherein the glucose uptake inhibitor in the small intestine is a postprandial hyperglycemia-improving agent.

45. The use according to claim 42 through 44, wherein the glucose uptake inhibitor in the small intestine is an agent for the prevention/treatment of diabetes, obesity or hyperlipemia.

46. Use of a compound or its salt that promotes the activity of a Na⁺/glucose transporter (SGLT) homolog to manufacture a glucose uptake promoter in the small intestine.

47. Use of a compound or its salt that promotes the activity of a Na⁺/glucose transporter (SGLT) homolog to manufacture a glucose uptake promoter in the small intestine.

48. The use according to claim 46 or 47, wherein the glucose uptake promoter in the small intestine is a glucose absorption promoter.
